# EUROPEAN PATENT SPECIFICATION

(11) **EP 3 645 088 B1**
(45) Date of publication and mention of the grant of the patent: **28.08.2024**
(21) Application number: 18824973.4
(22) Date of filing: 17.05.2018
(51) Int. Cl.: A61M 11/02, A61M 15/08, B05B 11/06, A61M 15/00

(54) **ADJUSTABLE DOSING DELIVERY**
EINSTELLBARE DOSIERUNGSABGABE
ADMINISTRATION DE DOSE RÉGLABLE

(30) Priority: 29.06.2017 US 201762526386 P
(43) Date of publication of application: 06.05.2020
(73) Proprietor: Sipnose Ltd, 2069200 Yokne' am Ilit (IL)
(72) Inventor: SHAHAF, Daniel, 1513000 Kibbutz Dganiya B (IL); SHICHOR, Iris, 3093414 Zichron Yaakov (IL)
(74) Representative: Vossius & Partner Patentanwälte Rechtsanwälte mbB
(86) International application number: PCT/IL2018/050545
(87) International publication number: WO 2019/003216

(56) References cited:
- WO-A1-2013/128447
- WO-A1-2015/025324
- WO-A1-2016/199135
- US-A- 5 740 794
- US-A1- 2002 023 641
- US-A1- 2006 151 629
- US-A1- 2016 129 205
- US-B1- 6 398 074

## Description

### FIELD OF THE INVENTION

The present invention generally pertains to a system for delivering aerosolized substance to a natural orifice of the body.

### BACKGROUND OF THE INVENTION

In the pharmaceutical and therapeutic areas, nasal delivery is a known and acceptable delivery route that can provide a solution for a wide range of therapeutics and medical indications.

Prior art nasal delivery devices suffer from difficulties in: dose control, delivery accuracy, drug storage, and treatment with multiple medications. Metered dose delivery, where a fixed dose is released in every activation is common in nasal and aerosol delivery of pharmaceuticals. For metered dose delivery, each delivery device is designed to deliver a specific, unchangeable dose per activation. In some therapeutics areas, there is a need for a different dose for each patient, sometimes even for each therapeutic treatment for the same patient. For example: for the pediatric population, the dose often must depend on the weight of the patient. In other cases, such as acute treatment in cases of breakthrough seizures, breakthrough pain, or Parkinson's "off stage", the treatment should reflect the patient's medical condition.

Loading of a desirable dose to fit a specific need is not common in nasal delivery applications, moreover, one of the main obstacles to providing an adjustable dose in nasal delivery devices, especially when released in the form of an aerosol, is maintaining, over a wide range of dose sizes, reproducible aerosol characteristics in terms of dose released, residual volume, droplet diameter, droplet size distribution and plume geometry.

In many therapeutic areas there is a need to provide the patient with a number of medications during the same treatment. With oral delivery, a patient can consume different pills one after the other, or consume one pill that contains more than one active ingredient/drug (For example: L-Dopa + Carbidopa; Topiramate + Phentermine (as in Qsymia) and more. In injectable and nasal delivery, the ability to mix compounds before or at the time of administration is less common.

In some cases there is a need to store compounds and materials separately in order to maintain stability and functionality. This can conflict with a need to deliver the compounds in a specialized formulation for better user experience and /or better absorption. For example, a drug which is a biologic or protein or an active compound that is not stable in solution can be highly stable as a dry powder. For such drugs, mixture of the dry powder drug with a liquid formations at the time of administration, could provide a homogenous solution to be delivered efficiently to the target tissue. User compliance could be high, since it is effective and provides a positive user experience. Another example is an insoluble compound that is stored in one compartment and is released with, slightly before or slightly after a formulation that will either affect the spread of the compound in the target tissue, or improve the absorption of the compound via the mucosal tissue, or change the adhesion of the compound to the mucosal tissue (to lengthen or shorten the exposure of the mucosal tissue to the compound, or, the formulation can protect the active compound from degradation and/or clearance. However, in the prior art there is no way to provide both long-term separate storage for components of a formulation and automatic mixing of the components at the time of administration.

It is therefore a long felt need to provide a system which can be optimized for efficient delivery of a substance to a target site, said optimization neglecting neither the need to bring sufficient material to the target site, nor the need to ensure adequate absorption into and through the mucosal layer. The document WO 2015/025324 A1 represents relevant prior art for the present patent application.

### SUMMARY OF THE INVENTION

The invention is set out in the appended claims.

### BRIEF DESCRIPTION OF THE DRAWINGS

The invention is herein described, by way of example only, with reference to the accompanying drawings, wherein:
Fig. 1A-D shows examples of the prior art;
Fig. 2 shows a cross-section of the face, illustrating zones in the nose from which material therein transfers to different parts of the body;
Fig. 3 illustrates an embodiment of a device of the present invention;
Figs. 4A-D, 6A-G and 7A-F illustrate embodiments of capsules;
Figs. 8A-D and 9A-C illustrate an embodiment of a device of the present invention;
Figs. 10 A-D and 11A-H illustrate embodiments of nozzles for devices of the present invention;
Figs. 12A-D, 13A-D, 14, 15A-C, 16A-B, 17A-C, 18, 19A-D, 20A-D, 21A-C, 22A-C, 23, 24A-D, 25 A-D, 26, 27A-C, 28A-E, 29A-C, 29D1-D2, 29E and 30 illustrate embodiments of the device of the present invention;
Fig. 31 illustrates the formation of an aerosol within the nozzle of the a device of the present invention;
Figs 32A-D, 33A1, 33A2, 33B-D, 34A-D, 35A-C and 36A-C show results of discharge from devices into the base of an oil-filled column;
Fig. 37 shows pressure developed in a closed tube after discharge from devices into the tube;
Fig. 38A-C shows a nasal cast and results of discharge of devices into the nasal cast;
Fig. 39 illustrates an orifice, identifying the plume angle;
Fig. 40A-B illustrates amount of material exiting a nasal cast for different devices;
Figs. 41A-B and 42 illustrate an experimental setup;
Fig. 43A-J shows the results of experiments using the setup;
Fig. 44 shows a typical particle size distribution;
Fig. 45A-E shows deposition of substance on a target.

### DETAILED DESCRIPTION OF THE PREFERRED EMBODIMENTS

The following description is provided, alongside all chapters of the present invention, so as to enable any person skilled in the art to make use of said invention and sets forth the best modes contemplated by the inventor of carrying out this invention. Various modifications, however, will remain apparent to those skilled in the art, since the generic principles of the present invention have been defined specifically to provide a device capable of improving the transfer of medicament to a predetermined desired location and to provide a device capable of improving the delivery of medicament through the tissue.

In the present invention, a combination of parameters and forces such as pressure, gas/air volume orifice diameter enable the formation of optimized aerosol characteristics for both improved delivery of aerosol to the target area (such as the olfactory epithelium in the nasal cavity) and enhanced absorption at that area for better delivery to a desired tissue (such as the brain).

The term **'ul'** or '**µl**' hereinafter refers to the unit micro liters.

The term **'capsule'** or **'container'** hereinafter refers to a container configured to contain a flowable substance. The term flowable refers hereinafter to any liquid, gas, aerosol, powder and any combination thereof. It should be emphasized that the term capsule can also refer to a predetermined volume within the same in which a flowable substance is placed. In other words, the predetermined volume is sized and shaped to enclose a predetermined volume of said substance.

The term **'plurality'** hereinafter refers to an integer greater than or equal to one.

The term **'olfactory epithelium'** hereinafter refers to a specialized epithelial tissue inside the nasal cavity. The olfactory epithelium lies in the upper top portion of the nasal cavity.

The term **'substance'** hereinafter refers to any substance capable of flowing. Such a substance can be a granular material, including a powder; a liquid; a gel; a slurry; a suspension; and any combination thereof.

The term **'gas'** refers to any fluid that can be readily compressed. Gases as used herein include, but are not limited to, air, nitrogen, oxygen, carbon dioxide, helium, neon, xenon and any combination thereof. Devices charged by hand will typically use air as the carrier gas.

The term **'channel'** hereinafter refers to a passageway allowing passage of a fluid through at least a portion of a mixing mechanism. The channel can be disposed within a portion of the mixing mechanism, forming a closed bore; it can be on an exterior of a portion of the mixing mechanism, forming a groove on the portion of the mixing mechanism, and any combination thereof.

The term **'about'** refers hereinafter to a range of 25% below or above the referred value.

The term **'biologic'** or **'biologic response modifier'** hereinafter refers to material manufactured in or extracted from biological sources such as a genetically engineered protein derived from human genes, or a biologically effective combination of such proteins.

All pressures herein are gauge pressures, relative to atmospheric pressure. Pressure units will be written herein using the standard abbreviation for "gauge', namely, "g". For example, atmospheric pressure is 0 barg and a pressure of 1 bar above atmospheric is 1 barg.

The term **'release time'** refers hereinafter to the time for the drug and carrier gas to substantially completely exit the device. Typically, the release time is affected by the activation time and reflects the time for the device to reconfigure from the *active configuration* to the *inactive configuration* or vice versa.

The terms **'the device', 'the present device', 'the SipNose device'** and **'SipNose'** will be used interchangeably to refer to the device of the present invention.

All figures shown herein are illustrative and none is to scale.

The present invention teaches a device for delivering a predetermined amount of a substance, preferably comprising a medication or combination of medications, into a body orifice of a subject, the orifice comprising any of the body's natural orifices, including a nostril, the mouth, the ear, the throat, the urethra, the vagina, the rectum and any combination thereof.

In preferred embodiments of the device, the device comprises a delivery mechanism and a medicament capsule, as described hereinbelow. The device can apply a broad range of drugs and materials to the nasal cavity for local effect, deliver a broad range of drugs and materials through the nasal cavity to the systemic circulation, deliver a broad range of drugs and materials through the nasal cavity to the central nerve system (CNS) the brain, spinal cord and associated nerves, and any combination thereof.

In some embodiments, the device is a single-use device, supplying a fixed dose of a substance. In other embodiments, the device is a multiple-use device, configured to supply a fixed dose a plurality of times. In other embodiments, the device is a single-use device which can deliver a wide range of dose sizes. In yet other embodiments, the device is a single-use, multiple dose size device, to increase efficiency in emergency situations, where both time and storage space can be at a premium. In still other embodiments, the device is a multiple-use, multiple dose size device. In some embodiments of the multiple dose size device, the dose size is set by the amount of substance emplaced in the device by a user before use. In other embodiments of a multiple-dose size device, the device comprises means of adjusting the dose size, for example, by means of a knob or slider.

Means of supplying the substance are also flexible, for non-limiting example, the treatment can be emplaced in the device at time of manufacture. It can be emplaced in a single or multiple-use cartridge, with the cartridge insertable into the device, or it can comprise a port or ports, via which an unpackaged substance can be emplaced within the device. An example of the last would be removing a desired amount of a substance from a conventional storage bottle via a syringe, and then emptying the syringe into a predetermined volume within the device.

The drugs to be applied could be, but are not limited to, pharmaceuticals, natural compounds, biologics, hormones, peptides, proteins, viruses, cells, stem cells and any combination thereof.

However, it should be emphasized that the device can be provided alone as well as in combination with a capsule.

In some cases the capsule would be provided with a known medicament within the same and in other cases the capsule would be 'filled' with the medicament just before use.

In some embodiments of the present invention, the device operating characteristics and the substance characteristics can be jointly optimized to maximize uptake of the substance at the desired site. In preferred variants of such embodiments, uptake is further optimized by exploiting synergies between delivery characteristics generated by the device and by the formulation or composition of the delivered material

In some embodiments, the substance comprises one or more agents to optimize delivery through the mucosal membrane by means of mucoadhesive agent and/or a permeability enhancer agent and/or a particulate formulation in the nano-particle or macro-particle range, and any combination thereof. In such embodiments, the combination of the device and substance enhance the delivery of the active agent to the target area (nasal epithelium and more specifically olfactory epithelium) and from there to the target tissue (for example the brain).

A non-limiting example is a composition comprising a drug to be delivered and at least one chemical permeation enhancer (CPE). In a preferred embodiment, the composition contains two or more CPEs which, by using a nasal delivery device, affect in an additive manner or behave synergistically to increase the permeability of the epithelium, while providing an acceptably low level of cytotoxicity to the cells. The concentration of the one or more CPEs is selected to provide the greatest amount of overall potential (OP). Additionally, the CPEs are selected based on the treatment. CPEs that behave primarily by transcellular transport are preferred for delivering drugs into epithelial cells. CPEs that behave primarily by paracellular transport are preferred for delivering drugs through epithelial cells. Also provided herein are mucoadhesive agents that enable the extension of the exposure period of the target tissue/ mucus membrane to the active agent, for the enhancement of delivery of the active agent to and through the mucus membrane.

In contrast to prior-art nasal delivery devices and technologies, the devices of the present invention can produce a fine aerosol in the nasal cavity or other desired body orifice at the target area and at the location of the target tissue instead of producing the aerosol only within the device or immediately after exit from the device. Utilizing the pressure as a driving force and the air as a carrier allows the material to be released from the nozzle as a mixture of aerosol and a pre-aerosolized state. The properties of the resultant aerosol are typically dependent on the properties of the device and of the medium into which the device is discharged. The properties of the device which affect the aerosol characteristics are the delivery pressure, the volume of the delivery gas, the characteristics of its orifice and time to activate.

In some embodiments, the aerosol properties are fairly independent of the delivered substance, while, in other embodiments, the pressure, volume, orifice characteristics, and delivered substance properties can be co-optimized.

In prior-art devices the aerosol is produced in proximity exit of the device. Typically, the aerosol comprises a wide "fan" of aerosol and a low driving force. Therefore, large droplets typically deposit very close to the exit from the device, while smaller droplets tend to quickly contact the walls of the passage, so that deposition is typically predominantly close to the delivery end of the device, with little of the substance reaching desired sites deeper in the body orifice, such as the middle and superior turbinates of the nose.

In contrast, in the present device, the pre-aerosolized mixture of gas and substance exits the device with a significant driving force as a mixture of aerosol and preaerosolized material (fluid or powder). When the preaerosolized material hits the walls of the nasal passages, it "explodes" into a fine aerosol that is capable of being driven by the pressure deep into the nasal passages to deposit in the desired region.

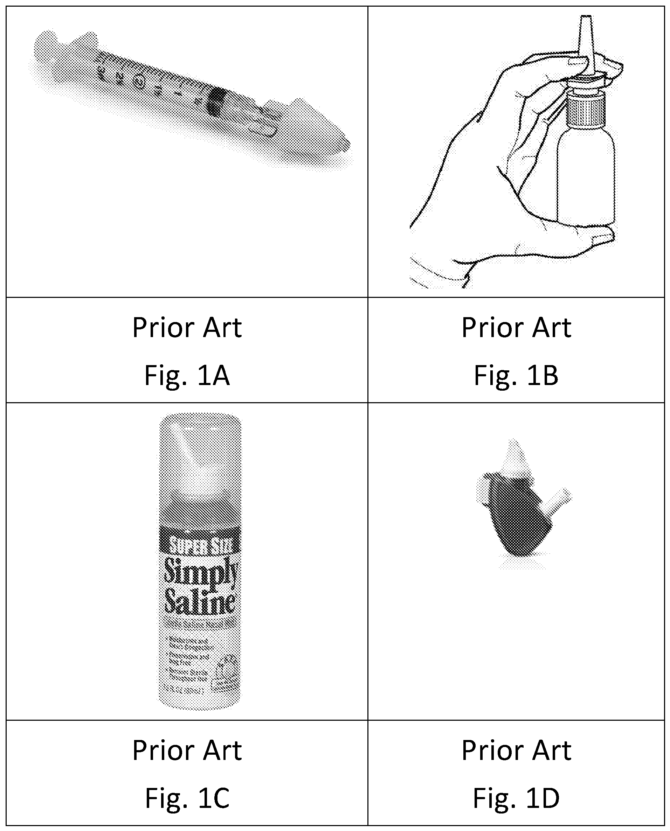

Figs. 1A-D illustrate capsules of prior art intended to deliver medicaments to the nasal passages. **Fig. 1A** illustrates the LMA MAD nasal atomizer from Wolfe Tory, **Fig. 1B** illustrates a typical nasal pump, **Fig. 1C** illustrates a Simply Saline nasal spray, and **Fig. 1D** illustrates the Optinose breath powered delivery device.

Typical prior art devices release aerosolized medicament. However, all have severe limitations.

The LMA MAD nasal atomizer **(****Fig. 1A****)** comprises a syringe, so the dose size can be quite accurate, if the user is careful. However, the delivery pressure is provided by the user depressing a plunger, so that control of delivery speed and delivery pressure (and the droplet size) depend on how hard the user depresses the plunger, making these parameters hard to control. Furthermore, syringe plungers are subject to stick-slip behavior, especially at the start of depression, making the delivery parameter harder to control accurately.

Devices such as nasal pumps **(****Fig. 1B****)** typically do not provide a fixed dose per activation, as the delivery energy is provided by pressure exerted by a user during activation. Typically, there is a wide dispersion in the size of the droplets produced following user activation, making it difficult to accurately target the medicament to a desired location.

The Simply Saline Nasal Mist **(****Fig. 1C****)** comprises a pressurized container. A button is pressed to release a portion of the contents. Each activation reduces the pressure inside the container, thereby reducing the velocity and pressure of delivery and altering the droplet size. The length of activation time depends on the time the button remains depressed, so that there is little control of the amount delivered.

The Optinose breath powered delivery device **(****Fig. 1D****)** is breath-powered. It uses a capsule containing a single dose of the medicament, so dose size is well-controlled. However, delivery speed and delivery pressure (and the particulates dispersion) depend on how hard the user exhales into the device and how long the user continues to exhale. Furthermore, exhalation pressures are typically higher at the start of exhalation than at the end, so the delivery parameters will vary during delivery.

Unlike the device of the present invention, none of the prior-art devices provide accurate control of all of the delivery parameters, which include dose volume, carrier volume, pressure, and delivery velocity.

A further advantage of the device of the present invention (the SipNose device) is that, unlike the prior art devices, it can be configured to accurately deliver large volumes (>100 ul) at high pressure, such that the high-velocity aerosol can be as reliably and reproducibly produced for large volumes as for small.

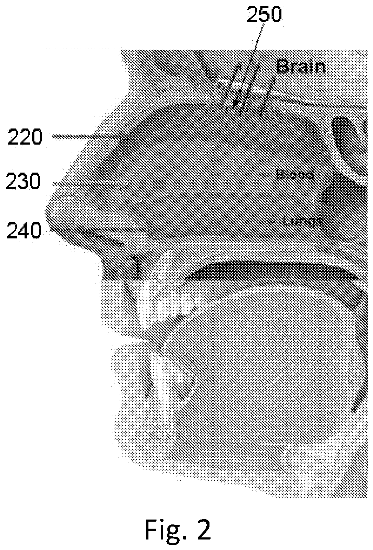

**Fig. 2** illustrates locations for deposition of substances entering the nostrils. Typical locations are (a) deposition in the lungs after passage through the lower turbinates **(240),** thereby enabling transfer of the substance across the walls of the alveoli of the lungs; (b) deposition in the mucous membranes lining the nasal passages, especially the lower **(240),** and middle **(230)** turbinates, facilitating transfer of the substance to the blood; and (c) deposition in the olfactory epithelium mucous membranes of the upper turbinates **(220)** facilitating transfer, via the thin ethmoid bone (not shown) to the brain through the olfactory nerve endings (250) substance, typically as an aerosol, with the mixture of gas and substance entering the body orifice via the delivery end. Typically, discharge (delivery) time is less than about 500 ms.

The embodiments disclosed below disclose non-limiting examples of devices and methods for providing the predetermined volume of gas at the predetermined pressure.

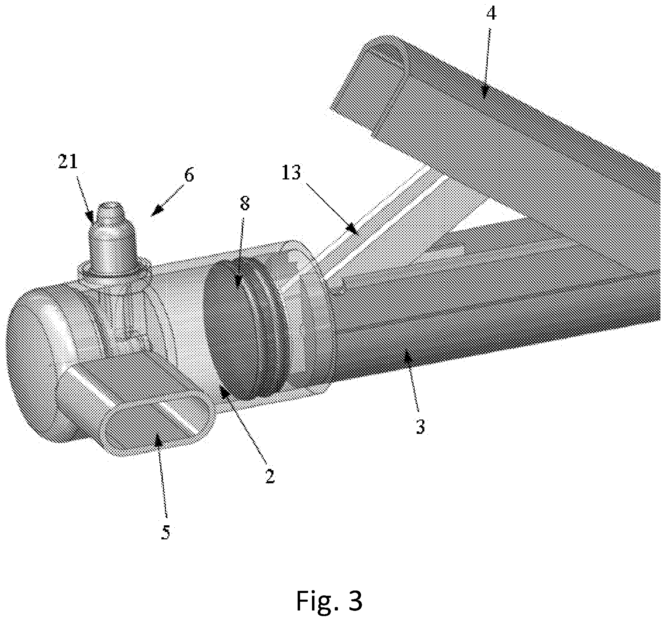

**Fig. 3** shows an exemplary embodiment of the device. In the embodiment of **Fig. 3****,** a piston **(8)** is used to compress the gas. The piston is moved by a lever **(4)** attached to the piston **(8)** by a rotatable connector **(13).** The piston **(8)** fits air-tightly and slideably in the air chamber **(2).** The lever **(4)** is rotatably attached to the handle **(3)** at its proximal end such that the distal end of the lever **(4)** can be pulled away from the handle **(3)** to retract the piston **(8)** toward the proximal end of the device and pull air into the air chamber **(2)** and the distal end of the lever **(4)** can be pushed toward from the handle **(3)** to extend the piston **(8)** toward the distal end of the device and compress the gas. In use, the mouthpiece **(5)** is placed in the mouth and the nosepiece (6) is placed in a nostril. Inhaling on the mouthpiece **(5)** activates the device (mechanism not shown), releasing the compressed gas from the air chamber **(2),** from whence it passes through the substance capsule **(21)** and exits the device through the distal end of the nosepiece **(6).**

The embodiments disclosed in **Fig. 3** are typically configurable into four states: (a) a non-activated state where the valve is in its *inactive configuration*, the chamber contains non-pressurized gas, and the portion of the chamber in fluid connection with the valve is at a minimum, (b) a pre-activated state where the valve is in its *inactive configuration*, the chamber contains non-pressurized gas, and the portion of the chamber in fluid connection with the valve is at a maximum, in this stage the tip to be entered to the body orifice (the delivery end) can be under "vacuum" conditions or not, (c) a loaded configuration where the chamber contains a predetermined amount of pressurized gas and the valve is in its INACTIVE state, and (d) an activated state where the valve is in its ACTIVE state. Typically, the activated state discharges the device, with the mixture of gas and substance released from the device, entering the body orifice via the delivery end.

The characteristics of the aerosol, namely its size, shape and velocity, depend on the speed of exit of the gas from the chamber, the volume of air delivered, the characteristics of the delivery orifice and the activation time. The speed of exit of the gas from the chamber and the volume of air delivered depend on the pressure of the gas in the chamber in the loaded state, on the volume of the chamber in the loaded state, and on the characteristics of the fluid connection between the chamber and the delivery orifice. The less change there is in these characteristics during an activation and between activations, the more reliable and the more reproducible the device will be. Therefore, in controlling the characteristics of the fluid connection, the time taken to open the valve needs to be taken into consideration. In devices of the current invention, the valve opening times are both reproducible and short and are not in any way dependent on the user, so that the delivery comprises a short, reproducible, high velocity pulse of the gas.

The non-activated state and the loaded state appear identical; they differ in that, in the loaded state the chamber contains pressurized gas whereas, in the non-activated state, the chamber does not contain pressurized gas.

In some embodiments, including embodiments intended for use in emergencies or daily home use, the device is a single-use device with only two states, a loaded state and an activated state. The device is provided in the loaded state; activation of the trigger mechanism discharges the gas and substance.

In other embodiments, the device is provided in the pre-activated state. The user transforms the device into the loaded state, pressurizing the gas, and activates the trigger mechanism to discharge the gas and substance.

Capsules can be flexible or rigid. Rigid capsules can comprise materials such as glass, metal, rigid polymer and any combination thereof. Flexible capsules are preferably of a flexible polymer such as silicone. Preferably, capsules are sealable at both ends

Multi-compartment capsules can contain different components of a substance in the different compartments; at least one compartment can contain a carrier gas, and any combination thereof.

In some embodiments, there is a single capsule for the carrier gas and the substance. Some embodiments have separate capsules for substance and gas.

Some embodiments have the carrier gas held in a gas holding chamber. The gas holding chamber can be filled at the time of manufacture or can be filled to the predetermined pressure by a charging mechanism.

Some embodiments have the substance held in a holding chamber. The holding chamber can be filled at the time of manufacture or can be filled by a filling mechanism such as, but not limited to, a syringe.

It should be emphasized that the present invention refers to both one compartment capsules as well as multi-compartment capsules.

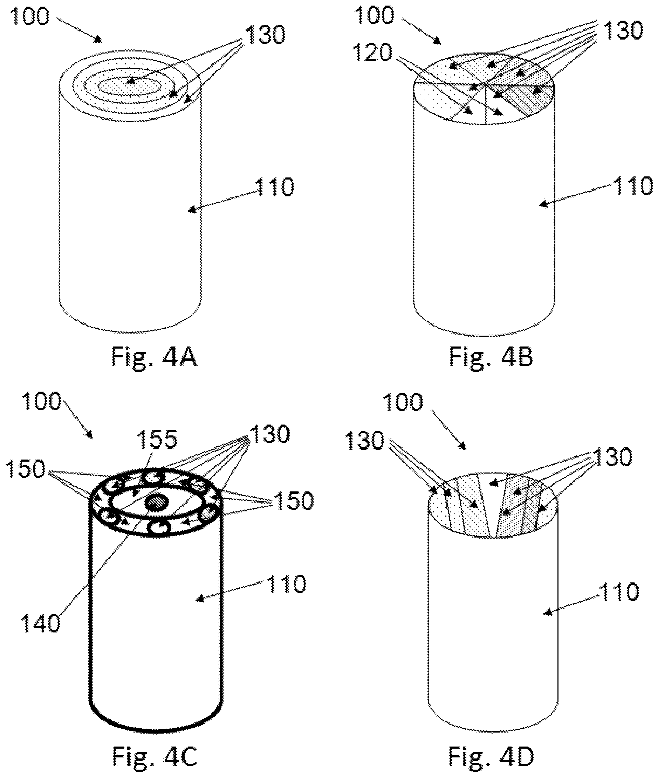

Fig. 4A-E shows exemplary embodiments of multi-compartment capsules.

In multi-compartment capsules, walls divide the capsule into compartments. The compartments can have approximately the same volume or different volumes, and the same thickness or different thicknesses; if circular, they can have the same diameter or different diameters. They can have the same area at the end faces, or different areas.

The compartments, taken together, can form a large fraction of the volume of the capsule, or they can form a small fraction of the volume of the capsule.

Compartment walls can be equally spaced, either angularly or linearly, or they can be unequally spaced. Spacings can be arbitrary, they can be regular, they can follow a pattern, and any combination thereof.

Compartments can be near the edge of the capsule or at other positions within the capsule.

Before use, the compartments are preferably hermetically sealed to prevent mixing of the substances contained therein.

Compartment walls can be substantially similar in shape to the capsule walls (for non-limiting example, lenticular walls within a lenticular capsule) or at least one of the compartments' walls' shape differs from the shape of the cross-section of the capsule. (For non-limiting example, a lenticular wall within a circular capsule.)

Compartment walls can be non-frangible or frangible. Frangible walls permit mixing or reaction of the contents of adjacent compartments before the substances leave the compartments.

Compartments can, but need not, have a frangible membrane at least one end.

Any compartments can contain one substance or a mixture of substances; any two compartments can contain the same substance or mixture thereof, or different substances or mixtures thereof.

The material of any combination of capsule walls and compartment walls can be rigid, semi-flexible, flexible and any combination thereof. Flexible or semi-flexible compartment or capsule walls can reduce dead space - regions of low gas flow - in the air path during activation.

In the embodiment shown in **Fig. 4A****,** the compartments **(130)** are coaxially disposed within the outer tegument **(110),** with the compartments nested within one another. The central compartment forms a cylinder and the remaining compartments, three in the exemplary embodiment of **Fig. 4A****,** each forming an annulus of a cylinder. Nested compartments need not be coaxial.

In the embodiment schematically illustrated in **Fig. 4B****,** the capsule **(100)** comprises an outer tegument **(110)** enclosing *n* angularly disposed compartments **(130)** separated by walls **(120),** where *n* is less than about 10. In the embodiment shown in **Fig. 4B****,** *n* is e.g., six.

In the embodiment schematically illustrated in **Fig. 4C****,** the capsule **(100)** comprises an outer tegument **(110)** enclosing six angularly disposed cylindrical compartments near the edge of the capsule **(130),** a central compartment **(140),** and auxiliary compartments **(150, 155),** for a total of 14 compartments.

In practice, the embodiment illustrated in **Fig. 4C** will have no more than about 20 compartments.

In some embodiments, there is no central compartment **(140).**

In the exemplary embodiment shown, the auxiliary compartments are hollow, containing a substance. In other embodiments, at least one of the auxiliary compartments **(150, 155)** is comprised of solid material, thereby forming part of the structure of the capsule.

In preferred embodiments, the central compartment **(140)** and the central auxiliary compartment **(155)** are solid, forming a solid central core for the structure. The remaining compartments **(130, 150)** comprise substance, where, in preferred embodiments, the compartments **(130)** contain a substance such as a medicament and the auxiliary compartments **(150)** contain a propellant, preferably compressed gas.

In the exemplary embodiment shown in **Fig. 4D****,** the compartments **(130)** form slices within the outer tegument **(110).** In the exemplary embodiment of **Fig. 4D****,** some of the slices have parallel sides, while the central slice is wedge-shaped; in other embodiments, all of slices have substantially parallel sides. In yet other embodiments, a plurality of slices are wedge-shaped. Slice-type capsules can have up to about 10 compartments.

In an exemplary embodiment the compartments are arranged longitudinally, with the walls between the segments being frangible. Any number of such compartments can be used and the lengths of the compartments can differ.

These embodiments are merely exemplary; any combination of the above arrangements can be used.

In the exemplary embodiments shown, the walls separating the compartments are planar. In other embodiments, the walls can form a curve, either regular or irregularly shaped.

The main longitudinal axis of at least one of the compartments can be parallel to the main longitudinal axis of the capsule, it can be spirally disposed it can be at an angle to the main longitudinal axis of the capsule, and any combination thereof.

The main longitudinal axes of the compartments can be straight, they can form regular curve, they can form irregular curves, and any combination thereof. For any pair of compartments, the main longitudinal axes can be the same or they can be different.

In most embodiments, at least part of the upstream closure surface (not shown) and the downstream closure surface (not shown) of the capsule are frangible or otherwise removable, such that, when broken or otherwise removed, the medications can be delivered to the desired deposition site. In a variant of these embodiments, different portions at least one closure surface have different breaking strengths, such that the different portions can be broken at different times during delivery of the medication, enabling either differential mixing of medical formulations in different compartments or differential delivery of the medications in at least two of the compartments.

In some embodiments, at least part of the side surface of the capsule is frangible, enabling yet another mixing path or delivery path.

Capsules can be cylindrical with circular cross-section, as shown, cylindrical with oval, elliptical, lenticular, or polygonal cross-section, with the polygon having at least three sides and not more than about 20 sides. The polygon can be a regular or irregular.

Capsules can be spherical, elliptical, ovoid, pillow-shaped, football-shaped, stellate and any combination thereof. Capsules can form regular or irregular shapes.

Compartments can have substantially constant cross-section through the device or the cross-section can vary in area, in shape, or in any combination thereof.

In an exemplary embodiment, the mixing mechanism comprises spirally-disposed air channels at the periphery of the mixing mechanism . The central part of the mixing mechanism is solid, forcing the carrier gas and the substances to pass through the channels . By narrowing the channel through which the gas passes and by changing the direction of the gas flow, mixing of the substances is enhanced. The mixing mechanism fits within the tegument of the capsule and mixing occurs within the capsule.

In some embodiments, a single channel is used. This can have a cross-section which is annular, circular, polygonal, lenticular, pie-shaped irregular, or any combination thereof. The channel main longitudinal axis can pass through any part of the capsule. Non-limiting examples include a circular cross-section with main longitudinal axis at the capsule center, and an annular cross-section at the periphery of the capsule, with main longitudinal axis at the capsule center.

In some embodiments, the capsule comprises two units, one comprising at least one substance and one comprising the mixing mechanism, such that the substances exit the compartments and are then mixed in the mixing mechanism.

In other embodiments, the mixing mechanism comprises channels disposed throughout its cross-section.

Channels can be arbitrarily arranged across a cross-section, regularly arranged across a cross-section, or irregularly arranged across a cross-section.

Channels can be linearly disposed, parallel to the main longitudinal axis of the capsule; or linear and disposed at an angle to the main longitudinal axis of the capsule.

The main longitudinal axis of at least one channel can be curved with respect to the main longitudinal axis of the mixing mechanism, with respect to an axis perpendicular to the main longitudinal axes, or any combination thereof.

Any combination of the above channel shapes can be used.

The shape of a channel cross-section can be substantially the same along the length of the channel, the shape can change along the length of the channel, the size of the cross-section can change along the length of the channel, and any combination thereof.

Shapes of the cross-sections of the channels can vary in the same manner along the length of the channel, or they can vary in different manners.

Shapes of the cross-sections of the channels can be the same for all the channels, or the shapes of the cross-sections of at least two channels can be different.

Sizes of the cross-sections of the channels can vary in the same manner along the length of the channel, or they can vary in different manners.

Sizes of the cross-sections of the channels can be the same for all the channels, or the sizes of the cross-sections of at least two channels can be different.

In some embodiments, the mixing mechanism comprises a plurality of longitudinal sections, with the sections having fluidly connected channels, but the channels are differently disposed longitudinally. For non-limiting example, a two-section device can have spirally disposed channels with left-handed spirals in the first section and right-handed spirals in the second section.

In some embodiments, there are different numbers of channels in the two sections. In other embodiments, there are the same number of channels in the two sections.

In other multi-section mixing mechanisms , sections comprising channels are fluidly connected by substantially channel-free regions.

Mixing mechanisms can comprise between 1 and 10 regions. Individual regions can have any of the channel dispositions described hereinabove.

In some embodiments, mixing can be done by an integral mixing mechanism, either a single-section or a multi-section device. In other embodiments, mixing can be done by disposing a plurality of single-section mechanisms end-to-end, either abutting each other or with spacers to provide channel-free regions.

During the process of mixing, the first and second flowable substances can be mechanically mixed with each other and with the air or other gas, they can be reacted with each other, and any combination thereof.

In some embodiments, reaction of at least one flowable substance can be enhanced by a catalyst deposited on or part of the walls of the mixing region.

Criteria of the capsule, whether single-compartment or multi-compartment, can be optimized to include: ensuring that a single dose of the substance is delivered in its entirety, ensuring that the single dose contains the predetermined amount of the substance, ensuring that the dose is delivered to the desired region of the nose, and ensuring that delivery of the dose causes the minimum possible discomfort to the patient. Any combination of these criteria can be optimized for each particular combination giving rise to a different embodiment of the capsule.

The capsule can also be optimized for ease of insertion into a delivery device, for ease of removal from a delivery device, for stability of the contents during storage, for resistance of the capsule materials to environmental degradation, for resistance to undesired fracture, for reliability of use, for completeness of mixing, for completeness of reaction, and any combination thereof.

In some embodiments, the capsule comprises a filter configured to remove from the air at least one selected from a group consisting of particles, particulates, bacteria, viruses, moisture, and undesired gases before the air contacts the user. Such a filter, by preventing unpleasant odors or tastes from reaching the user and by preventing particles or particulates from reaching the user, can make the experience of using the device much more pleasant for the user and much safer. By removing bacteria and viruses, infection of the user can be prevented.

In some embodiments, the capsule contains only a single dose of the substance, the capsule being replaced after each use. In other embodiments, the capsule contains multiple doses of the substance, preferably packed separately, so that the dose is fresh for each use.

During dispensing of the substance, the gas passing through the capsule entrains the substances contained within the compartments such that the substances have a predetermined distribution within the dispensed mixture, where the predetermined distribution can be a homogeneous distribution or a heterogeneous distribution. Heterogeneous distributions can be: an arbitrary distribution, a distribution in which the dispersion of the at least one substance within the mixture follows a predetermined pattern, and any combination thereof.

According to another embodiment of the present invention, movement of air into the chamber during transformation of the device into said pre-activated state creates a vacuum in the region near or in the capsule.

As disclosed above, the capsule can be designed in various forms to allow various options for drug component mixing; drug component maintenance at low humidity; temperature variation (heating or cooling), viscosity and variation, and combinations of these options.

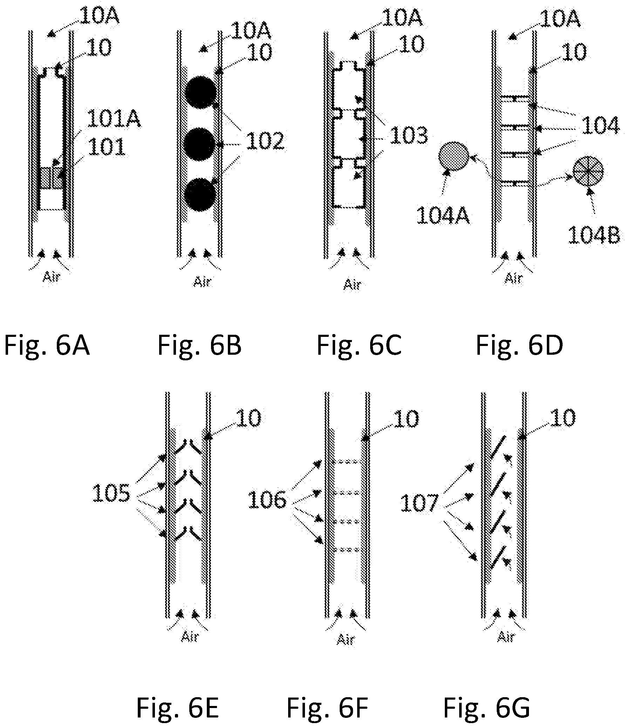

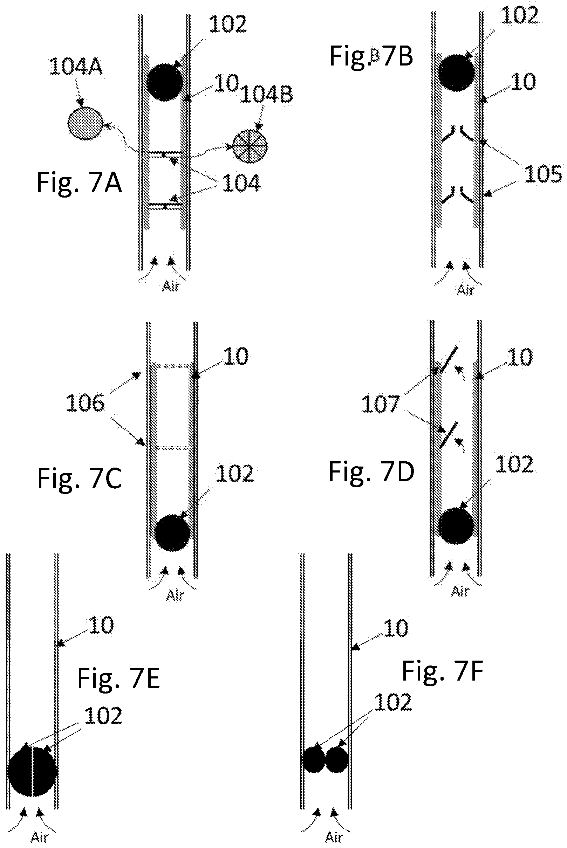

**Figs. 6** and 7 show embodiments of multi-compartment capsules, with exemplary embodiments of the separators configured to subdivide the capsules into compartments.

In some embodiments, the multi-compartment substance storage medium can be a container or an integral volume in a delivery device. For simplicity, the multi-compartment substance storage medium will be referred to as a capsule herein.

**Fig. 6A** shows a plunger-type barrier **(101)** between compartments. In this exemplary embodiment, there is one plunger **(101).** In other embodiments, more or fewer plungers **(101)** can be present. The plunger **(101)** comprises a hole or slot small enough to prevent passage of substance therethrough, but wide enough to allow passage of compressed air therethrough. When the device is activated, compressed gas (curved arrows at bottom) enters the capsule **(10).** The pressure forces the plunger **(101)** upward, forcing substance above the plunger **(101)** out of the top of the capsule. Substance below the plunger **(101)** will be forced upward by the compressed air, to mix with the substance above the plunger in a nose piece (not shown). The plunger **(101)** passes through the top of the capsule into an intermediate space **(10A)** below the nosepiece (not shown; a shoulder or other barrier (not shown) prevents the plunger **(101)** from exiting the nosepiece.

The hole or slot **(101A)** in the plunger **(101)** is narrow enough to prevent substance leakage during storage, and wide enough to allow compressed gas passage during activation, wiping the substance from the container during activation. The hole or slot **(101A)** in the plunger **(101)** can be designed in many ways to allow delivery that is very efficient, having a residual volume of less than 15% of the original volume. The plunger **(101)** can be made either from a flexible materials such as, but not limited to, silicone, rubber, flexible plastic or from a hard material such as, but not limited to, a polymer such as Delrin^{®}, a plastic, nylon, metal and any combination thereof.

**Fig. 6B** shows ball-type barriers **(102)** between compartments. The balls **(102)** provide both a separation function, before activation, and a mixing function during activation. In this exemplary embodiment, there are 3 balls **(103).** In other embodiments, more or fewer balls **(103)** can be present. When the device is activated, compressed gas (curved arrows at bottom) enters the capsule **(10).** The pressure forces the balls **(102)** upward, forcing substance above the topmost ball **(102)** out of the top of the capsule. The topmost ball **(102)** passes through the top of the capsule into an intermediate space **(10A)** below the nosepiece (not shown; a shoulder or other barrier (not shown) prevents the balls **(102)** from exiting the nosepiece. The substance between the first and second balls can then pass through the top of the capsule **(10)** into the nosepiece (not shown, and mix with the first substance. The second ball **(102)** can then enter the intermediate space **(10A),** and similarly with all balls **(102)** in the capsule **(10)** until the capsule **(10)** is empty.

Ball-type barriers **(102)** are useful when mixing of several components should occur only upon delivery, when one or more substance should be maintained at low humidity, when the viscosity of the substance varies significantly, and any combination thereof. In addition, contact between the ball **(102)** and the walls of the capsule **(10)** can also ensure effective release of the substance from the capsule **(10).** Examples of substances which tend to cling to walls include, but are not limited to, oils and some powders. The barriers can be balls, as in the embodiment shown, angular dividers or any other shape which can be easily moved by the released compressed gas (low-friction contacts), and still provide effective sealing between the elements to avoid mixing during, for example, shipment and storage.

**Fig. 6C** shows an embodiment with linked drug containers **(103)** within the capsule **(10).** In this exemplary embodiment, there are 3 linked drug containers **(103).** In other embodiments, more or fewer linked drug containers **(103)** can be present. The linked drug containers **(103)** are sealed by frangible membranes. A single frangible membrane can seal the top of one drug container **(103)** and the bottom of the adjacent drug container **(103),** separate frangible membranes **(103)** can be used for adjacent ends of drug containers, and any combination thereof. When the device is activated, compressed gas (curved arrows at bottom) enters the capsule **(10).** The pressure bursts the membranes, allowing mixing and exit into the nosepiece of the substance s within the linked drug containers **(103).**

In a preferred embodiment, each drug containers **(103)** is made of a soft thin sheet. The sheet can be a polymeric membrane, a continuous sheet or any other form which is thin enough to be easily torn when desired by the released of the compressed air. All drug containers **(103)** are connected to each other during manufacturing. Mixing occurs only during activation, with the compressed gas tearing the membranes/sheets dividing the compartments. Once the membranes are torn, the substance s are exposed to the compressed gas, mixed and delivered.

**Fig. 6D** shows an embodiment with sets of two-layer membranes **(104A, 104B)** within the capsule **(10).** In this exemplary embodiment, there are 4 sets of two-layer membranes **(104A, 104B).** In other embodiments, more or fewer sets of two-layer membranes **(104A, 104B)** can be present. The lower membrane **(104B)** is reticulated, with portions separable from each other, and the upper membrane **(104A),** frangible. When the device is activated, compressed gas (curved arrows at bottom) enters the capsule **(10).** The pressure causes the separable portions of the lower membrane **(104B)** to rotate upward, tearing the upper membrane **(104A)** and allowing mixing and exit into the nosepiece of the substance s within the capsule **(10).**

This embodiment differs from the previous one in that: (a) the drug containers do not form one unit; (b) the separate zones are separated from each other by membrane which is composed of two layers: one provides the rigidity of the membrane and is made of a rigid material, and the other one is a continuous flexible sheet which seals against the lower rigid part during until activation and which opens when air is pressed against its lower side The membranes **(104A, 104B)** open only one way, when air presses against their lower side during activation, allowing mixing of the substances during delivery.

**Fig. 6E** shows an embodiment with duckbill valves **(105)** within the capsule **(10).** In this exemplary embodiment, there are 4 duckbill valves **(105).** In other embodiments, more or fewer duckbill valves **(105)** can be present. When the device is activated, compressed gas (curved arrows at bottom) enters the capsule **(10).** The pressure causes the duckbill valves **(105)** to rotate upward, allowing exit and mixing of the substance s within the capsule **(10).**

**Fig. 6F** shows an embodiment with frangible membranes **(105)** within the capsule **(10).** In this exemplary embodiment, there are 4 frangible membranes **(105).** In other embodiments, more or fewer frangible membranes **(105)** can be present. When the device is activated, compressed gas (curved arrows at bottom) enters the capsule **(10).** The pressure causes the frangible membranes **(105)** to tear, allowing mixing and exit into the nosepiece (not shown) of the substance s within the capsule **(10).**

**Fig. 6G** shows an embodiment with bendable membranes **(106)** within the capsule **(10).** In this exemplary embodiment, there are 4 bendable membranes **(106).** In other embodiments, more or fewer bendable membranes **(106)** can be present. When the device is activated, compressed gas (curved arrows at bottom) enters the capsule **(10).** The pressure causes the bendable membranes **(106)** to rotate upward (curved arrows in middle) about connection points between the bendable membranes **(106)** and the capsule **(10)** wall, allowing mixing and exit into the nosepiece (not shown) of the substances within the capsule **(10).**

These exemplary embodiments allow holding the substances separate during storage and mixing the substances only upon activation and delivery. In some embodiments, the device or the substances therein can be configured to generate a temperature change, either heating or cooling, during mixing and delivery. The device can further be configured so that components for creating a temperature change in the device are not released with the delivered substances.

Heating and cooling can be triggered by mechanical force, by pressure, by chemical reaction and any combination thereof. This can be done inside the drug capsule, around the drug capsule, or outside the device itself in its packaging, to be triggered right before activation of the device.

Such temperature change can be generated during activation (short time temperature change) or prior to activation (long time temperature change). Long time temperature changes require a temperature activation separated from the delivery activation.

Either option, or at least the long time temperature change, further requires proper device sealing to allow temperature to be maintained inside the device and to allow equilibration prior delivery. Such options can further include a temperature indicator, such as by a color change in a dedicated control window, to allow the user to know that the device is ready for activation.

A temperature change can be an increase in temperature, a decrease of temperature, or both.

A temperature change can be useful for example for:
- Substance mixing
- Dissolution of one substance in another
- Absorption of a substance or mixture of substances in tissue, for example, a delivery temperature regulated with respect to the temperature of the nasal passages.
- Effective scattering of a substance or mixture of substances on tissue, for example, to create a flat, thin, uniform layer in the nasal passages and hence improve absorption
- Affect the viscosity of a substance or mixture of substances (both increase and decrease of viscosity can occur).
- Affect nature of a substance or mixture of substances. For example: polymerization can be initiated only during delivery, or during or after contact with tissue.

One embodiment comprises two heating agents. These heating agents are in compartments of a capsule. Upon activation of the device, or upon activation of heating (for example, buy pressing a button), a membrane separating the two compartments is torn, allowing the heating agents to mix and to generate heat within the device. Other membranes are not torn by this activity, which keeps the heating agents in a sealed compartment - sealed so as to prevent delivery of heating agent delivery but allow gas passage to other compartments. Passage of the compressed gas then delivers the heated substances or other desired substances. Mixing, as disclosed above, can occur during delivery.

**Fig. 7A** shows an embodiment with sets of two-layer membranes **(104A, 104B)** and a mixing ball **(102)** within the capsule **(10).** In this exemplary embodiment, there are 2 sets of two-layer membranes **(104A, 104B)** and a single mixing ball **(102)** at the top of the capsule **(10).** In other embodiments, more or fewer sets of two-layer membranes **(104A, 104B)** and more or fewer mixing balls **(102)** can be present; the mixing balls **(102)** can be at any desired location within the capsule **(10).** The lower membrane **(104B)** is reticulated, with portions separable from each other, and the upper membrane **(104A),** frangible. When the device is activated, compressed gas (curved arrows at bottom) enters the capsule **(10).** The pressure causes the separable portions of the lower membrane **(104B)** to rotate upward, tearing the upper membrane **(104A)** and allowing mixing and exit into the nosepiece of the substances within the capsule **(10).** Further mixing is provided by the mixing ball **(102).** As disclosed above, a shoulder or other stopper in the nosepiece (not shown) prevents the mixing ball **(102)** from exiting the nosepiece (not shown).

**Fig. 7B** shows an embodiment with duckbill valves **(105)** and a mixing ball **(102)** within the capsule **(10).** In this exemplary embodiment, there are 2 duckbill valves **(105)** and a single mixing ball **(102)** at the top of the capsule **(10).** In other embodiments, more or fewer duckbill valves **(105)** can be present and more or fewer mixing balls **(102)** can be present; the mixing balls **(102)** can be at any desired location within the capsule **(10).** When the device is activated, compressed gas (curved arrows at bottom) enters the capsule **(10).** The pressure causes the duckbill valves **(105)** to rotate upward, allowing exit and mixing of the substances within the capsule **(10).** Further mixing is provided by the mixing ball **(102).** As disclosed above, a shoulder or other stopper in the nosepiece (not shown) prevents the mixing ball **(102)** from exiting the nosepiece (not shown).

**Fig. 7C** shows an embodiment with frangible membranes **(105)** and a mixing ball **(102)** within the capsule **(10).** In this exemplary embodiment, there are 4 frangible membranes **(105)** and a single mixing ball **(102)** at the top of the capsule **(10).** In other embodiments, more or fewer frangible membranes **(105)** can be present and more or fewer mixing balls **(102)** can be present; the mixing balls **(102)** can be at any desired location within the capsule **(10).** When the device is activated, compressed gas (curved arrows at bottom) enters the capsule **(10).** The pressure causes the frangible membranes **(105)** to tear, allowing mixing and exit into the nosepiece (not shown) of the substances within the capsule **(10).** Further mixing is provided by the mixing ball **(102).** As disclosed above, a shoulder or other stopper in the nosepiece (not shown) prevents the mixing ball **(102)** from exiting the nosepiece (not shown).

**Fig. 7D** shows an embodiment with bendable membranes **(106)** and a mixing ball **(102)** within the capsule **(10).** In this exemplary embodiment, there are 4 bendable membranes **(106)** and a single mixing ball **(102)** at the top of the capsule **(10).** In other embodiments, more or fewer bendable membranes **(106)** can be present and more or fewer mixing balls **(102)** can be present; the mixing balls **(102)** can be at any desired location within the capsule **(10).** When the device is activated, compressed gas (curved arrows at bottom) enters the capsule **(10).** The pressure causes the bendable membranes **(106)** to rotate upward (curved arrows in middle) about connection points between the bendable membranes **(106)** and the capsule **(10)** wall, allowing mixing and exit into the nosepiece (not shown) of the substances within the capsule **(10).** Further mixing is provided by the mixing ball **(102).** As disclosed above, a shoulder or other stopper in the nosepiece (not shown) prevents the mixing ball **(102)** from exiting the nosepiece (not shown).

**Fig. 7E** shows an embodiment with two half balls **(102).** In this exemplary embodiment, there is one pair of half-balls **(102).** In other embodiments, more pairs of half-balls **(102)** can be present. When the device is activated, compressed gas (curved arrows at bottom) enters the capsule **(10).** The pressure causes the half-balls **(102)** to move upward. They will separate and tumble as they move, allowing gas to pass between and around them thus mixing and delivering the substance. As disclosed above, a shoulder or other stopper in the nosepiece (not shown) prevents the mixing ball **(102)** from exiting the nosepiece (not shown).

**Fig. 7F** shows an embodiment with two attached mixing balls **(102).** In other embodiments, more mixing balls **(102)** can be present. When the device is activated, compressed gas (curved arrows at bottom) enters the capsule **(10).** The pressure causes the mixing balls **(102)** to move upward, thus causing efficient mixing of the substances. As disclosed above, a shoulder or other stopper in the nosepiece (not shown) prevents the mixing ball **(102)** from exiting the nosepiece (not shown).

The mixing balls need not be spherical; any shape that will provide good sealing during storage and low-friction movement during activation can be used.

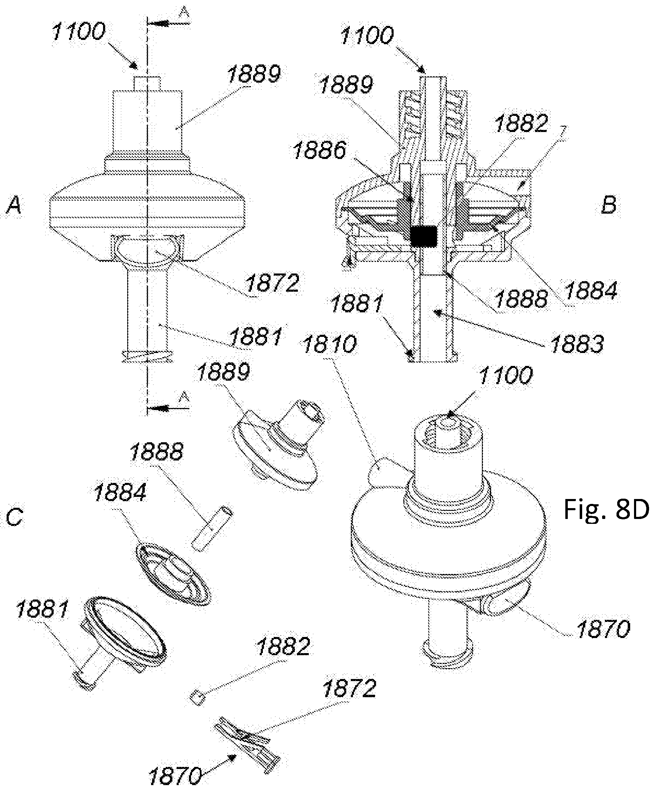

**Figs. 8-9** show exemplary embodiments of the loading and triggering region of embodiments of devices with mechanical triggering mechanisms, all of which are configured to open fully, quickly and reproducibly, with the time over which the valve opens being reproducible, independent of how the user may operate the device. For example, in the suction devices described herein, a weak suction will induce the same full opening over the same time period as a strong suction, and, in the mechanical devices disclosed herein a slow activation of the triggering mechanism will induce the same full opening over the same time period as a rapid activation of the triggering mechanism.

In some embodiments, the loading region of the device comprises at least one filter to remove from the air (or other gas) at least one selected from a group consisting of particles, particulates, bacteria, viruses, moisture, and undesired gases before the air contacts the user.

Preferably, the air or gas is filtered on entrance to the air chamber from the outer environment (the room, the surrounding area). Alternatively or additionally, air can be filtered on exit from the air chamber, while within the loading air chamber, and any combination thereof.

Fig. 8A-D shows a preferred embodiment of the loading portion of the device **(1000)** with a pinch triggering mechanism. **Fig. 8A** shows a side view of the device, **Fig. 8B** shows a cross-section, taken along the line AA in **Fig. 8A, Fig. 8C** shows an exploded view, and **Fig. 8D** shows a perspective view.

The device comprises a hollow upstream portion **(1881)** fluid-tightly connected to a hollow downstream portion **(1889).** In this embodiment, the activation mechanism **(1880)** comprises a cup-shaped insert **(1884)** fitting snugly and fluid-tightly within the hollow interior of the device. The outer rim of the insert **(1884)** is preferably fixed to the outer wall of the activation mechanism **(1880),** with its inner rim able to slide on an inner wall **(1886),** preferably tubular, of the activation mechanism **(1880).** In the activation mechanism's **(1880)** closed position, a stop **(1882)** is firmly held by the inner rim of the insert.

The inner wall of the activation mechanism **(1880)** comprises a throughgoing bore **(1883).** In some variants of this embodiment, a flexible tube **(1888)** is fluid-tightly fixed to the wall **(1886)** such that there is flexible tubing in at least the portion of the wall abutting the stop **(1882).** In other variants of this embodiment, the flexible tube **(1888)** passes through the bore **(1883).**

In preferred variants of this embodiment of an activation mechanism, in the closed position, the stop **(1882)** fits into and sits in a hole in the inner wall **(1886).** In other variants, the stop **(1882)** fits into and sits in a depression in the inner wall **(1886).**

When the activation mechanism **(1880)** is in the closed position, the flexible tube **(1888)** is pinched between the stop **(1882)** and the inner side of the throughgoing bore **(1883).**

When the activation mechanism **(1880)** is activated, the insert **(1884)** slides up along the wall, releasing the stop **(1882)** so that the pinched region in the flexible tube **(1888)** is released, thereby releasing the pressurized gas and dispensing the substance.

In the embodiment shown in **Fig. 8****,** the activation mechanism can be activated either by sucking on the suction mechanism **(1810),** creating a partial vacuum above the cup-shaped insert **(1884)** and pulling it upward, thereby releasing the stop **(1882),** or by pressing the pressable lever **(1870).** Pressing the pressable lever **(1870)** forces it inward so that the ramp portion **1872** of the pressable lever pushes the cup-shaped insert **(1884)** upward, thereby releasing the stop **(1882),** releasing the pressurized gas and dispensing the substance.

In some embodiments, flexible filling material such as, but not limited to, flexible tubing, can be placed within the region of the device (not shown) containing the substance to be delivered in order to reduce dead space within the device. Reducing dead space will not affect the characteristics of the aerosol formed after release, but it will decrease pressure loss and increase air speed within the device, thereby substantially reducing residual substance remaining within the device after completion of activation, either within the capsule or adhering to the interior walls of the device, e.g., within the nozzle. It is well known in the art that residual material within a delivery device can be released on subsequent uses of the device and that the amount of such residual material released during a given use of a device is extremely variable. Therefore, minimizing residual substance within the device will increase the accuracy and reproducibility of delivery, thereby increasing its repeatability and reliability, both by maximizing the fraction of the substance actually delivered from the current capsule and by minimizing the amount of residual substance on the walls of the device.

It should be noted that the capsules (disclosed hereinbelow) are designed so as to avoid residual volume within the capsule itself, since, even in the case of a single dose or disposable capsule there are safety issues involved in disposing of capsules containing residual amounts of hazardous drugs or other hazardous component in the composition.

Other trigger mechanisms include, but are not limited to, a releasable catch, a pressable button a detectable predetermined sound pattern, a detectable predetermined light pattern, a moveable lever, a slider moveable from a first position to a second position, a rotatable knob is rotated, a releasable latch configured and any combination thereof.

The predetermined sound pattern can be: a constant-pitch sound, a varying-pitch sound, a constant volume sound, a varying volume sound and any combination thereof.

The predetermined light pattern can be: a constant-color light, a varying-color light, a constant brightness light, a varying brightness light and any combination thereof.

In some embodiments, the device comprises a unidirectional valve such that gas can flow from the charging mechanism to the delivery end, but is unable to flow in the reverse direction.

In some embodiments, a substance to be dispensed (which can comprise any number of materials) can be stored within a capsule, either as the substance to be dispensed or as a precursor or precursors, with the capsule placeable within the device, as described hereinbelow. In such embodiments, the capsule is ruptured during activation, either all at once or in stages, thereby dispensing the substance.

In other embodiments, a substance, prepared in a conventional matter, is introducible into a holding chamber within the device and, on activation of the device, the substance is dispensed. Embodiments of this kind can be used as emergency dispensing devices, since any flowable substance can be introduced into the holding chamber and since the holding chamber, which has no facilities for separating precursors or for providing an inert atmosphere in the chamber, is not intended for long-term storage of substances.

In some embodiments, the capsule chamber in which the capsule can be placed can also function as a holding chamber, so that the substance can be dispensed either from the capsule or directly from the holding chamber.

In other embodiments, an insert can be placed within the capsule chamber, with the interior of the insert being a holding chamber.

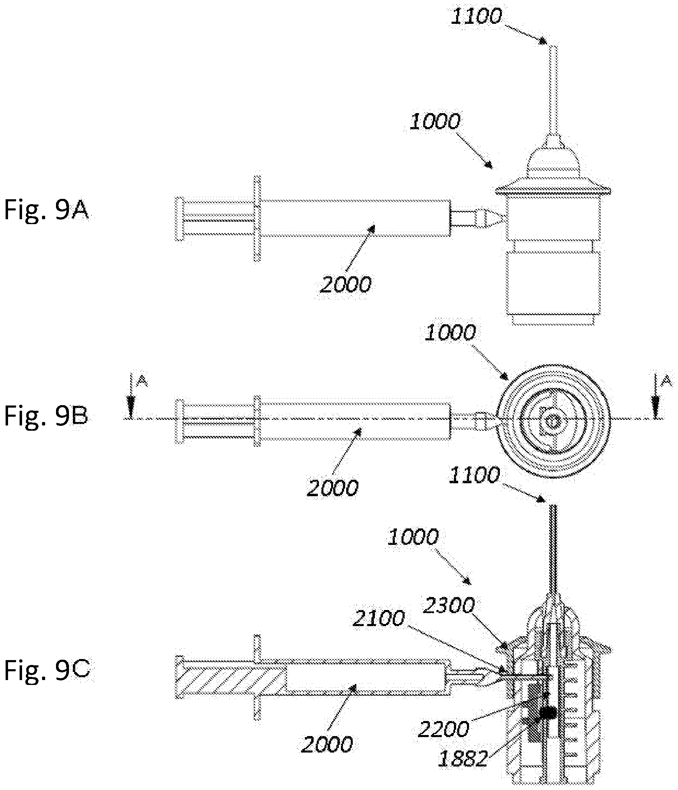

An embodiment of the activation mechanism a dispensing device **(1000)** into which any flowable substance is introducible is shown in Fig. 9A-C. The charging mechanism is not shown. **Fig. 9A** shows a side view of the embodiment, **Fig. 9B** shows a top view of the embodiment, and **Fig. 9C** shows a cross-section, taken along the line AA in **Fig. 9B****.**

In this embodiment, the means of loading the substance into the device is a syringe **(2000).** The syringe **(2000)** can be placed in the injection port **(2100,** **Fig. 9C****)** and the syringe plunger depressed so that the flowable substance enters a dispensing chamber **(2200)** within the device **(1000).** Before, during or after injection of the substance into the chamber, the device can be charged, in any manner described herein, using any activation mechanism described herein or known in the art.

In some embodiments, the syringe is left in the injection port. In other embodiments, a cover (**2300**) is provided for the injection port, so that, after loading the substance into the chamber, the injection port can be sealed by means of the cover. As shown in the embodiment of **Fig. 9****,** the cover **(2300)** can slide longitudinally onto and off the injection port **(2100),** In other embodiments, it can rotate or spiral around the device to cover or uncover the injection port **(2100),** it can rotate around a hinge on the body of the device so that it flips onto and off the injection port **(2100),** or any other method of sealing the port can be used. In the embodiment as shown, in the open position, the syringe goes through a hole in the cover in order to reach the chamber. Any combination of the above embodiments can be used in a cover.

In some embodiments, the substance is stored in a capsule or in a sealed compartment in the device. Before or during activation, the capsule or sealed compartment is breached and pressure on the capsule (e.g., by pressing a button to move the piston of a built-in syringe) forces the contents into a dispensing chamber **(2200).** Dispensing gas passing through the dispensing chamber **(2200)** then entrains the substance and delivers it.

In some embodiments of a device with separate storage chamber and holding chamber, the capsule comprises a syringe or a syringe like compartment, a rubber piston and seals. The longitudinal axis of the syringe and piston are at right angles to the longitudinal axis of the device. Pressure on the piston moves the substance from the syringe into the holding chamber, in a manner similar to the syringe **(2000)** and holding chamber **(2200)** in **Fig. 9****.**

In the embodiment shown, a pinch triggering mechanism is used, as shown hereinabove in **Fig. 8****,** although any of the other activation mechanisms described herein or any conventional valve known in the art can be used.

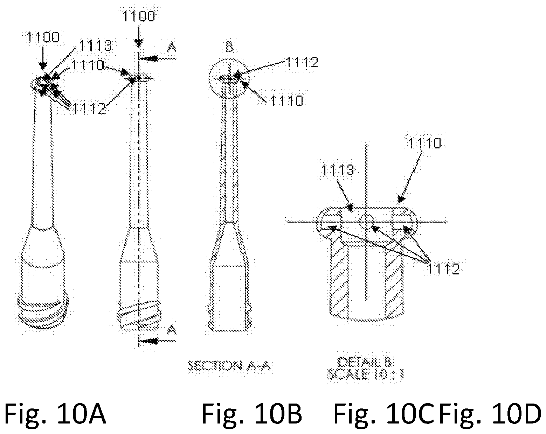

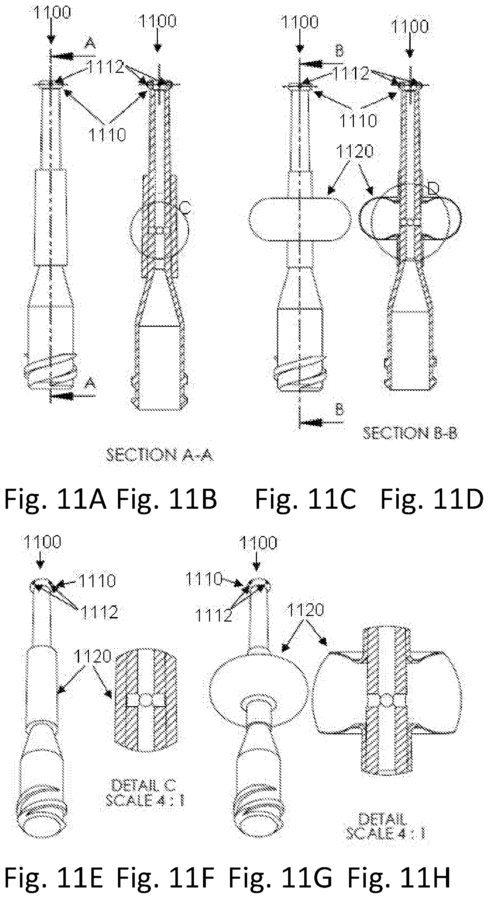

In reference to **Figs. 9-11****,** three exemplary embodiments of nozzles **(1100)** are shown. In both **Fig. 10** and **Fig. 11****,** the nozzle **(1100)** has a tip extension **(1110)** with a larger diameter than the nozzle, the tip extension substantially surrounding the distal end of the nozzle **(1100).** In **Fig. 9****,** the nozzle tip is substantially conical, lacking the optional tip extension **(1110).**

In the exemplary embodiment of both **Fig. 10** and **Fig. 11****,** the tip extension **(1110)** has holes (**1112**) in it to allow substance to exit laterally from the extension, and the tip **(1110)** has at least one hole **(1113)** in its distal end to allow substance to exit longitudinally from the nozzle **(1100).** Fig. 10A-D shows an embodiment of a nozzle **(1100)** with a tip extension **(1110).** **Fig. 10A** shows a perspective view of the nozzle **(1100)** from the distal end, while **Fig. 10B** shows a side view. **Fig. 10C** shows a cross-section of the nozzle along the line AA in **Fig. 10A****,** while **Fig. 10D** shows an enlarged view of the circled region B at the tip of the nozzle in **Fig. 10C****,** showing the tip of the nozzle and the tip extension in more detail. The holes **(1112)** in the tip extension **(1110)** and the hole **(1113)** in the tip can be clearly seen. In some embodiments, the nozzle **(1110)** has only lateral holes **(1112),** so that no substance escapes from the distal end of the nozzle **(1110).**

In preferred embodiments, the distal end of the tip extension does not comprise any longitudinal protuberances, being substantially flat in the area around the opening **(1113)** and, where nonplanar, extending proximally from the plane of the opening.

In order to prevent material from escaping from the nasal passages or entering undesired areas in the nasal cavity, in some embodiments, the nozzle comprises a medial extension, an expandable portion **(1120).** **Fig. 11** shows an embodiment of a nozzle with a tip extension (**1110**) and an expandable portion **(1120).** **Figs. 11E** and **11G** show perspective views of the nozzle from the proximal end, while **Figs. 11A** and **11C** show side views of the nozzle **(1100).** **Figs. 11B** and **11D** show cross-sections of the nozzle **(1100)** along the lines AA in **Fig. 11A** and BB in **Fig. 11C****,** respectively. **Fig. 11F** shows an enlarged view of the circled region C in the center of the nozzle in **Fig. 11B****,** while **Fig. 11H** shows an enlarged view of the circled region D in the center of the nozzle in **Fig. 11D****.**

**Figs. 11A, 11B, 11E** and **11F** show the nozzle with unexpanded expandable portion, while **Figs. 11C, 11D, 11G** and **11H** show the nozzle with expanded expandable portion.

In the exemplary embodiments of **Figs. 10-11****,** the tip extension and the expanded medial extension are substantially toroidal; in other embodiments, they can be substantially spherical, substantially ovoid, substantially ellipsoidal, substantially the frustum of a cone (preferably with a rounded distal edge), substantially conic (preferably with a rounded distal edge) and any combination thereof.

The nozzle tip and the tip extension **(1110)** have a number of holes **(1112, 1113)** which fluidly connect the bore of the nozzle **(1100)** to the exterior of the device, allowing material to exit from the interior of the device. In the exemplary embodiments shown, there is a hole **(1113) (****Fig. 10A** and **C;** not shown in **Fig. 11****)** in the distal end of the nozzle and four holes **(1112)** in the tip extension **(1100).** Both the extension and the distal end of the nozzle can have more or fewer holes and, in some embodiments, one or the other can have no holes. The holes **(1112)** can be regularly spaced around the periphery of the extension, the holes **(1112)** can be irregularly spaced around the periphery, the holes **(1112)** can be concentrated in a predetermined part of the periphery, and any combination thereof. Similarly, the holes in the distal end of the tip can be regularly or irregularly spaced in the tip.

In some embodiments, the extension **(1110)** can be padded, can comprise soft material, can comprise flexible material and any combination thereof.

Extensions, both tip extensions and medial extensions, can have a number of functions. A non-limiting list of such functions is (1) ensuring proper positioning of the nozzle **(1100)** in the nasal passages, where the proper position can be the nozzle **(1100)** centralized in the nasal passages, the nozzle **(1100)** touching a predetermined portion of the nasal passages, or the nozzle **(1100)** closer to a predetermined portion of the nasal passages, (2) sealing the nasal passages so that material can not escape therefrom, (3) sealing the nasal passage so that substance does not contact undesired portions thereof, (4) sealing the nasal passage so that substance remains in a predetermined region of the nasal passage, (5) reducing the discomfort of contact between the nozzle and the nasal passages, especially in embodiments where the extension is intended to seal against the walls of the nasal passages, by providing a soft and/or flexible contact region and any combination thereof. Proper positioning can be for the purpose of improving delivery of a substance to a predetermined area, preventing clogging of the holes by nasal secretions, preventing clogging of the holes by contact with the nasal passages, mucosa and any combination thereof.

Nozzle extensions, both those that are expanded during the activation procedure and those that have a predetermined shape and do not expand, can either (1) be attached to the nozzle in a way that they are removed from the nasal cavity with the nozzle tip itself, or (2) have the option of being releasable from the nozzle tip so that they stay in the nasal cavity until they are pulled out by the user or by a caregiver, or any combination thereof. In embodiments where at least one nozzle extension remains in a nasal cavity, preferably, the nozzle extension or extensions are removed after a predetermined time, preferably a short time.

In some embodiments, the holes **(1112)** in the nozzle **(1100)** do not lie substantially in a plane perpendicular to the main longitudinal axis of the nozzle **(1100).** In such embodiments, the holes **(1112)** can lie along a line parallel to the main longitudinal axis of the nozzle **(1100),** along a line forming a spiral around the nozzle **(1100),** irregularly in the distal portion of the nozzle **(1100),** regularly spaced in the distal portion of the nozzle **(1100),** and any combination thereof.

Therefore, dispersion of the drug can be substantially from a ring perpendicular to the main longitudinal axis of the nozzle **(1100)** (holes **(1112)** around the edge of the extension **(1110),** from a circle perpendicular to the main longitudinal axis of the nozzle **(1100)** (holes **(1113)** in the distal tip of the nozzle **(1100),** from a line (holes **(1112)** parallel to the main longitudinal axis of the nozzle **(1100)** or in a spiral around the main longitudinal axis of the nozzle **(1100),** or from at least part of the surface of a volume extending along the side of the nozzle **(1100).**

In some embodiments, the size of the tip extension **(1110)** is selected so that the extension **(1110)** is in contact with the nasal passages substantially along its entire circumference. In such embodiments, material exiting holes **(1113)** in the distal tip of the nozzle **(1100)** or holes **(1112)** on the distal face of the extension **(1110)** can not reach regions proximal to the extension (**1110**) and will reach only regions deeper in the nasal passages than the extension **(1110).** In such embodiments, the substance will reach the upper parts of the nasal passages.

Material exiting from holes **(1112)** in locations where the extension **(1110)** is in contact with the nasal passages will deposit directly on the walls of the nasal passages. In such embodiments, deposition is in a very narrow band; the location of the band can be tailored for the material of interest.

Material exiting holes **(1112)** proximal to the region of the extension **(1110)** in contact with the walls of the nasal passages will be unable to reach locations distal to the region of the extension (**1110**) in contact with the walls of the nasal passages and will therefore deposit in the lower parts of the nasal passages.

Returning to **Fig. 11****,** in this embodiment, the expandable portion **(1120)** surrounds the nozzle (1100). In other embodiments, the expandable portion **(1120)** can partially surround the nozzle (1100). A single expandable portion **(1120)** or a plurality of expandable portions **(1120)** can be used. An expandable portion can be on the surface of the nozzle or it can be stored within the nozzle, popping out when it expands. An expandable portion can have a predetermined shape when expanded. The shape of the outward-facing part of an expandable portion can be part of the surface of a spheroid, can be part of a cylinder, a part of a cone, or can conform to the shape of a predetermined portion of a nasal passage. Such shaping can help ensure that, on inflation, the expandable portion or portions gently guide the nozzle so that it rests in the position with respect to the nasal passages or in the correct portion of the nasal passages. It can also reduce the user's discomfort when the device is in place or, if detachable from the device, it can seal the nasal passage for a time, before being removed by the user or a caretaker.

The expandable portion **(1120)** is preferably inflated after insertion of the device into the nasal passage. Inflation can be before or at the time of activation of the device.

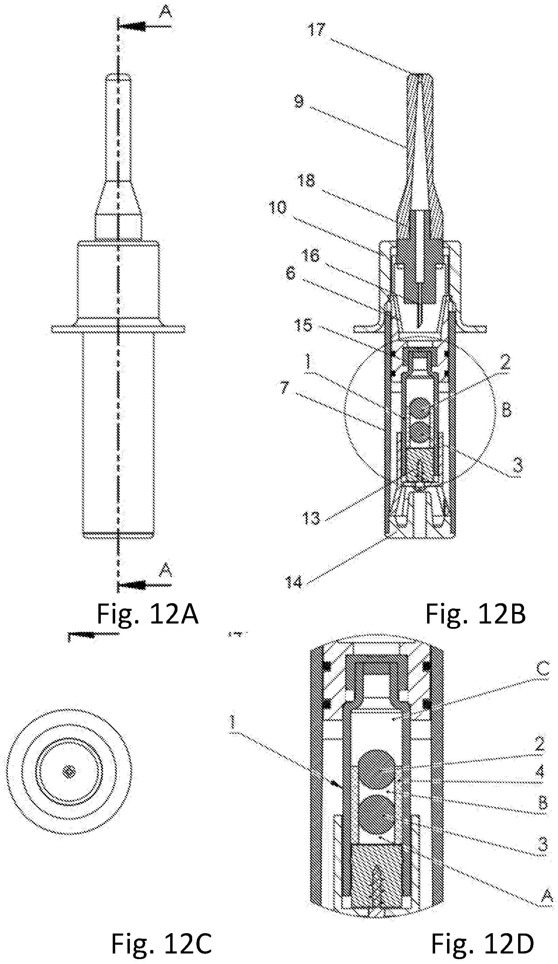

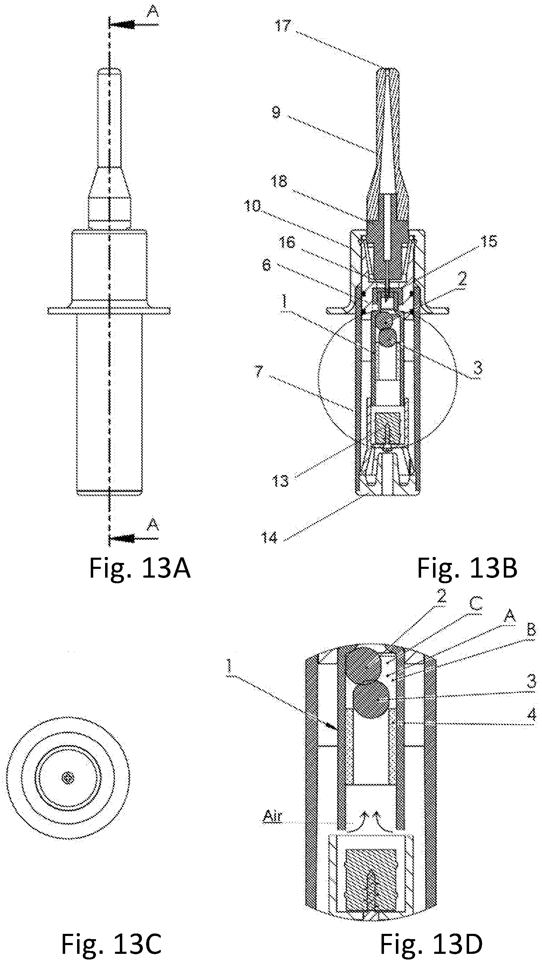

**Figs. 12-13** show embodiments of a device with ball-type **(2)** drug container dividing and mixture elements. The nose piece is not shown. With two dividing elements, 3 substances can be stored in the device prior to delivery. Fig. 12A-D shows the device before activation, while Fig. 13A-D shows the device after activation.

**Fig. 12A** shows the exterior of the device from the side, while **Fig. 12C** shows the exterior of the device from the top. **Fig. 12B** shows a cross-section of the device along the line **A-A** in **Fig. 12A****,** while **Fig. 12D** shows an enlarged view of the area within the circle B in **Fig. 12B****.**

As shown in the cross-section of **Fig. 12B****,** inside the body (7) is a drug container housing **(1)** and, within that, a drug container **(4)** configured to contain three separate substances. Between the drug container housing **(1)** and the body **(7)** is compressed gas. The drug container **(4)** configured to contain three substances, **A, B and C;** substances **A, B and C** being separated by dividing and mixing elements **(2, 3).** The base **(14)** comprises an integral plunger anchor, with the plunger anchor anchored into a drug container plunger **(13)** at the proximal end of the drug container **(4).** Sealing the drug container **(4)** at its distal end is a drug container cover **(15),** preferably made of a biocompatible material. The drug container **(4)** is prevented from moving distally by a drug container holder **(6),** with the drug container holder **(6)** clasping the neck of the drug container **(4)** and preventing it from moving. The activation button **(10)** slideably covers the drug container holder **(6),** the distal end of the body **(7),** and the proximal end of the nose piece **(9).** Before activation, a flange, the drug container holder stopper at the distalmost end of the drug container holder sits in the activation rib slot. A needle **(16)** is configured to pierce the drug container cover **(15)** during activation. A nose piece **(9)** is attached to the distal side of the body **(7)** and passes through the activation button **(10).** The distal end of the nose piece **(9)** comprises an aerosol release orifice **(17)** to allow passage of an aerosol from the interior of the device to its exterior.

**Fig. 12C** shows a view of the device of **Fig. 12A****,** from the top.

Fig. **12D** shows an enlarged view of the area inside the circle B in the cross-section shown in **Fig. 12B****,** before activation. The gap between the drug container housing **(1)** and drug container (4) can be seen more clearly. Inside the drug container **(4),** substances **A** and **B are** separated by dividing/mixing element 3, while substances **B and C** are separated by dividing/mixing element 2. The proximal end of the drug container housing **(1)** surrounds the drug container plunger **(13,** see **Fig. 12B****),** sealing the proximal end of the drug container **(4),** Fig. 13A-D shows the embodiment of the device of Fig. 12A-D after activation.

**Fig. 13A** shows the exterior of the device from the side, while **Fig. 13C** shows the exterior of the device from the top. **Fig. 13B** shows a cross-section of the device along the line **A-A** in **Fig. 13A****,** while **Fig. 13D** shows an enlarged view of the area within the circle B in **Fig. 13B****.**

As shown in the cross-section of **Fig. 13B****,** inside the body **(7)** is a drug container housing **(1)** and, within that, a drug container **(4).** Before activation, between the drug container housing (**1**) and the body **(7)** was compressed gas, which exited its storage area via the drug container **(4).** After activation, the dividing and mixing elements **(2, 3)** have substantially exited the drug container **(4),** but are prevented by the drug container holder **(6)** from entering the nose piece **(9).** The dividing and mixing elements **(2, 3)** cannot either block the nose piece **(9)** or exit the device and therefore are prevented from entering the body of a user. The base **(14)** with plunger anchor anchored into the drug container plunger **(13,** see **Fig. 12B****)** is now separate from the proximal end of the drug container **(4),** allowing the compressed gas to enter the base of the drug container (4); the arrows show the direction of gas movement. The drug container cover **(15),** has been pierced by the needle **(16),** allowing an aerosol to form from the gas and the substances, during activation, pass through the nose piece **(9),** through the aerosol release orifice **(17)** and pass from the interior of the device to its exterior.

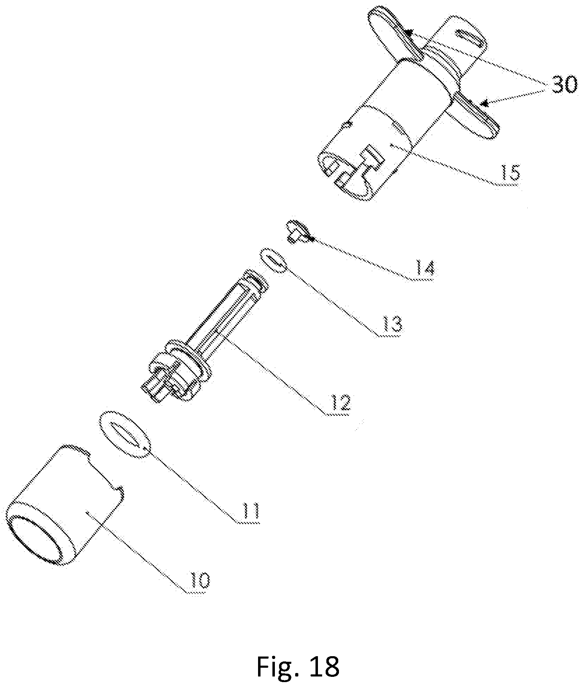

In some embodiments of the device, the substances can also be held inside the nosepiece, as demonstrated in **Figs. 18-20****.**

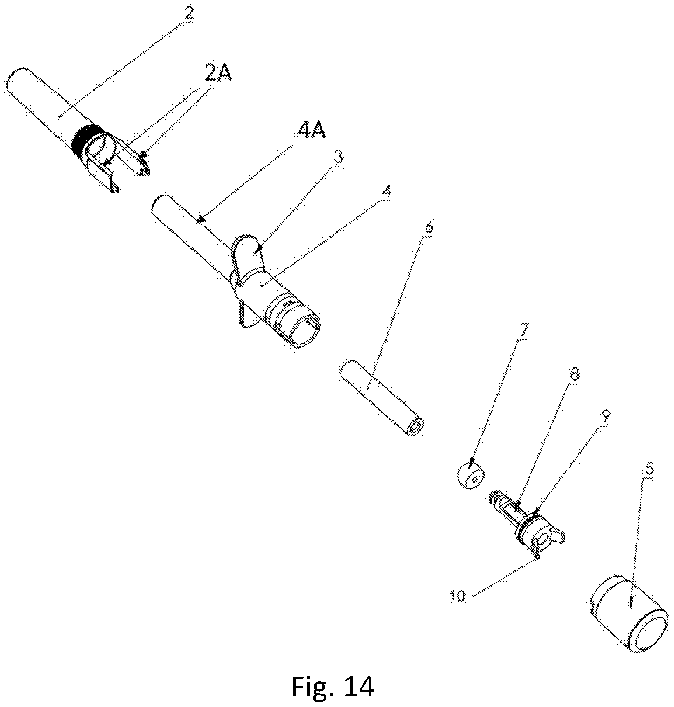

**Fig. 14** shows an exploded view of an embodiment of the device. Inside the base **(5)** is an air chamber gate **(8)** for control of passage of gas from the compressed gas chamber **(4).** The air chamber gate **(8)** comprises a stopper **(10)** at its proximal end, with the stopper **(10)** resting against a shoulder in the base **(5),** with the shoulder preventing proximal movement of the air chamber gate **(8);** and a gate O-ring **(9)** proximal to the air chamber gate **(8)** to ensure an air-tight seal between the air chamber gate **(8)** and the base **(5).** A septum **(7)** surrounds the distal end of the air chamber gate **(8),** providing an air-tight seal between the base unit **(5)** and the compressed gas chamber **(4).** The device comprises a drug chamber **(6),** which can contain a single-substance or multiple substances, as disclosed above; a compressed gas chamber **(4),** activation holders **(3)** and a nosepiece **(4A),** and a nosepiece cover **(2)** with activation safety locks **(2A)** to prevent both accidental activation of the device and loss of the cove during storage or transport. In preferred embodiments, the compressed gas chamber **(4),** activation holders **(3)** and nosepiece **(4A)** form an integral unit; in other embodiments, at least one of the compressed gas chamber **(4),** activation holders **(3)** and nosepiece **(4A)** is a separate unit, joined to the others by any means known the art.

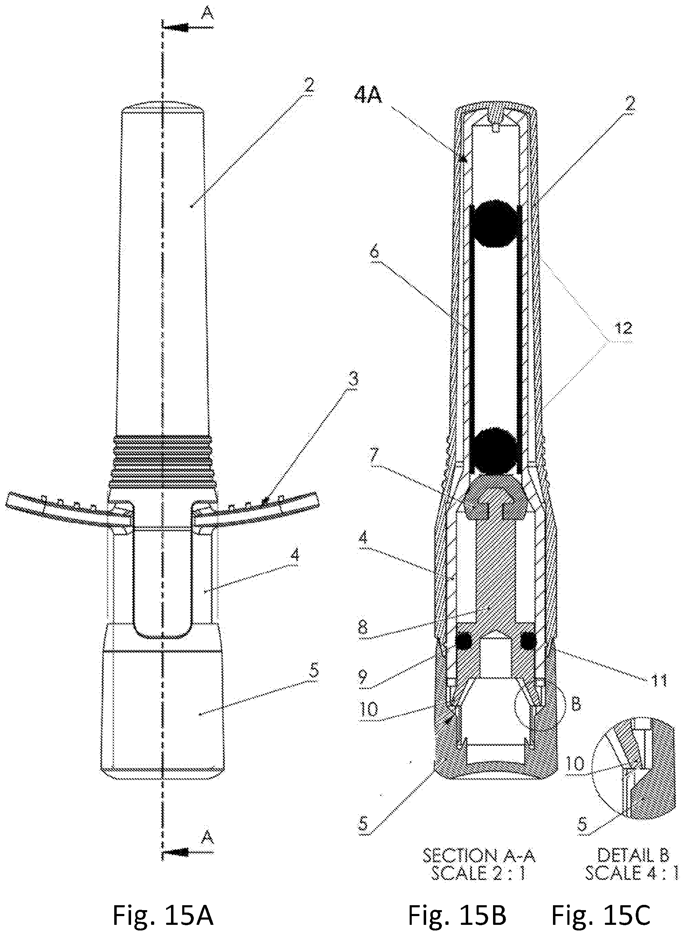

**Fig. 15B** shows a cross-section of the device of **Fig. 14****,** before activation, with the cross-section taken along the line A-A of **Fig. 15A****,** which shows the exterior of the device, illustrating the nose piece cover **(2),** activation holders **(3),** compressed air chamber **(4)** and the activation mechanism base **(5).**

**Fig. 15C** is an enlarged view of the area inside the circle B in **Fig. 15B****,** showing the stopper, resting on a shoulder in the base unit **(5).**

The device is shown with a substance container containing substance in the nose piece. The device comprises a nose piece cover **(2)** with an activation safety lock **(2),** activation holders **(3),** a compressed air chamber **(4)** and an activation mechanism base **(5).** After the nose piece cover with its activation safety lock **(2,** see **Fig. 14****)** is removed, activation is allowed. The at least one substance is held inside the drug container **(6),** with the drug container **(6)** sealed in place by a septum **(7)** at its proximal end and by a protruding element at distal end of the nose piece cover **(2),** which blocks the aerosol release orifice at the distal end of the nose piece **(4A);** the nose piece cover **(2)** being held in place by the activation safety lock. At the proximal end of the device are the activation mechanism base **(5),** a unit which has two zones/functions- one is the air chamber gate **(8)** and the other one is the stopper **(10),** and a gate O-ring **(9).** The at least one substance is held in the drug container **(6)** between the drug container dividing and mixture elements **(12).** The compressed air is locked between the compressed air chamber wall **(4)** and the air chamber gate **(8).** Removal of the nose piece cover and activation safety lock **(2)** releases the safety lock anchor **(11)** and allows activation. Upon activation, the stopper **(7)** is pressed inward, allowing the air chamber gate **(8)** to slide proximally, since the proximal area of the stopper exposed to the pressure is greater than the distal area of the stopper exposed to the same pressure. A gap is generated between the septum **(7)** and the drug container **(6),** allowing air passage through the at least one substance and generation of aerosol. In embodiments where drug container dividing and mixture elements **(12)** are used, they are released to a wider zone in the drug container **(6)** to allow substance mixing and release, contact between the substances and the air and aerosol formation and delivery.

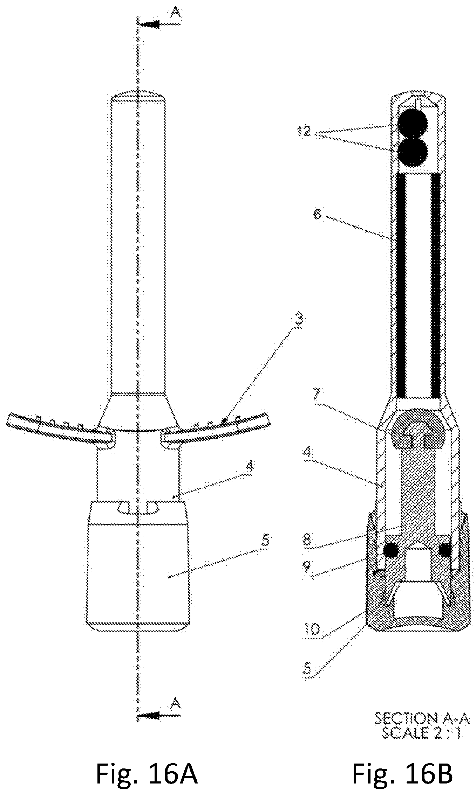

**Fig. 16B** shows a cross-section of the device of **Figs. 14-15****,** after activation, with the cross-section taken along the line A-A of **Fig. 16A****,** which shows the exterior of the device, illustrating the activation holders **(3),** compressed air chamber **(4)** and the activation mechanism base **(5).**

After activation, the dividing and mixture elements **(12)** have been moved to a holding chamber at the tip of the nose piece and the drug container **(6)** is empty. The air chamber gate **(8)** has been moved proximally by the air pressure so that the stopper **(5)** rests against the interior of the bottom of the base **(5),** leaving a gap between the septum (7) and the compressed air chamber **(4).** The gate O-ring **(9)** still forms a seal around the stopper.

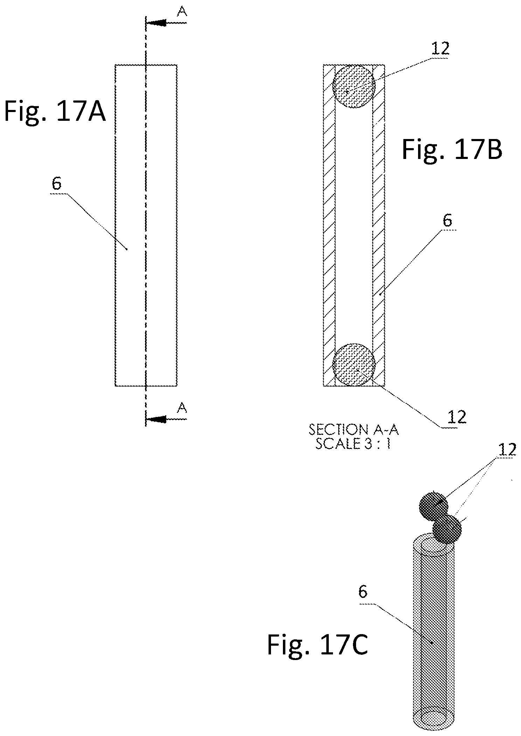

Fig. 17A-C illustrates an embodiment of a drug container **(6).** **Fig. 17A** shows the exterior of the container **(6),** with **Fig. 17B** showing a cross-section of the drug container **(6)** before activation taken along the line A-A of **Fig. 17A. Fig. 17C** shows the exterior of the drug container after activation. In **Fig. 17B****,** there is one dividing and mixing unit **(12)** at each end of the drug container **(6),** thus effectively sealing the drug container **(6).** In **Fig. 17C****,** both dividing and mixing units **(12)** have been displaced from the interior of the drug container **(6.)**

**Fig. 18-29** illustrate embodiments of devices with a dose-adjustable drug chamber, where it is possible for a user to adjust the amount of medicament delivered in each dose, either for a single-use device or for a multi-use device.

**Fig. 18** shows an embodiment of the body of a nasal delivery device. The nosepiece is not shown. The body comprises a base **(10),** an air chamber gate **(12)** with a first gate O-ring **(11)** at its proximal end and a second gate O-ring **(13)** at its distal end. The first gate O-ring **(11)** corresponds to the gate O-ring of the embodiments of **Figs. 14-17** and the second gate O-ring corresponds to the septum of **Figs. 14-17****.** The distal end of the air chamber gate **(12)** is covered by a drug container base cover **(14)** which comprises a biocompatible material to ensure that substance that is to contact living tissue only contacts biocompatible material before the contact with living tissue. The compressed gas chamber **(15)** will fit over the air chamber gate **(12),** with the first gate O-ring **(11)** and the second gate O-ring **(13)** providing airtight seals before activation so that compressed gas is storable between the air chamber gate **(12)** and the compressed gas chamber **(15).** The compressed gas chamber **(15)** is connectable at its distal end with a nose piece (not shown). The distal portion of the compressed gas chamber **(15)** comprises activation holders **(30)**

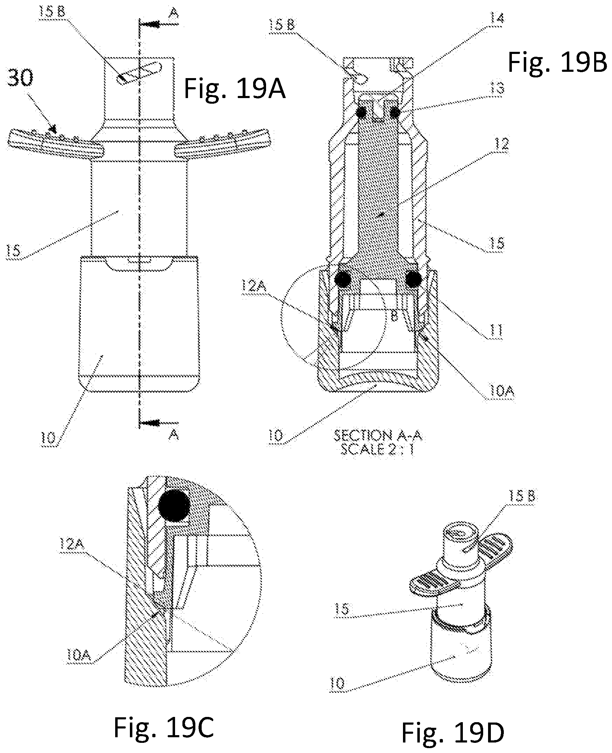

Fig. 19A-D shows an embodiment of the body of Fig. **18****,** as assembled, before activation. **Fig. 19A** shows the exterior of the body, while. **Fig. 19B** shows a cross-section taken along the line A-A in **Fig. 19A. Fig. 19C** is an enlarged view of the circled section B in **Fig. 19B****,** while **Fig. 19D** is a perspective view of the body of **Fig. 19A****.** Activation is by compressing the upper end of the device toward its base, by holding the activation holders **(30)** with the fingers and the bottom of the base **(10)** with the thumb, and bringing the fingers toward the thumb.

As shown in **Figs. 19A** and **19D****,** in the embodiment of **Figs. 18-19****,** the base of the device forms the activation button **(10),** to activate, the activation button **(10)** is pressed upward while the compressed gas chamber (gas chamber **(15)** is held stationary by fingers on the activation holders **(30).** The nosepiece is attachable to the compressed gas chamber **(15)** by means of the nose piece connector slot **(15B);** a protuberance on the nose piece engages with the nose piece connector slot **(15B);** permitting fast and easy replacement of the nose piece.

As shown in **Fig. 19B****,** the activation button **(10)** comprises a gate anchor **(10A),** a shoulder on which the air chamber gate stopper **(12A)** rests before activation. This to prevent movement of the air chamber gate (12) before activation. The first gate O-ring **(11),** at the proximal end of the gate anchor **(10A)** and the second gate O-ring **(13),** at its distal end, provide airtight seals before activation so that compressed gas is storable between the air chamber gate **(12)** and the compressed gas chamber **(15).** The distal end of the air chamber gate **(12)** is covered by a drug container base cover **(14)** which comprises a biocompatible material to ensure that substance that is to contact living tissue only contacts biocompatible material before the contact with living tissue. The compressed gas chamber **(15)** is connectable at its distal end with a nose piece (not shown) by means of the nose piece connector slot **(15B).**

**Fig. 19C****,** the enlargement of the area within the circle B of **Fig. 19B** , clearly shows the gate anchor **(10A),** with the air chamber gate stopper **(12A)** resting on it.

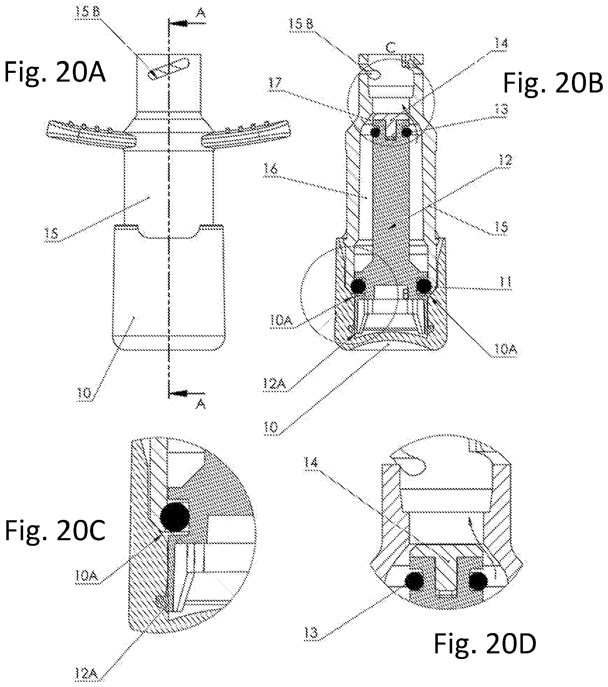

Fig. 20A-D shows an embodiment of the body of Fig. **18****,** as assembled, after activation. **Fig. 20A** shows the exterior of the body, while. **Fig. 20B** shows a cross-section taken along the line A-A in **Fig. 20A. Fig. 20C** is an enlarged view of the circled section B in **Fig. 20B****,** while **Fig. 20D** is an enlarged view of the circled section C in **Fig. 20B****.** Activation is by compressing the upper end of the device toward its base, by holding the activation holders **(30)** with the fingers and the bottom of the base **(10)** with the thumb, and bringing the fingers toward the thumb.

As shown in **Figs. 19A** and **19D****,** in the embodiment of **Figs. 18-18****,** the base of the device forms the activation button **(10),** to activate, the activation button **(10)** is pressed upward while the compressed gas chamber (gas chamber **(15)** is held stationary by fingers on the activation holders **(30).** The nosepiece is attachable to the compressed gas chamber **(15)** by means of the nose piece connector slot **(15B);** a protuberance on the nose piece engages with the nose piece connector slot **(15B);** permitting fast and easy replacement of the nose piece.

As shown in **Fig. 19B****,** the activation button **(10)** comprises a gate anchor **(10A),** a shoulder on which the air chamber gate stopper **(12A)** rested before activation. During activation, the air chamber gate stopper **(12A)** is pressed inwards, so that the air chamber gate **(12)** moves proximally, opening up a gap **(17)** between the air chamber gate **(12)** and the distal end of the compressed gas chamber **(15),** allowing the gas **(16)** to exit the compressed gas chamber **(15)** through the gap, and to enter the nosepiece and forma an aerosol with the substance. The first gate O-ring **(11),** at the proximal end of the gate anchor **(10A)** still provide an airtight seal after activation, but the second gate O-ring **(13),** at its distal end, so that compressed gas is storable between the air chamber gate **(12)** and the compressed gas chamber **(15)** is no longer in contact with the compressed gas chamber **(15).** The distal end of the air chamber gate **(12)** is covered by a drug container base cover **(14)** which comprises a biocompatible material to ensure that substance that is to contact living tissue only contacts biocompatible material before the contact with living tissue. The compressed gas chamber **(15)** is connectable at its distal end with a nose piece (not shown) by means of the nose piece connector slot **(15B).**

**Fig. 20C****,** the enlargement of the area within the circle B of **Fig. 20B****,** clearly shows the gate anchor **(10A),** with the air chamber gate stopper **(12A)** no longer in contact with it, but is resting near the base of the activation button.

**Fig. 20D****,** the enlargement of the area within the circle C of **Fig. 20B****,** clearly shows the distal end of the air chamber gate **(12),** the drug container base cover **(14),** the second gate O-ring **(13)** and the gap (arrow) permitting air to escape from the compressed gas chamber into the intermediate space and then to the nose piece (not shown)..

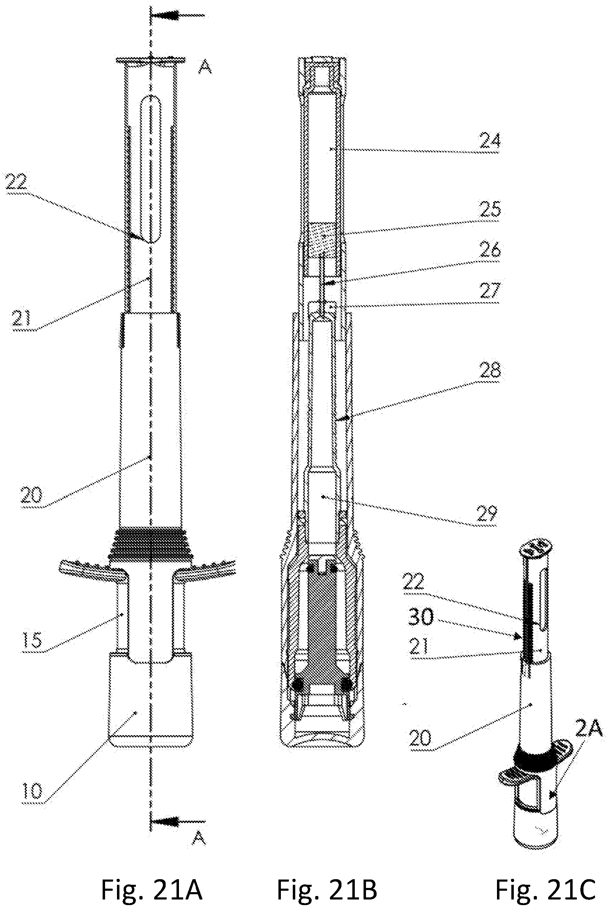

Fig. 21A-C shows an embodiment of the device with the primary drug container in the nose piece cover. The medicament or substance is delivered from the primary drug container to a secondary drug volume in the nose piece. The nose piece cover can then be removed and the device activated to aerosolize and deliver the drug.

**Fig. 21A** shows the exterior of the device. It comprises an activation button **(10),** a compressed gas chamber **(15),** and a nosepiece (not shown) protected by a nose piece cover **(20).** At the distal end of the nose piece cover **(20)** is a primary drug container (not shown) in a drug container housing **(21).** The embodiment also comprises an indicator window **(22)** to determine the quantity of drug remaining in the primary drug container.

**Fig. 21B** shows a cross-section of the device, taken along the line A-A in **Fig. 21A****.** The base and aerosol generation and delivery mechanism are similar to those disclosed above in **Figs. 21-24****.** The device comprises a nose piece **(28)** with an integral drug volume **(29)** in its proximal portion. The deliverable substance is stored in a primary drug container **(24)** near the distal end of the nose piece cover **(20).** The primary drug container **(24)** is sealed at its proximal end by a plunger stopper **(25).** A loading needle **(26)** is fixed to the nose piece cover **(20),** with a needle adaptor **(27)** to guide the loading needle's **(26)** proximal end so that the proximal end of the needle adaptor **(27)** passes through the orifice at the tip of the nose piece **(28).** As shown in **Fig. 21B****,** unless drug is being loaded into the integral drug volume **(29),** the needle does not pierce the plunger stopper **(25)** and the primary drug container **(24)** remains sealed.

**Fig. 21C** shows a perspective view of the device. The indicator window **(22)** and the drug container housing **(21)** can be seen, as well as the nose piece cover **(20),** which terminates at its proximal end in a safety lock **(2A)** to prevent unwanted activation of the device.

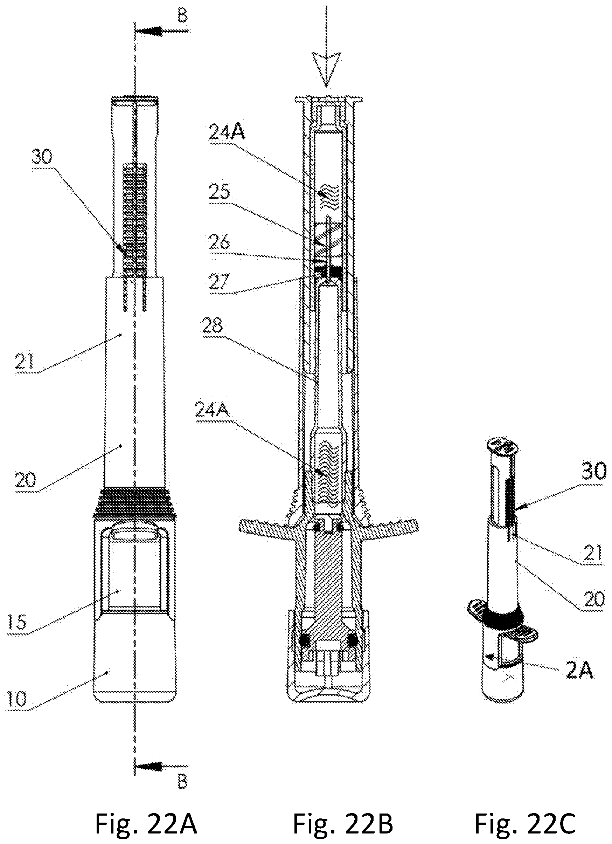

Fig. 22 A-C shows the embodiment of the device of **Fig. 21** during loading of the drug into the integral drug volume **(29)** from the primary drug container **(24).**

**Fig. 22A** shows the exterior of the device. It comprises an activation button **(10),** a compressed gas chamber **(15),** and a nosepiece (not shown) protected by a nose piece cover **(20).** At the distal end of the nose piece cover **(20)** is a primary drug container (not shown) in a drug container housing **(21).** The embodiment also comprises a volume scale **(30),** here a ratchet, to allow adjustment of the size of the dose.

**Fig. 22B** shows a cross-section of the device, taken along the line B-B in **Fig. 22A****.** The base and aerosol generation and delivery mechanism are similar to those disclosed above in **Figs. 17-20****.** As shown in **Fig. 22B****,** a drug or medicament **(24A)** is being loaded from the primary drug container into the integral drug volume **(29).** Loading is activated by pressing the primary drug container **(24)** proximally. It then slides along the nose piece **(28).** The loading needle (26) is steadied by the needle adaptor **(27).** Pressing the primary drug container **(24)** proximally forces the loading needle **(26)** through the plunger stopper **(25)** and into the primary drug container **(24).** Drug **(24A)** can then flow through the loading needle **(26)** into the integral drug volume **(29).** Releasing the primary drug container **(24)** will cause it to move distally and remove the loading needle **(26)** from the plunger stopper **(25).** The nose piece cover **(20)** can then be removed and a dose of the drug can be administered.

**Fig. 22C** shows a perspective view of the device. The volume scale **(30)** and the drug container housing **(21)** can be seen, as well as the nose piece cover **(20),** which terminates at its proximal end in a safety lock **(2A)** to prevent unwanted activation of the device.

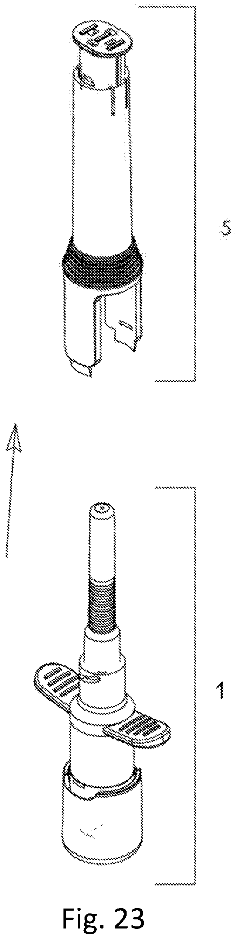

**Fig. 23** illustrates removal of the nose piece cover or medicine chamber **(5)** from an aerosol delivery device **(1)** by pulling (arrow) the medicine chamber **(5)** away from the aerosol delivery device **(1),**

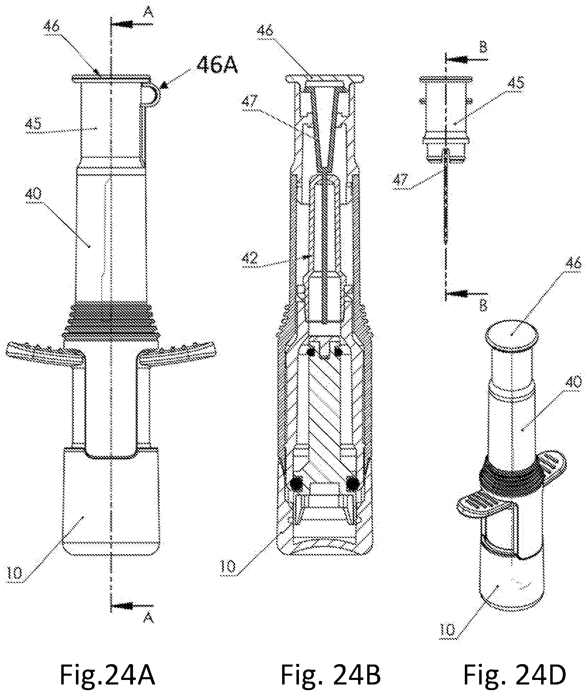

Fig. 24A-D illustrates a device which can be loaded with a medicament, drug or substance via a syringe. **Figs. 24A** and **24D** show the exterior of the device, **Fig. 24A** from the side and **Fig. 24D** from an angle. **Fig. 24B** shows a cross section taken along the line A-A in **Fig. 24A** and Fig. 24C shows the loading needle.

As shown in **Fig 24A****,** the device comprises an activation button **(10)** and compressed gas chamber, as disclosed above. The nose piece cover **(40)** comprises a drug loading adaptor **(45)** and a reversibly removable drug loading adaptor cap **(46)** at its distal end. In the embodiment shown, the drug loading adaptor cap **(46)** is attached to the nose piece cover **(40)** by an integral flexible strip **(46A),** to prevent the drug loading adaptor cap **(46)** from getting lost.

As shown in **Fig 24A****,** a drug loading needle **(47)** is held firmly within the drug loading adaptor **(45).** The drug loading needle **(47)** extends from the top of the nose piece cover **(40)** through the distal end of the nose piece **(42)** to a drug storage volume near the proximal end of the nose piece **(42).** The distal portion of the drug loading needle **(47)** is configured by means of shape and size to accept the delivery end of a syringe (not shown). During storage and transport, the drug loading needle **(47)** is retained firmly in place with its distal portion help firmly between the closed drug loading adaptor cap **(46)** and the distal tip of the nose piece **(42).**

Fig. 24C shows the drug loading adaptor **(45)** with the drug loading needle **(47)** extending proximally therefrom.

**Fig. 24D** shows the nose piece cover **(40),** the drug loading adaptor cap **(46)** and the drug delivery deice with activation button **(10).**

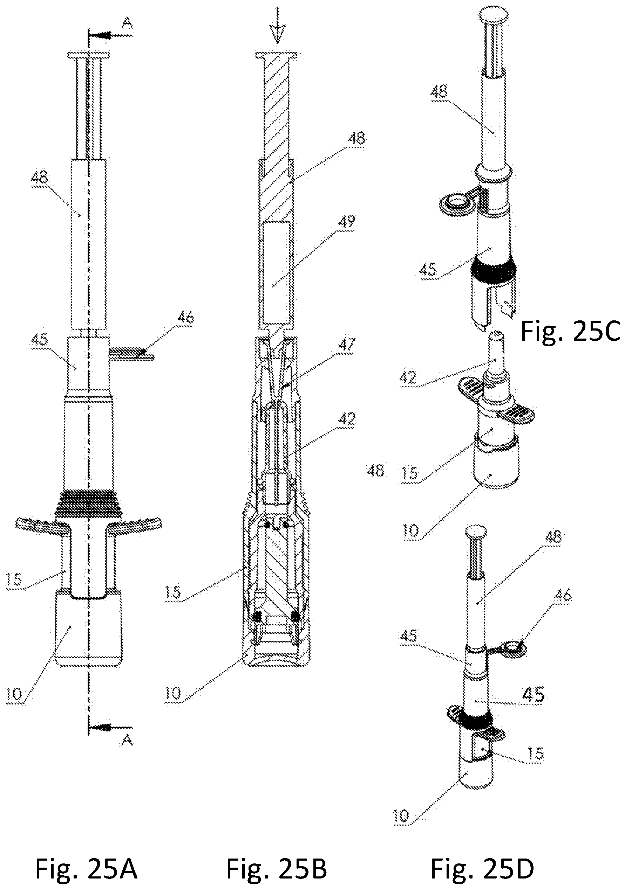

Fig. 25A-D shows the device of Fig 24A-D with a syringe in place. The syringe can be a proprietary syringe, with a tip matched in shape and size to the opening in the distal portion of the drug loading needle **(47)** or it can be a commercial syringe with a tip that fits into the opening in the distal portion of the drug loading needle **(47).**

**Fig. 25A** shows a side view of the device with a labeled **(49)** loading syringe **(48)** in place. The drug loading adaptor cap **(46)** is open and the tip (not shown) of the loading syringe **(48)** is resting in the distal portion of the drug loading adaptor **(45)** and nose piece cover **(40),** with the nose piece cover in communication with the activation button **(10)** and compressed gas chamber **(15)** of the delivery device.

**Fig. 25B** shows a cross-section of the set-up of **Fig. 25A****,** taken along the line A-A of **Fig. 25A****.** The loading syringe **(48)** is resting in the distal portion of the drug loading needle **(47).** The proximal portion of the drug loading needle **(47)** passes through the nose piece **(42).** The nose piece **(42)** is attached, either reversibly or fixedly, to the activation button **(10)** and compressed gas chamber **(15)** of the delivery device.

Fig. 25C-D shows how a loading syringe **(48)** in place in a drug loading adaptor **(45),** is connectable to a drug delivery device, comprising nose piece **(42),** compressed gas chamber **(15)** and activation button **(10).** **Fig. 25C** shows the loading syringe **(48)** in place in a drug loading adaptor **(45),** with the drug loading adaptor **(45)** in position to be attached to the delivery device. **Fig. 25** **D** shows the loading syringe **(48)** and drug loading adaptor **(45),** with the drug loading adaptor cap **(46)** open, attached to the compressed gas chamber **(15)** and activation button **(10)** of the delivery device.

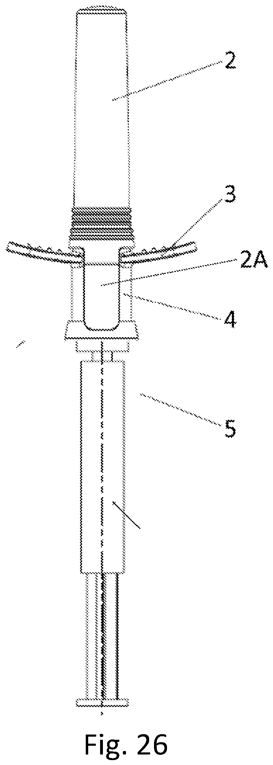

**Fig. 26** shows an embodiment of delivery device where adjustment of the size of the dose is made from the proximal end - the opposite end of the device from the adjustment mechanisms disclosed above. The nose piece is covered by a nose piece cover **(2)** with a safety lock **(2A)** to prevent accidental activation of the device. The safety lock **(2A)** is latched to the compressed gas chamber **(4)** of the delivery device. Proximal to the compressed gas chamber **(4)** is an activation mechanism base **(5).The** activation holders **(3)** are also shown.

For many medicines, one dose is supplied to each nostril, with the patient receiving two doses altogether. In the prior art, for a single-dose delivery device, this required two delivery devices, with the consequent waste of packaging material, waste of time spent unpacking two devices, both of which tend to reduce patient compliance.

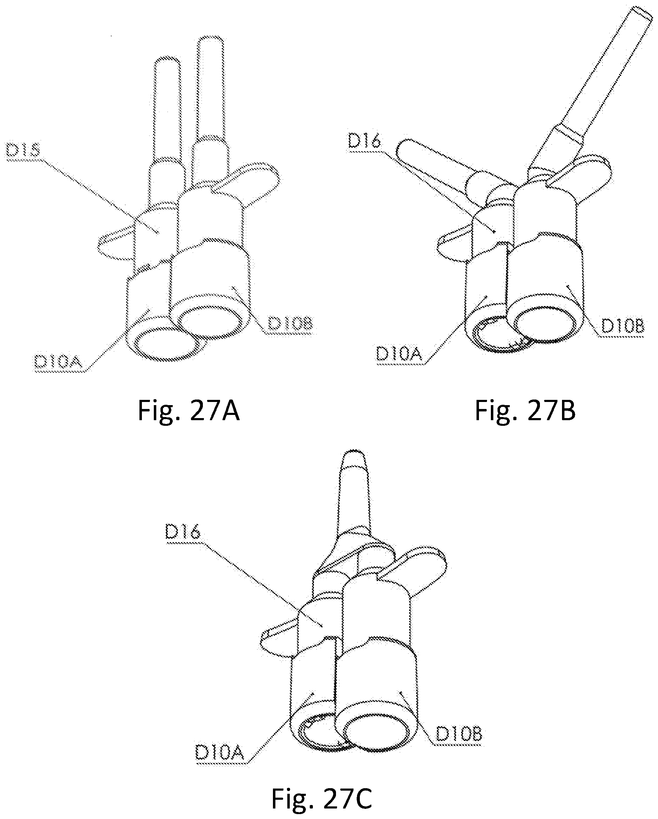

Fig. 27A-C shows embodiments of a devices configured to supply a single dose of a medicament to each of two nostrils. The devices **(D15** and **D16)** of **Figs. 27A** and **27B** have two independent aerosolization and delivery devices **(D10A** and **D10B),** each containing a single dose of a drug, and each of which is in independent fluid communication with a nosepiece. In **Fig. 27A****,** the nosepieces are parallel to each other, whereas in **Fig. 27B****,** the nosepieces are at approximately right angles to each other.

The device **(D16)** of **Fig. 27C****,** also comprises two independent single-dose aerosolization and delivery devices **(D10A** and **D10B),** but both of these are in communication with a single nosepiece.

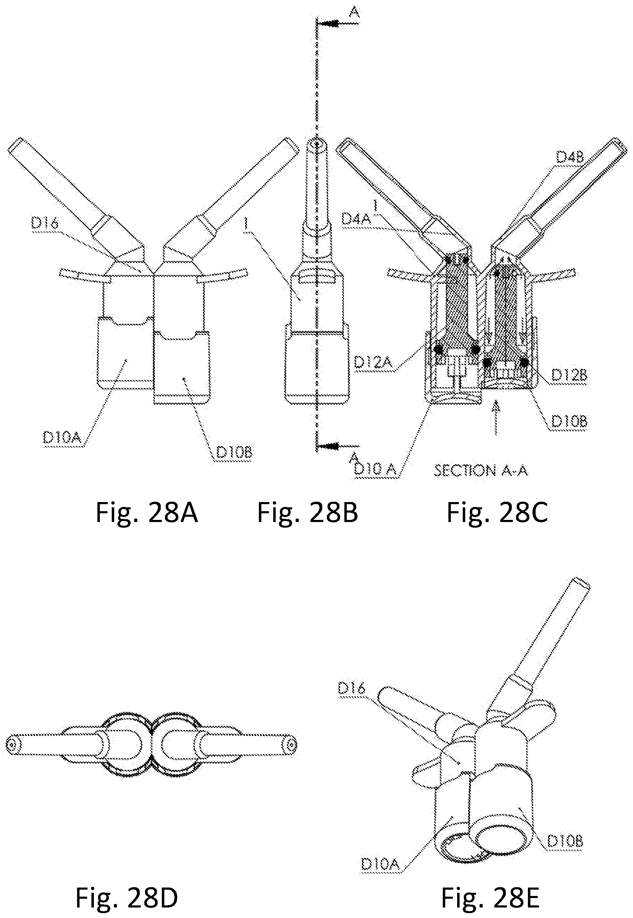

Fig. 28A-E shows a front view **(****Fig. 28A****),** a side view **(****Fig. 28B****),** a cross-section view **(****Fig. 28C****)** a top view **(****Fig. 28D****),** and a perspective view **(****Fig. 28E****)** of an embodiment with nose pieces at approximately right angles to each other **(****Fig. 27B****).**

The device **(D16)** comprises two independent aerosolization and delivery devices **(D10A** and **D10B),** each in fluid connection with a single nosepiece. Each aerosolization and delivery device **(D10A** and **D10B)** comprises a single dose of a drug, which can comprise a single substance or a plurality of substance, stored as a mixture or stored in independent compartments, as disclosed above. The device also comprises activation holders; the aerosolization and delivery devices **(D10A** and **D10B)** will be activated one at a time, as disclosed above, with fingers on the activation holders; and a thumb on the activation button at the base of an aerosolization and delivery device **(D10A** or **D10B).** It can be seen from **Figs. 28B** and **28D** that the nose pieces of the device **(D16)** lie substantially in the same plane in this embodiment; in other embodiments, they could lie in different planes.

**Fig. 28C** is a cross-section through the device, along the line of A-A in **Fig. 28B****.** In **Fig. 28C****,** one aerosolization and delivery device **(D10A)** has not yet been activated, while the other (**D10B**) has been activated (large upward arrow). In the non-activated delivery device **(D10A),** the air chamber gate **(D12A)** is in the distal position, with the distal end of the air chamber gate (**D12A**) blocking passage of gas into the mixing chamber **(D4A).** In the activated delivery device **(D10B),** the air chamber gate **(D12B)** has moved proximally (large downward arrows), leaving a gap between the distal end of the air chamber gate **(D12B)** and the mixing chamber (D4B) which allows passage of gas (small upward arrows) into the mixing chamber **(D4B)** from whence gas can pass through the nose piece and exit the device..

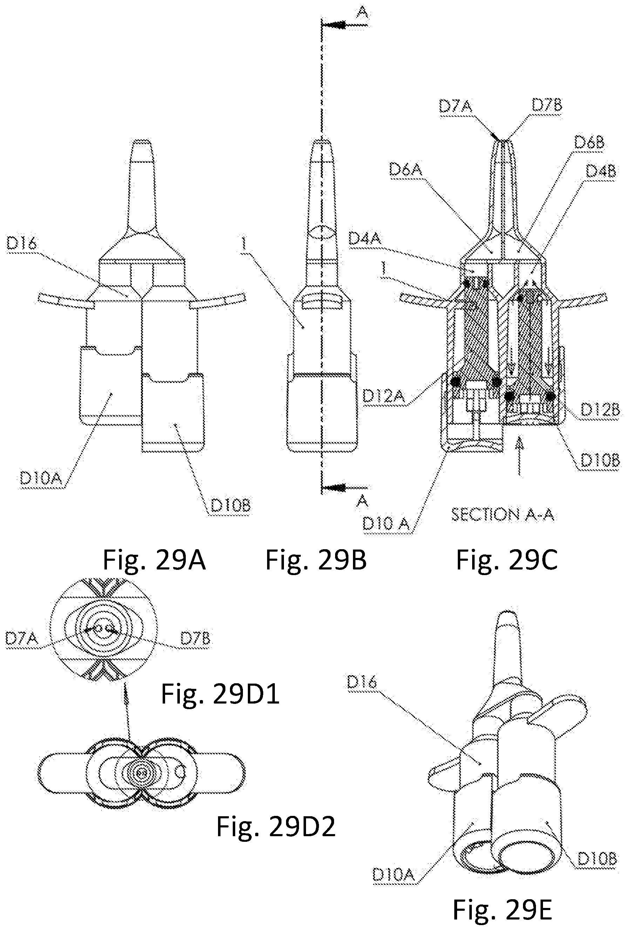

Fig. 29A-E shows a front view **(****Fig. 29A****),** a side view **(****Fig. 29B****),** a cross-section view **(****Fig. 29C****),** a top view (Fig. 29D2), an enlarged top view **(29D2)** and a perspective view **(****Fig. 29E****)** of an embodiment with a single nose piece **(****Fig. 27C****).**

The device **(D16)** comprises two independent aerosolization and delivery devices **(D10A** and **D10B),** and a single nosepiece, with both aerosolization and delivery devices **(D10A** and **D10B)** in fluid communication with the single nosepiece. Each aerosolization and delivery device **(D10A** and **D10B)** comprises a single dose of a drug, which can comprise a single substance or a plurality of substance, stored as a mixture or stored in independent compartments, as disclosed above. The device also comprises activation holders; the aerosolization and delivery devices **(D10A** and **D10B)** will be activated one at a time, ass disclosed above, with fingers on the activation holders; and a thumb on the activation button at the base of an aerosolization and delivery device **(D10A** or **D10B).**

**Fig. 29C** is a cross-section through the device, along the line of A-A in **Fig. 29B****.** As shown in **Fig. 29C****,** the single nosepiece comprises two mixing chambers **(D4A** and **D4B),** two sets of air passages **(D6A** and **D6B)** and two aerosol exits **(D7A** and **D7B)** allowing the aerosol to exit from the device. Therefore, although there is only one nose piece, substance from one of the delivery devices **(D10A** or **D410B)** will not come into contact, within the device, with substance from the other delivery device.

In **Fig. 29C****,** one aerosolization and delivery device **(D10A)** has not yet been activated, while the other **(D10B)** has been activated (large upward arrow). In the non-activated delivery device **(D10A),** the air chamber gate **(D12A)** is in the distal position, with the distal end of the air chamber gate **(D12A)** blocking passage of gas into the mixing chamber **(D4A).** In the activated delivery device **(D10B),** the air chamber gate **(D12B)** has moved proximally (large downward arrows), leaving a gap between the distal end of the air chamber gate **(D12B)** and the mixing chamber (D4B) which allows passage of gas (small upward arrows) into the mixing chamber **(D4B)** from whence gas can pass through the nose piece and exit the device..

Fig. 29D2 shows a top view of the device. In the enlarged view (Fig. 29D1), the two independent exits **(D7A** and **D7B)** can be clearly seen.

.It should be noted that the embodiments of the device are not limited to the exemplary embodiments shown above.

In embodiments where delivery is to a nostril, delivery of the substance can be improved by inducing sniffing in the user.

Sniffing (short, sharp breaths through the nose, for example, when smelling something) is highly correlated with soft palate (Velum) position. Sniffs are rapidly modulated in an odorant-dependent fashion by a dedicated olfactomotor system, and affect the position of the soft palate at the posterior end of the nasal cavity. When sniffing through the nose, the palate is in its upper position to cause separation between the nasal cavity and the oral cavity.

In addition to conscious control, sniffing may be reflexively elicited by chemicals, functioning as either irritants or odors in the nose. Overall sniff duration and pattern can be modulated in real time to optimize olfactory perception. When the olfactory system encounters a concentrated odorant, sniff vigor is reduced and sniff time is reduced; when it encounters a diluted odorant, sniff vigor is increased and duration lengthened. Odorant pleasantness also affects sniffing; sniff vigor and duration increase when smelling a pleasant odor and decrease when smelling an unpleasant odor.

In preferred embodiments, the device disclosed herein can release odorant into the nasal cavity of the user in order to reflexively elicit sniffing. The odorant can be a single odorant or a mixture of odorants and can comprise compounds from different chemical families, for non-limiting example:
- Esters: Geranyl Acetate, Ethyl Acetate, Benzyl Acetate, Octyl Acetate.
- Linear Terpens: Geraniol, Citral, Citronella, Nerolidol.
- Cyclic Terpens: Terpineol, Thujone.
- Aromatic: Eugenol, Vanillin, Anisole, Thymol.
- Amines: Indole.

Also aromatic compounds of alcohols, aldehydes, esters, ketones, lactones, and thiols.

In preferred embodiments, the substance is contained within a capsule. The capsule can have a single compartment or it can be multi-compartment. The capsule can contain a broad range of drugs and materials. The aromatic compound can be stored in the nozzle, or the nozzle or a portion thereof can be impregnated with aromatic compound, so as to trigger the closing of the velum when the nozzle tip is being placed in the nasal cavity. The delivery can be for local effect, to the systemic circulation, to the central nerve system (CNS), to the brain, preferably via the olfactory epithelium, to the spinal cord and associated nerves, and any combination thereof.

As described hereinabove, the drugs and materials to be delivered can be, but are not limited to, pharmaceuticals, natural compounds, biologics, hormones, peptides, proteins, viruses, cells, stem cells and any combination thereof.

The stored substance or substances can be stored as a liquid, an aerosol, a powder, a slurry, a suspension, or a gel, if thin enough. The substance or substances can be stored either with or without a carrier; the carrier can be a liquid, a gas or a powder.

The substance as delivered can comprise a powder, a mixture of liquid and powder, a mixture of gas and powder, a mixture of powders, a liquid, a mixture of liquid and gas, a mixture of liquids, a gas, or a mixture of gases.

The stored substance or substances can be packaged to minimize degradation, for example, by packaging it in vacuum or under an inert atmosphere. Preferably, capsules are single-use so that a single, controllable dose can be delivered with each use of the device. Capsules can be placed in the container of the device, or the container can comprise the capsule.

Use of an inert gas for the carrier for delivery of the medication obviates the possibility of interactions between the user and the delivery carrier; allergies to carriers, especially in medications used for chronic illnesses, are a growing problem. Furthermore, the delivery carrier is in contact with the medicament for no more than a few seconds and more commonly for no more than a few milliseconds, thereby minimizing degradation of the medicament due to interactions with the delivery carrier.

Examples of drugs and materials deliverable using the device are given hereinbelow. All examples listed below are exemplary and are not limiting.

Deliverable drugs and materials include: treatments for allergic rhinitis; treatments for osteoporosis; vaccinations and immunizations; sexual dysfunction drugs; treatments for B12 deficiency; smoking cessation; treatment of gynecological problems; treatment of other women's health issues; general anesthetics; local anesthetics; opioid analgesics; agonist-antagonists and antagonists; antitussives; drugs used in the treatment of motor disorders; antiepileptics; drugs used in affective disorders; antipsychotics (neuroleptics); sedative-hypnotics, anxiolytics, and centrally acting muscle relaxants; treatments for anxiety disorders; skeletal muscle relaxants; treatments for Parkinson's disease; treatments for Alzheimer's disease; treatment for pain and anti-migraine treatment.

Medicaments for treatment of allergic rhinitis include: steroids, including corticosteroids, Flonase, Patanase, Beconase, Antihistamine, Astelin, Otrivin^{™}, Livostin, Theramax, Avamys, Lufeel, Sinofresh, Nasonex, Nasocort and Veramyst.

Medicaments for treatment of osteoporosis include: Miacalcin, Fortical and Stadol.

Medicaments for vaccinations and immunizations include: LAVIN, and influenza vaccines including FluMist.

Medicaments for smoking cessation include: NasalFent.

Other medicaments which can be delivered include: calcitonin and parathyroid hormone.

Neurotransmitters and neuromodulators that can be delivered include: acetylcholine (ACH), Anticholinergic drugs, adenosine triphosphate (ATP), aspartate (Asp), beta-amyloid, beta-endorphin, bradykinin, dopamine (DA), L-DOPA, Carbidopa, epinephrine, dynorphins, endomorphins, enkephalins, 5-hydroxytryptamine (5-HT), Sumatriptan, Imitrex, Migranal, Zolmitriptan, Zomig, Gamma-aminobutyric acid (GABA), glutamate (glu), glycine, histamine, leptin, nerve growth factor and other growth factors), norepinephrine, nitric oxide, and Substance P.

General anesthetics which can be delivered include: alfentanil, desflurane, enflurane, etomidate, fentanyl, halothane, isoflurane, ketamine, methohexital, methoxyflurane, midazolam, lorazepam, diazepam morphine, nitrous oxide (N₂O), propofol, sevoflurane, Sufentanil, Sublimase, and thiopental.

Local anesthetics which can be delivered include: benzocaine, bupivacaine, cocaine, lidocaine, prilocaine, procaine, ropivacaine, and tetracaine.

Opioid analgesics, agonist-antagonists, and antitussives which can be delivered include: agonists, codeine, diphenoxylate, fentanyl, heroin and other opioids, hydrocodone, l-alpha-acetyl-methadol, levomethadyl acetate, loperamide, meperidine, methadone, morphine, oxycodone, d-propoxyphene, combinations of opioids plus acetaminophen and asa, and tramadol.

Agonist/antagonists and antagonists which can be delivered include: buprenorphine, butorphanol, nalbuphine, nalorphine, naloxone, naltrexone, nalmefene, pentazocine, codeine, dextromethorphan, and hydrocodone.

Drugs used in the treatment of Parkinson's disease and motor disorders which can be delivered include: amantadine, apomorphin, baclofen, benzodiazepines, benztropine, bromocriptine, carbidopa, cyclobenzaprine, dantrolene, dopamine, entacapone, haloperidol, L-DOPA, pergolide, pramiprexole, ropinerole, selegiline (deprenyl), trihexyphenidyl, rasagiline, azilect, selegiline, ladostigil, rotigotine, neupro, mono amine oxidase inhibitor, and COMT inhibitor.

Antiepileptics which can be delivered include: acetazolamide, carbamazepine, clonazepam, diazepam, ethosuximide, felbamate, gabapentin, Lamotrigine, lorazepam, phenobarbital, phenytoin, primidone, tiagabine, topiramate, valproic acid, Vigabatrin and Midazolam.

Drugs used in affective disorders which can be delivered include: antidepressants, amitriptyline, bupropion, citalopram, clomipramine, desipramine, fluoxetine, fluvoxamine, imipramine, nortriptyline, paroxetine, phenelzine, sertraline, trazodone, tranylcypromine, venlafaxine, antimanic drugs, carbamazepine, lithium carbonate and valproic acid.

Antipsychotics (neuroleptics) which can be delivered include: chlorpromazine (CPZ), clozapine, fluphenazine, haloperidol, olanzapine, quetiapine, risperidone, sertindole, thioridazine, thiothixene and ziprasidone.

Sedative-hypnotics, anxiolytics, and centrally acting muscle relaxants which can be delivered include: alprazolam, chloral hydrate, diphenhydramine, flumazenil, flurazepam, hydroxyzine, lorazepam, oxazepam, phenobarbital, temazepam, triazolam, zaleplon and zolpidem.

Anxiety disorders and skeletal muscle relaxants which can be delivered include: alprazolam, chlorazepate, chlordiazepoxide, diazepam, flumazenil (antagonist), lorazepam, and oxazepam.

Treatments for Alzheimer's disease which can be delivered include: donepezil, galantamine, rivastigmine, Tacrine, Detemir, Novolin, Humulin, Insulin, insulin like hormone, an insulin analog such as NPH Insulin, Lispro, Aspart, Detemir Insulin, Glulisin, Glargin Insulin, Insulin degludec, BDNF, GDNF, MIBG, anti-cancer agents, anti-cancer drugs, dopamine agonist and dopamine antagonist.

Other drugs which can be delivered include: amphetamine, caffeine, ephedrine, methamphetamine, methylphenidate, phentermine, sibutramine, disulfiram, ethanol, methanol, naltrexone, atropine, scopolamine, ketamine, lysergic acid diethylamide (LSD), MDMA (methylene dioxy-methyl amphetamine), mescaline, phencyclidine (PCP), donabinol, marijuana/THC, organic solvents, nicotine, Pentobarbital, neuroprotective compounds, neuroprotective peptides, neuroprotective factors, davunetide, anti-schizophrenic drugs, anti-depression drugs, comtan, Entacopone, anti ADHD agents, anti ADHD drugs such as Methylphenidrate (ritalin), and anti-autism and anti-autism symptoms drugs.

Other materials that can be delivered include: both purified natural and synthetic biologics, peptides, proteins, antibodies, cells including stem-cells, parts of cells, nanoparticles and microparticles. The nanoparticles and microparticles can comprise drugs; they can be carriers for drugs, cells or parts of cells; and any combination thereof.

In preferred embodiments, the substance comprises permeation enhancers to improve penetration of the active components of the substance through the mucosal membranes.

In some formulations, the formulation can comprise polymeric microparticles comprising at least one active agent and a permeation enhancer, where the active agent is selected from a group consisting of a peptide, a protein, an antibody, nucleic acid, small molecules, cells and any combination thereof.

A great number of penetration enhancers are known in the literature.

One such penetration enhancer is Hyaluronic acid (also referred to as HA or hyaluronan), which is a polysaccharide that occurs naturally in the body. Due to its exceptional water-binding, visco-elastic and biological properties, HA can improve the attributes, such as, but not limited to, the absorption characteristics, of existing formulations and can also add new attributes to existing formulations. Inclusion of HA can be advantageous when developing new formulations.

When used for drug delivery and targeting, HA can provide clear advantages over traditional polymeric substances such as synthetic polymers such as, but not limited to, poly(ethylene glycol), poly(lactic acid), poly(glycolic acid), poly Acrylic Acid and Poly-(N-isopropylacrylamide), or other biopolymers such as chitosan and alginate.

HA's benefits in the drug delivery area include, but are not limited to:
- Flexibility when designing controlled drug release profiles;
- More stable drug formulations;
- Effective drug targeting via accumulation at the targeted site and receptor-mediated uptake;
- Enhancement of bioavailability and biocompatibility of drugs; and
- Reduction of drug cytotoxicity in healthy tissues polymeric microspheres polymeric controlled release preparation a mucoadhesive agent.

Other penetration enhancers include, but are not limited to the following:
A group containing: a fatty acid, a medium chain glyceride, surfactant, steroidal detergent, an acyl carnitine, Lauroyl-DL-carnitine, an alkanoyl choline, an N-acetylated amino acid, esters, salts, bile salts, sodium salts, nitrogen-containing rings, and derivatives. The enhancer can be an anionic, cationic, zwitterionic, nonionic or combination of both. Anionic can be but not limit to: sodium lauryl sulfate, sodium decyl sulfate, sodium octyl sulfate, N-lauryl sarcosinate, sodium carparate. Cationic can be but not limit to: Cetyltrimethyl ammonium bromide, decyltrimethyl ammonium bromide, benzyldimethyl dodecyl ammonium chloride, myristyltimethyl ammonio chloride, deodecyl pridinium chloride. Zwitterionic can be but not limit to: decyldimethyl ammonio propane sulfonate, palmityldimethyl ammonio propane sulfonate. Fatty acid including but not limit to: butyric, caproic, caprylic, pelargonic, capric, lauric, myristic, palmitic, stearic, arachidic, oleic, linoleic, linolinic acid, their salts, derivatives and any combinations or glyceride, monoglyceride, a diglyceride, or triglyceride of those fatty acids. Bile acids or salts, including conjugated or un conjugated bile acids, such as but not limited to: cholate, deoxycholate, tauro-cholate, glycocholate, taurodexycholate, ursodeoxycholate, tauroursodeoxycholate, chenodeoxycholate and their derivatives and salts and combinations. Permeation enhancer as comprises a metal chelator, such as EDTA, EGTA, a surfactant, such as sodium dodecyl sulfate, polyethylene ethers or esters, polyethylene glycol-12 lauryl ether, salicylate polysorbate 80, nonylphenoxypolyoxyethylene, dioctyl sodium sulfosuccinate, saponin, palmitoyl carnitine, lauroyl-l-camitine, dodecyl maltoside, acyl carnitines, alkanoyl cjolline and combinations. Other include but not limited, 3-nitrobenzoate, zoonula occulden toxin, fatty acid ester of lactic acid salts, glycyrrhizic acid salt, hydroxyl beta-cyclodextrin, N-acetylated amino acids such as sodium N-[8-(2-hydroxybenzoyl)amino]caprylate and chitosan, salts and derivatives and any combinations.

Other enhancers include: formulations of water in oil, formulations of oil in water; emulsions, double emulsions, micro-emulsions, nano-emulsions, water in oil emulsions, oil in water emulsions; steroidal detergent, and an acylse; to allow better absorption in the mucosal tissue, better permeation and absorption in the target cells, better stability of the encapsulated drug/ active ingredient.

Some embodiments comprise, either alone or in combination with a penetration enhancer, a mucoadhesive agent such as, but not limited to, bioadhesive proteins, carbohydrates and mucoadhesive polymers

In the capsule of the present invention, the device comprises at least one compartment, and preferably a plurality of compartments, each containing a flowable substance. The delivery device is designed to rupture the compartments such that the flowable substances are mixed with a carrier, preferably air, and delivered to a predetermined deposition site, typically, but not exclusively, in the nasal passages.

Medicaments may be supplied as liquids, as powders, or as aerosols. In the preferred embodiment, the medicament is supplied in a single-dose capsule. In other embodiments, the medicament is supplied in a multi-dose capsule means, the multi-dose capsule configured to provide a single dose per activation.

In preferred embodiments, the flowable-substance capsule has a plurality of compartments. A compartment can contain at least one medicament, at least one medicament precursor, carrier gas, compressed gas, and any combination thereof.

The different compartments can contain different medicaments, with the plurality of medicaments delivered to the nostril or other delivery site in a single dose. In this manner, a plurality of medicaments may be supplied to the nostril in a single injection, with interactions occurring between the medicaments at most during the short time between activation of the device and the delivery of the substances and their deposition at the target site.

In some embodiments, interactions between components are unwanted. In such embodiments, a sequential release will utilize the short time period between release of the components and their absorption in the body to prevent such unwanted interactions and/or reactions.

In other embodiments, mixing and/or reactions are desired. In such embodiments, the reactions can occur all at once, by rupturing all of the compartments at the same time and mixing/interacting the components, either in the aerosol or in at least one mixing chamber. In other embodiments, a component can be added by needle insertion at a desired time before use, either into an empty compartment or into an occupied compartment (so that a desired reaction can occur). In other embodiments, the compartment walls rupture in a predetermined order, so that mixing/interaction occurs in stages, in a predetermined order. Mixing/interaction can occur in a compartment or compartments, in a mixing chamber, in the air passages of the device, in the aerosol, in the nasal (or other) passages of the body, and any combination thereof.

As a non-limiting example, a medicament can comprise four components, stored in four compartments of a capsule. Prior to activation, a fifth component is injected into compartment 1. After a predetermined time, the device is activated and the walls between compartment 1 and compartment 2 are broken, allowing mixing of 5/1 and 2. This followed by rupture of the walls surrounding component 3, which then mixes with 5/1/2 and reacts with 2. The last walls to rupture are those surrounding compartment 4; material 4 remains in a separate part of the aerosol and deposits on the nasal passages after deposition of 5/1/2/3.

In another example, precursor A mixes with precursor B to form intermediate C, and, subsequently, intermediate C mixes with precursor D to form final product E.

Mixing or reactions or release of components from different compartments can occur simultaneously, in different linked compartments, or they can occur sequentially, as in the example above. Any combination of sequential and simultaneous reactions and/or mixing and/or release can be used. Components can arrive at the deposition site simultaneously, either mixed or unmixed, sequentially, and any combination thereof.

It should be noted that there can be a predetermined delay of some fractions of a second between rupturing of walls of different compartments, in order to, for non-limiting example, allow complete mixing of one set of components or allow a reaction between one set of components to go to completion before the next mixing/reaction starts or the delivery starts.

In some embodiments, the device or, preferably, the capsule, comprises a mixing mechanism or mixing chamber, so that, as described above, components of the composition can mix and/or react during the activation process, enabling components to be stored separately and/or to be stored as stable precursors, but to deliver a predetermined treatment comprising at least one medicament to a predetermined delivery site.

In preferred embodiments of the device, the mixture of aerosol and pre-aerosolized mist is formed within the nozzle, with the hole at the lateral end of the nozzle having little effect on either the shape of the dispersion plume or the velocity of the aerosol.

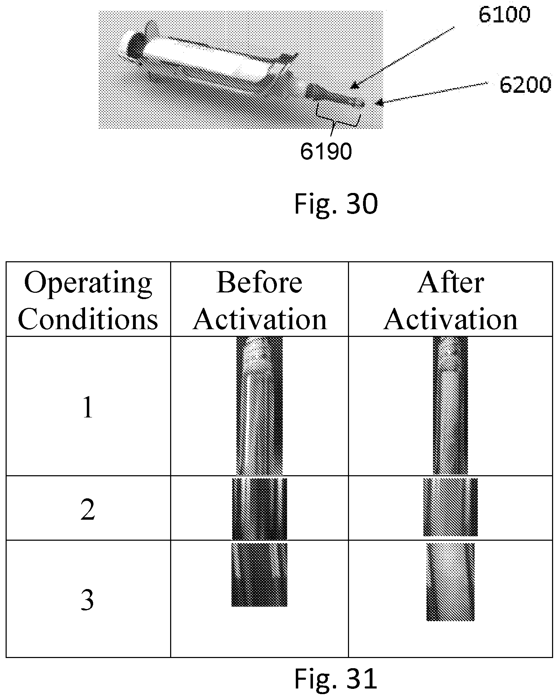

An experimental setup to demonstrate the location of formation of the mist is shown in **Fig. 30****,** and the results of tests for three different operating conditions (1, 2 and 3) are shown in Table 1 and **Fig. 31****.**

**Table 1: Location of Aerosol Formation**

| **Test** | **Air Volume (ml)** | **Pressure (bar)** | **Orifice Diameter (mm)** | **Aerosol produced Before Exit from Device?** |
|---|---|---|---|---|
| 1 | 19 | 6 | 0.8 | Yes |
| 2 | 8 | 4 | 0.8 | Yes |
| 3 | 8 | 6 | 0.8 | Yes |

**Fig. 30** shows an embodiment of the device, with the nozzle **(6100)** and the nozzle tip **(6200)** on the right, with the bracket indicating the region shown enlarged **(6190)** in **Fig. 31****.**

Representation before activation is shown in the center of **Fig. 31****,** and representation during activation is shown on the right. Before activation, the nozzle is clear; there is no aerosol therein. After activation, the nozzle appears opaque due to the aerosol and/or pre-aerosolized mist therein. If no aerosol or pre-aerosolized mist had been formed, the liquid would exit as a thin stream, which would appear in the image as a streak down the center of the nozzle.

### Droplet Distribution for travel down a tube

In all known other mechanisms of creating aerosols, an orifice is placed at the end of a nozzle and the inner diameter of the device's nozzle and, especially, its orifice, is the main parameter that influences aerosol formation and the aerosol's characteristics. In contrast, in the present invention, no orifice is needed. More than that, putting a conventional orifice at the end of the nozzle will actually limit the forces reaching the liquid or powder being dispensed, and thus will reduce the ability to create the desired fine aerosol at the target site. Thus, the large diameter tubing that can be used in the present invention, about an order of magnitude larger than the diameter of commonly-used tubes and orifices, results in the desired fine aerosol, carried efficiently into the nasal cavity with droplet median diameters (DV50) on the order of 1-100 micrometer.

In the present invention, the aerosol is created as a result of the air volume-pressure parameters of the device and is influenced by the nasal cavity resistance rather than primarily by the orifice diameter.

In order to model nasal friction and air resistance and as a model for aerosol formation in the nasal cavity, a 36cm long glass tube with an inner diameter of 2cm, filled with oil up to 22cm of its length, was used.

Theoretical analysis has indicated that 5 cm of tube is equivalent to about 0.1 - 0.5 cm of the nasal passages; therefore the 22 cm. tube would approximately simulate the full depth of a nasal passage.

The test material was 200 microliter of Methylene Blue liquid solution.

The liquid solution was discharged from a device into the base of the tube and pictures and videos were taken in order to be able to follow the process of aerosol formation. The length of the deposition region, the aerosol distribution and the diameter of the aerosol droplets were determined as a function of time.

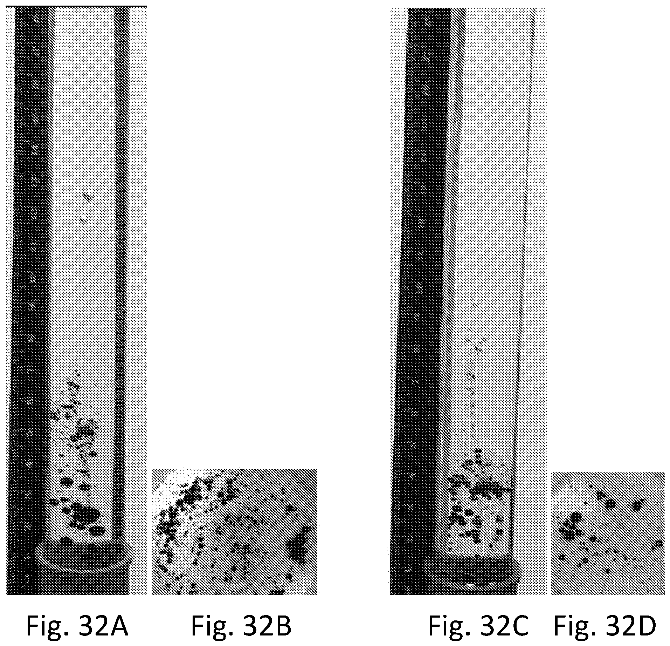

Figs. 32A-D show the effect of orifice size on droplet size **(**Figs. 32B, D) and droplet distribution **(****Figs. 32A**, C) in a conventional device.

The Methylene blue solution was injected into the tube using a syringe. Figs 32A-B show droplet distribution and size for a larger needle (21G; approx.0.5mm) and Figs. 32C-D show droplet distribution and size for a smaller needle (25G; approx.0.2mm). The larger diameter needle **(**Figs 32A-B) creates larger droplets than the smaller diameter needle **(**Figs. 32C-D).

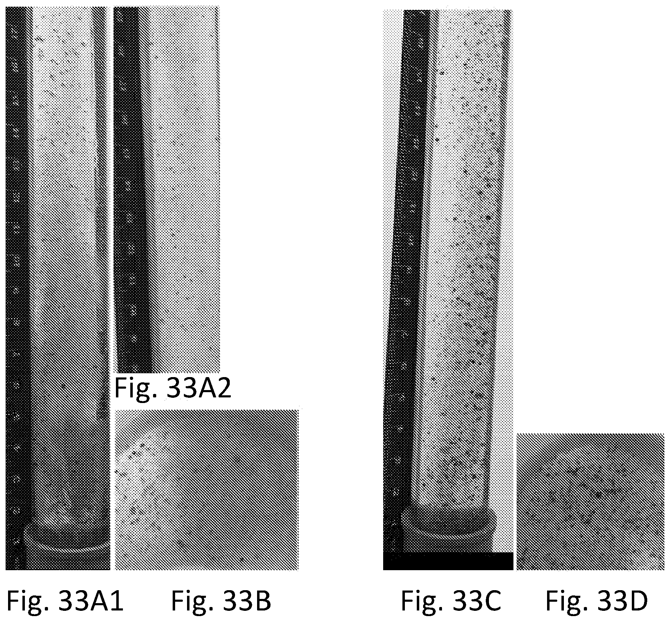

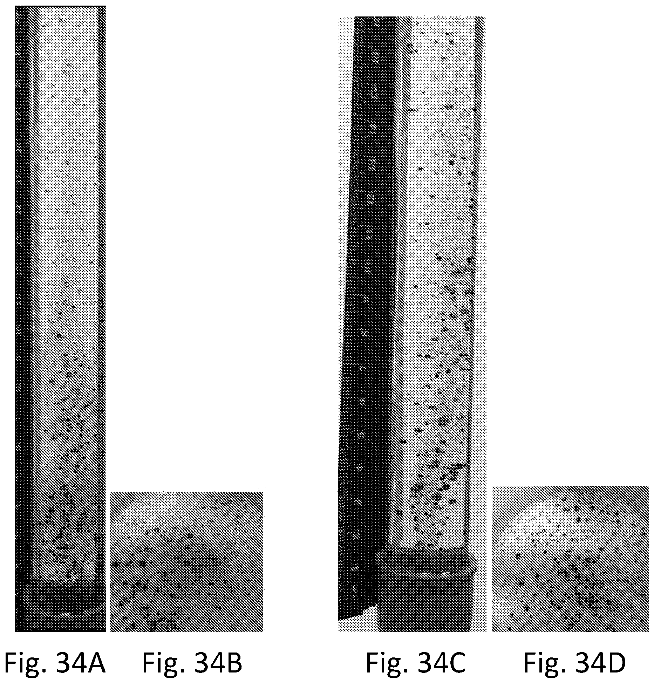

In contrast, Figs. 33A-D and 34A-D show that the opposite is true if the technique of the present invention is used, where the aerosol is created by means of a pressurized gas.

In reference to **Fig. 33**, Figs. 33A-D show the effect of orifice size on droplet size **(****Figs. 33B**,D) and droplet distribution (Figs. 33A1, A2, C) in a device of the present invention. Fig. 33A1 shows the distribution in the lower part of the tube, while Fig. 33A2 shows the distribution in the upper part of the tube.

In Figs. 33A-D, the device of the present invention is charged to 7barg pressure and 20ml of Methylene Blue solution is discharged through an orifice into the base of the tube. Figs 33A-B show droplet distribution and size for a larger needle (21G; approx.0.5mm) and **Figs. 33C-****D** show droplet distribution and size for a smaller needle (25G; approx.0.2mm). In this case, the larger nozzle (Figs. 33A, B) has smaller diameter droplets, a more homogeneous aerosol and a distribution that extends much further up the tube than the smaller diameter nozzle **(****Figs. 33C**, D).

In reference to Fig. 34, Figs. 34A-D show the effect of orifice size on droplet size **(****Figs. 34B,** D) and droplet distribution **(****Figs. 34A**, C) in a device of the present invention.

In Figs. 34A-D, the device of the present invention is charged to 4barg pressure and 18ml of Methylene Blue solution is discharged through an orifice into the base of the tube. **Figs 34A-****B** show droplet distribution and size for a larger needle (21G; approx.0.5mm) and **Figs. 34C-****D** show droplet distribution and size for a smaller needle (25G; approx.0.2mm). In this case, the larger nozzle **(****Figs. 34A**, B) has smaller diameter droplets and a more homogeneous aerosol than the smaller diameter nozzle **(****Figs. 34C**, D).

A comparison of **Figs. 33** and **34** shows that the higher volume-higher pressure combination (20ml, 7barg) has smaller diameter droplets with a greater homogeneity and a distribution that extends much further up the tube than the lower volume-lower pressure combination (18cc, 4barg).

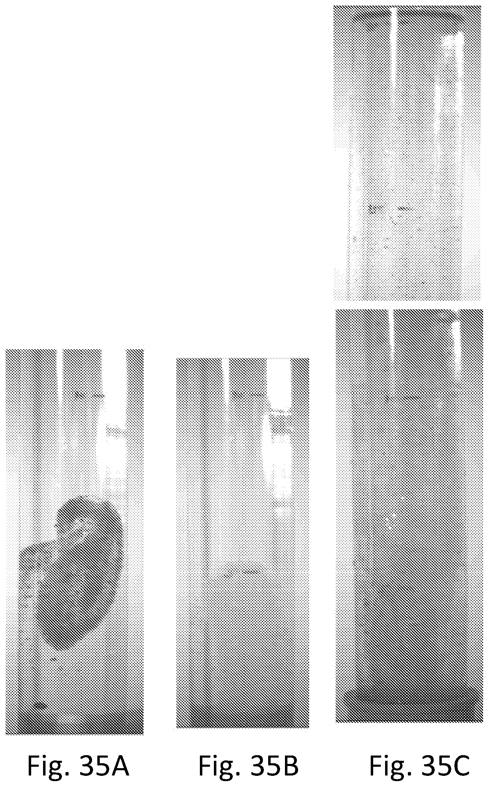

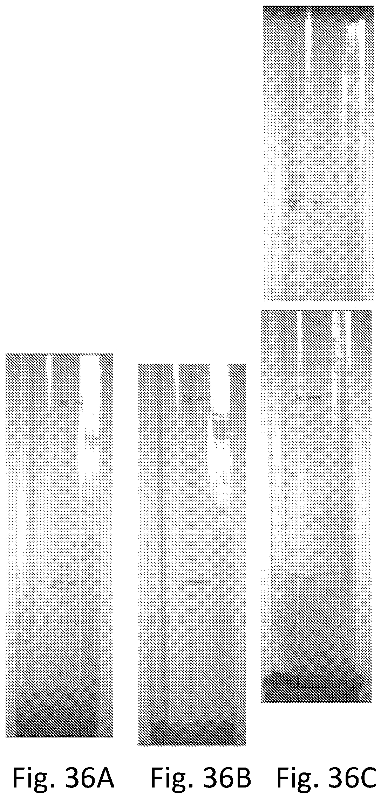

In **Figs. 35** and **36****,** a comparison is made between 2 commercial, prior art devices and the present invention device. In all cases, 0.1 ml of aqueous solution was tested. In **Figs. 35A** and Cand 36A and C, the liquid was Methylene Blue in aqueous solution; in **Figs. 35B** and **36B****,** saline solution alone was used. The liquid was discharged from the device into the base of a tube filled with oil. In **Figs. 35A** and **36A****,** the Otrivin^{™} device was use, in **Figs. 35B** and **36B****,** the Otrimer^{™} device was used, and in **Figs. 35C** and **36C****,** the present invention technology was used. For both the Otrivin^{™} **(****Fig. 35A****)** and the Otrimer^{™} **(****Fig. 35B****)** devices, the height reached by the solution at the time of application is less than 10 cm and the liquid forms a distinct bolus near the bottom of the tube. In contrast, with the device demonstrates the present invention **(****Fig. 35C****),** the liquid appears in the tube as small droplets, with some of the droplets reaching a height in the tube of 20 cm.

Two minutes later, **(**Figs 36A-C), the liquid from the Otrivin^{™} device has reached a height of about 5 cm **(****Figs 36A****),** while the liquid in from the Otrimer^{™} device has fallen to the base of the tube; it is barely visible at the bottom of the tube in **Fig. 36B****.** In contrast, the droplets are fairly stable in the tube in the present invention technique **(****Fig. 36C****);** there is a fairly even distribution of droplets until a height of about 12 cm is reached, and some of the droplets have reached a height of nearly 20 cm.

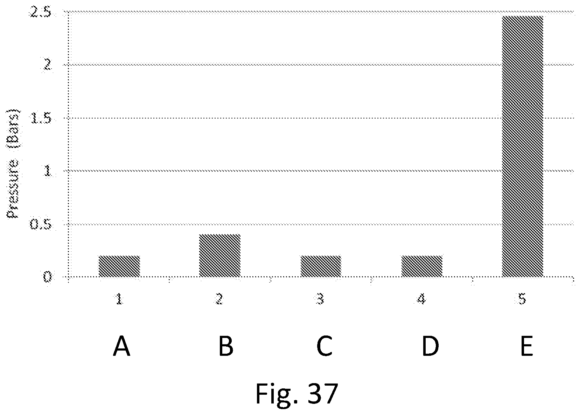

In reference to **Fig. 37****,** nasal applicators were connected to a ~7ml volume closed tubing, with a connection to a pressure sensor. The ~7ml volume represents the approximate volume of the nasal cavity. The devices were discharged into the closed tubing and the maximum pressure developed in the tubing was measures. The pressure in the tubing for the four commercial devices, the Otrivin^{™} device (Fig. 37A), the Marimer device (Fig. 37B), the Rhinox device (Fig. 37C), and the Alrin^{™} device (Fig. 37D) were less than 1 barg. In contrast, the pressure in the tubing from the present invention technology (Fig. 37E), was almost 2.5 barg, more than 2 1/2 times as much as the closest commercial device, the Marimer device (Fig. 37B).

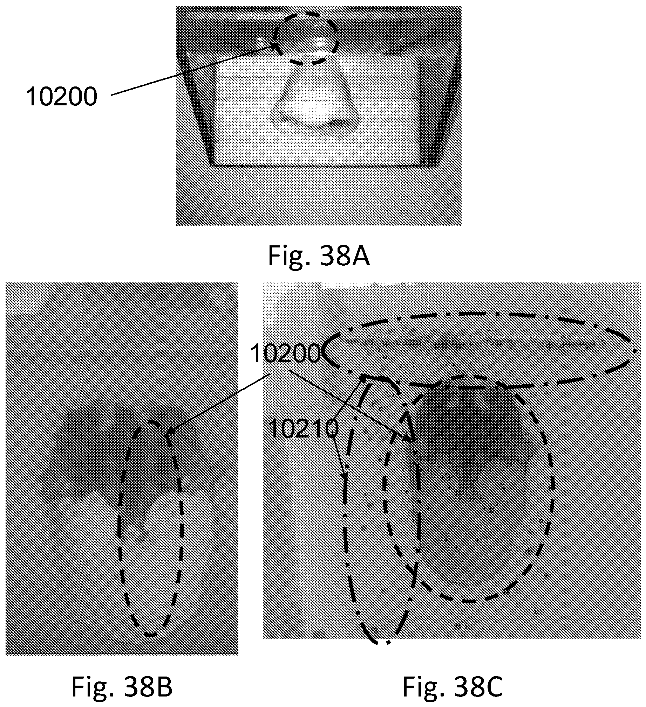

In reference to **Fig. 38****,** Methylene Blue in aqueous solution was delivered through a nostril of a human nasal cast model **(****Fig. 38A****)** filled with oil to mimic the nasal cavity's inner pressure and conditions. The dashed circles **(10200)** show the exit from the top of the nasal cast; material that reaches the exit would reach the olfactory epithelium in the nose. **Figs. 38B** and C show nasal cast models after application of the Otrivin^{™} device **(****Fig. 38B****)** and the present invention technology based device (**Fig. 38C**) to a nostril of the nasal cast. As can be seen from **Fig. 38B****,** no material would reach the olfactory epithelium with the Otrivin^{™} device - no material has reached the exit from the nasal cast. In contrast, droplets of material (dotted circle, **10210**) have exited the nasal cast, showing that, unlike the commercial devices, the material discharged from the present invention technology based device is capable of reaching the olfactory epithelium.

### Plume angle

In contrast to prior-art nasal delivery devices and technologies, the devices of the present invention can produce a fine aerosol in the nasal cavity or other desired body orifice at the target area and at the location of the target tissue instead of immediately after exit from the device. Utilizing the pressure as a driving force and the air as a carrier allows the material to be released from the nozzle as a combination of material in a pre-aerosolized state and an aerosol. The properties of the resultant aerosol are typically dependent on the properties of the device and of the medium into which the aerosol is discharged. The properties of the device which affect the aerosol characteristics are the delivery speed, the volume of the delivery gas, and the characteristics of the delivery orifice.

In some embodiments, the aerosol properties are fairly independent of the delivered substance, in other embodiments, the pressure, volume, orifice characteristics and delivered substance properties can be co-optimized.

In prior-art devices the aerosol is produced at the exit to the device. Typically, the aerosol comprises a wide dispersion of particle sizes, a wide "fan" of aerosol and a low driving force. Therefore, the large droplets typically deposit very close to the exit from the device; smaller droplets tend to quickly contact the walls of the passage, so that deposition is typically predominantly close to the exit from the device, with little of the substance reaching desired sites deeper in the orifice, such as the turbinates of the nose,

In contrast, in the present device, the aerosol and pre-aerosolized mixture of gas and substance exits the device with a significant driving force, when the preaerosolized fluid hits the walls of the nasal passages, it "explodes" into a fine aerosol that is capable of being driven by the pressure deep into the nasal passages to deposit in the desired region.

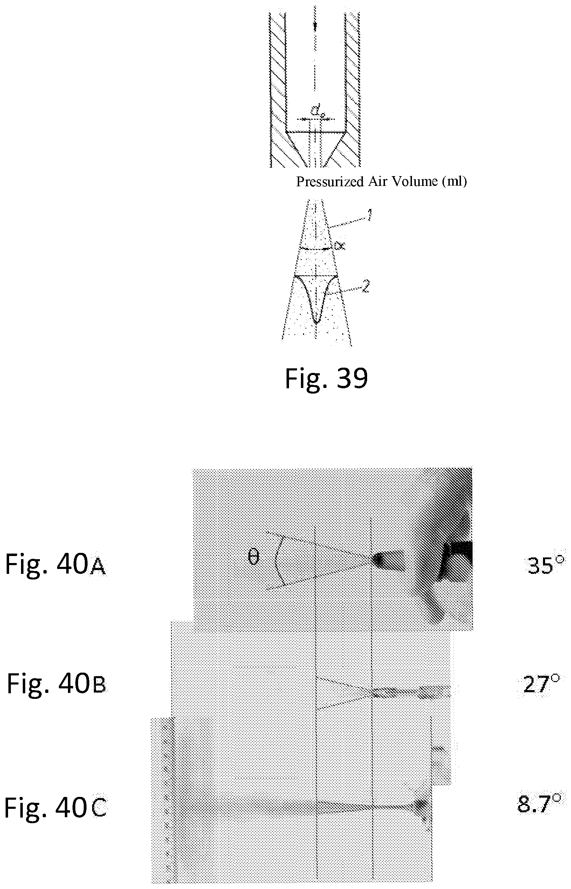

In reference to **Fig. 39****,** a schematic is shown of a nozzle and the aerosol it releases. The orifice emits an aerosol which forms a conical plume (**1**) with a distribution of particles (**2**) in it.

The plume angle is the total angle subtended by the plume, as shown by the angle α **in** **Fig. 39** and by the angle θ subtended between the lines, as shown in F**ig. 40A.**

In **Fig. 40****,** the plume angle θ is compared for 2 commercial nasal delivery devices (**Fig. 40A-****B**) and the SipNose device (**Fig. 40C**). Aerosol was measured at room temperature. The widths of the plumes were measured at the same distance (3cm) from the discharge site in each device.

The SipNose device has a much narrower plume than the two commercial devices. The plume angles for the commercial devices, the Alrin^{™} from Teva (**Fig. 40A**) had a plume angle of 35°, the LMA MAD Nasal^{™} (**Fig. 40B**) had a plume angle of 27°, while the plume angle for the SipNose device (**Fig. 40C**) had a plume angle of only 8.7°.

All the above parameters allow the aerosol to better deposit in the area of interest- such as the area of the olfactory epithelium in the nasal cavity; and to be better absorbed by the target tissue such as the brain.

### Plume intensity

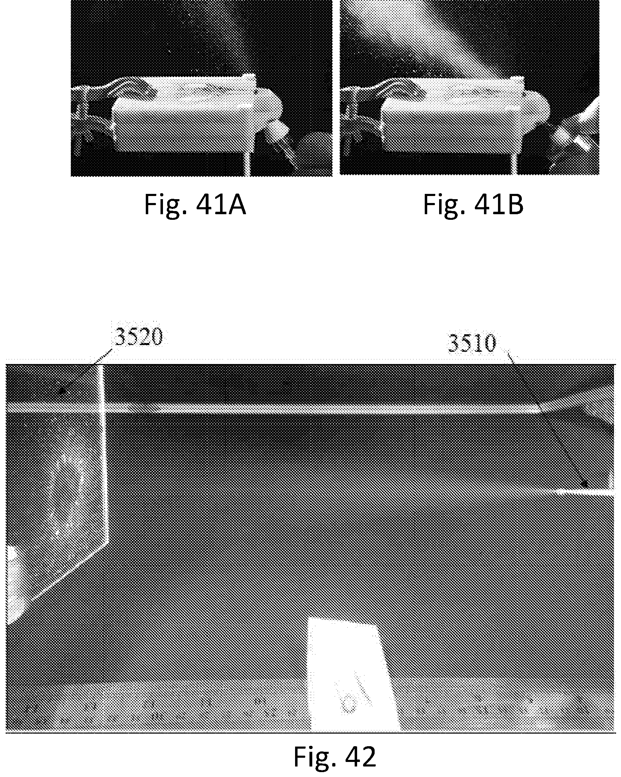

**Fig. 41** compares the amount of material reaching the upper layers of the nasal model for one of the prior-art devices (commercial 2 - LMA MAD Nasal^{™,}, **Fig. 41A**) and the device of the present invention (SipNose, **Fig. 41B**). In each case, 100µl of liquid aerosol was discharged into a human nasal model (a nasal cast). The upper layer of the nasal cast was removed in order to observe the amount and characteristics of the aerosol that reaches the area of the upper portion of the nasal cavity. It is clear that there is a better distribution of the substance in the area of interest, the uppermost portions of the nasal cavities when the present invention technology is in use (**Fig. 41B**).

**Fig. 42** shows an experimental setup for determining coverage of a spray plume. A device (**3510**) is at the left, and a screen for measuring coverage is at the right (**3520**). The distance between device and screen is about 25 cm.

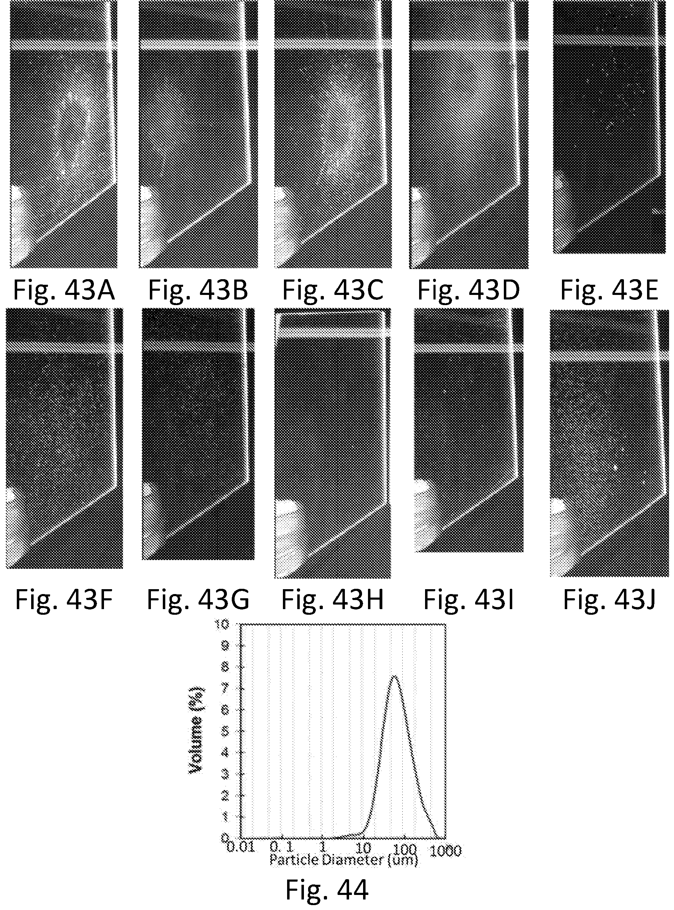

Figs. 42A-J show examples of spray coverage for different devices. Figs. 43A-D show coverage and droplet distribution for the SipNose device for different device parameters, while Figs. 43E-J show coverage and droplet distribution for a number of different commercial devices.

In all cases, the SipNose device produces a spray pattern covering a well-defined area of the screen. A large number of particles reach the screen and, in the coverage area, this is significantly more than for any of the commercial devices.

Commercial devices F and J are the best of the prior-art devices, with a reasonable amount of the aerosol reaching the screen, but the distribution is very much wider than for the SipNose device, covering virtually the entire screen. Commercial devices H and I are the worst of the prior-art devices, with very little of the aerosol even reaching as far as the screen.

Tables 2 and 3 show plume characteristics for the SipNose device for different operating parameters and an orifice size of 0.8 mm (Table 11) and for four commercial devices (Table 12).

**Table 2: Plume Characteristics for the SipNose device**

| **Pressure (Bar)** | **Gas Volume V_{gas} (ml)** | **Substance Volume V_{sub} (ul)** | **Angle C)** | **Height at 3cm (mm)** | **Height at 6cm (mm)** | **Velocity (m/s)** |
|---|---|---|---|---|---|---|
| 2 | 8 | 500 | 15 | 10 | 16 | 23 |
| 6 | 8 | 500 | 20 | 12 | 18 | 15.8 |
| 6 | 19 | 100 | 15 | 10 | 21 | |
| 6 | 19 | 500 | 20 | 11 | 18 | |
| 2 | 5 | 100 | 9 | 10 | 12 | |
| 2 | 10 | 100 | 15 | 8 | 15 | |
| 2 | 5 | 500 | 17 | 18 | 20 | |
| 2 | 10 | 500 | 16 | 11 | 17 | 11.9 |
| 6 | 12 | 100 | 12 | 9 | 18 | |
| 6 | 6 | 500 | 12 | 5 | 6 | |
| 6 | 12 | 500 | 16 | 8 | 13 | |

**Table 3: Plume Characteristics for the commercial devices**

| **Device** | **Pressure (Bar)** | **Gas Volume V_{gas} (ml)** | **Substance Volume V_{sub} (ul)** | **Angle (°)** | **Height at 3cm (mm)** | **Height at 6cm (mm)** | **Velocity (m/s)** |
|---|---|---|---|---|---|---|---|
| MAD Nasal^{™}; | --- | 6 | 100 | 35 | 18 | 30 | 2.3 |
| Wolfe Tory | --- | 18 | 100 | 55 | 30 | 40 | |
| | --- | 0 | 500 | 55 | 38 | 55 | |
| | --- | 3 | 500 | 35 | 25 | 30 | |
| | --- | 6 | 500 | 35 | 21 | 35 | 2.3 |
| | --- | 18 | 500 | 33 | 20 | 29 | |
| Simply Saline Nasal Mist; Church & Dwight Co., Inc. | 7 | --- | 500 | 45 | 37 | 53 | |
| Otrimer; Novartis | 7 | --- | 500 | 35 | 20 | 33 | |
| Alrin, Teva | --- | 0 | 100 | 35 | 26 | 33 | 3.3 |

Significant differences were seen between the properties of the plumes between the SipNose device and the commercial devices; small, if any, overlap was seen between the plume angles, the plume heights or the plume velocities. For the SipNose devices, the range of plume angles was 5° to 25°, the range of plume heights 3 cm from the device was 1 to 20 mm, the range of plume heights 6 cm from the device was 5mm to 25 mm and the range of plume velocities was 5m/s to 50 m/s. For the commercial devices, the plume angles were over 33°, the plume heights 3 cm from the device were over 18 mm, the plume heights 6 cm from the device were over 29mm and the plume velocities were less than 5 m/s.

An example of the droplet size distribution is given in **Fig. 44****,** which shows a plot of the number of particles vs. particle size, with the X axis (particle size) being logarithmic. The distribution is asymmetrical, having a long "tail" on the large-diameter side. The largest droplets are on the order of 900µm, with the smallest particles being on the order of a 2-5 µm. The DV50 diameter is about 75 µm.

Tables 4 and 5 show droplet size distributions averaged over 10 repeats for 100ul and 400ul saline in two SipNose devices (23-11for 100ul and 23-12 for 400ul) for parameters 6 barg pressure, 19ml of gas, and 0.8mm orifice diameter. In all cases shown, low variability was seen for the 10 repeats of the measurements.

**Table 4: Device 23-11**

| Device Version | D v (0, 0.5) (µm) | Obscuration (%) |
|---|---|---|
| 23-11 | 73.0 | 1.3 |
| 23-11 | 70.6 | 1.5 |
| 23-11 | 78.8 | 0.9 |
| 23-11 | 86.6 | 1.2 |
| 23-11 | 74.2 | 1.3 |
| 23-11 | 88.7 | 1.0 |
| 23-11 | 64.9 | 1.2 |
| 23-11 | 86.7 | 1.3 |
| 23-11 | 55.3 | 1.2 |
| 23-11 | 58.3 | 1.1 |
| Mean | 73.7±11.8 | |

**Table 5: Device 23-12**

| Device Version | D v (0, 0.5) (µm) | Obscuration (%) |
|---|---|---|
| 23-12 | 68.7 | 3.8 |
| 23-12 | 83.5 | 2.4 |
| 23-12 | 81.7 | 4.8 |
| 23-12 | 71.4 | 22.9* |
| 23-12 | 92.1 | 3.3 |
| 23-12 | 83.8 | 4.3 |
| 23-12 | 83.3 | 5.3 |
| 23-12 | 100.6 | 3.4 |
| 23-12 | 100.8 | 2.8 |
| 23-12 | 92.3 | 6.4 |
| 23-12 | 108.6 | 3.4 |
| Mean | 88.3±12.9 | |

| | | |
|---|---|---|
| * anomalous value | | |

Table 6 shows an example of the reproducibility for the SipNose device. The measurements were done by weighing, and part of the variability shown probably depends on the measurement technique.

**Table 6: Reproducibility for a SipNose device**

| | Amount loaded (gm) | Residual amount (gm) | Released (%) | Residual volume (%) |
|---|---|---|---|---|
| 1 | 0.3996 | 0.0584 | 85.4 | 14.6 |
| 2 | 0.4058 | 0.0414 | 89.8 | 10.2 |
| 3 | 0.3915 | 0.0054 | 98.6 | 1.4 |
| 4 | 0.4143 | 0.0063 | 98.5 | 1.5 |
| 5 | 0.3772 | 0.0069 | 98.2 | 1.8 |
| 6 | 0.3902 | 0.0509 | 87.0 | 13.0 |
| 7 | 0.4010 | 0.0626 | 84.4 | 15.6 |
| 8 | 0.3853 | 0.0490 | 87.3 | 12.7 |
| 9 | 0.4302 | 0.0511 | 88.1 | 11.9 |
| 10 | 0.4052 | 0.0482 | 88.1 | 11.9 |
| Average | 0.4000 | 0.0380 | 90.5 | 9.5 |
| Std. Dev. | 0.0152 | 0.0227 | 5.6 | 5.6 |

SipNose aerosol droplets have a mean diameter in the typical range of other nasal delivery devices, and even smaller.

Although the droplets have a small diameter, the width of the aerosol plume is very narrow, and this allows the aerosol to be better distributed in the inner part of the nasal cavity, without depositing at the front of a cavity such as the nasal cavity.

The SipNose device shows high consistency

### Penetration through Fabric

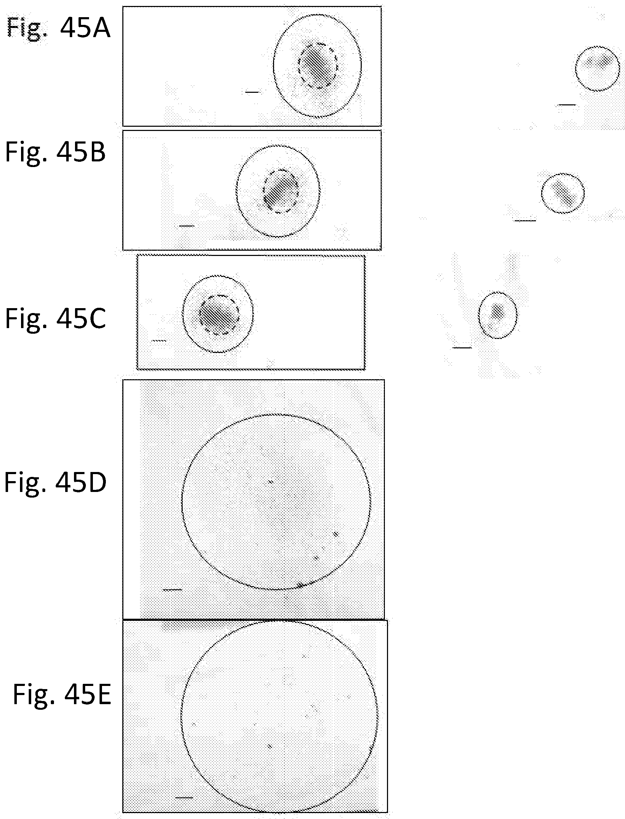

For a distance between nozzle and target of 30 cm, dispensing 100 µl a liquid in a carrier volume, the penetration of the aerosol through 4 mm of a fabric medium was compared for different operating conditions for the SipNose device and three commercial devices, the Alrin, the MAD Nasal from Wolfe Tory and the Otrivin devices. In all cases, the aerosol from the SipNose device penetrated the 4 mm of fabric (Fig. 45A-E and Table 7).

**Table 7: Spread on absorbing surface**

| | | | Spread | | | | |
|---|---|---|---|---|---|---|---|
| | | | Diameter | | Area | | |
| | Pressure (barg) | Carrier Volume (ml) | Inner (cm) | Outer (cm) | Inner (cm²) | Outer (cm²) | Penetration? |
| SipNose | | | | | | | |
| 1 | 5.6 | 18 | 2.5 | 6 | 4.9 | 28.3 | YES |
| 2 | 4 | 8 | 2 | 6.5 | 3.1 | 33.2 | YES |
| 3 | 6 | 8 | 3 | 5 | 7.1 | 19.6 | YES |

| Commercial | | | | | | | |
|---|---|---|---|---|---|---|---|
| Alrin^{™} nasal pump | --- | --- | --- | 15 | --- | 177 | NO |
| MAD Nasal | --- | --- | 8 | 12 | 50.2 | 113 | NO |
| Otrivin^{™} nasal pump | --- | --- | --- | 23 | --- | 415 | NO |

Fig 45A-C shows that, for the three typical operating conditions, significant amounts of the material penetrate through the 4 mm of fabric, consistent with what was seen for the nasal cast example (**Fig. 43**).

Fig. 45A-C also shows the inner area (dashed circle) delineating the area of heavier deposition and the outer area (solid circle) delineating the area of lighter deposition. For the two commercial devices, the MAD Nasal from Wolfe Tory (**Fig. 25D**) and the Alrin (Fig. 25E), deposition is light across the entire area, and the edges of the deposition region are not well define

## Claims

1. A device for delivering a predetermined amount V_{sub} of at least one substance, within at least one body cavity of a subject, said device comprising:
at least one capsule (1, 6, 21, 24, 10, 100) sized and shaped for containing said predetermined amount V_{sub} [ml] of said at least one substance;
a delivery end (9, 4A, 28, 42, 1100) for placement in proximity to said at least one body cavity, said delivery end being in fluid communication with said at least one capsule; said delivery end comprises at least one orifice of diameter D [mm];
at least one valve mechanically connectable to said at least one capsule, wherein the valve comprises at least two configurations: (i) an active configuration in which said at least one valve enables delivery of predetermined amount V_{sub} of said at least one substance from said at least one capsule to said at least one body cavity via said delivery end; and, (ii) an inactive configuration, in which said at least one valve prevents delivery of said predetermined amount V_{sub} of said at least one substance from said at least one capsule to said at least one body cavity;
said at least one valve is reconfigurable from said inactive configuration to said active configuration, and vice versa, within a predetermined period of time, dT, in response to activation of said at least one valve; and
a fluid tight chamber (2, 4) configured to contain a predetermined volume V_{gas} [ml] of pressurized gas at a predetermined pressure, P_{gas} [barg];
said pressurized gas, once said at least one valve is reconfigured from said inactive configuration to said active configuration, entrains said at least one substance and delivers said at least one substance via said at least one orifice in said delivery end within said at least one body cavity;
wherein said device is configured to deliver said predetermined amount V_{sub} of said at least one substance and said predetermined volume V_{gas} of said pressurized gas through said at least one orifice of diameter D [mm] in (a) pressure rate of dP_{gas} /dT; (b) volume rate of dV_{gas} /dT; and (c) volume rate of dV_{sub}/dT; and
wherein further
(a) the predetermined pressure P_{gas} is in a range of 1 - 10 barg;
(b) the predetermined volume V_{gas} is in a range of 1 - 21 ml;
(c) the predetermined amount V_{sub} is in at least one range selected from a group consisting of: 0.01-7 ml and 1-1000 mg; and
(d) the diameter D is in a range of 0.2 - 6 mm,
**characterized in that** said at least one capsule comprises at least one mixing ball (102) configured to provide a mixing function of said at least one substance during activation.

2. The device of claim 1, wherein said at least one mixing ball is configured to provide a separating function before activation.

3. The device of claim 1 or 2, wherein said predetermined amount V_{sub} of said at least one substance is contained in the at least one capsule.

4. The device of claim 3, wherein the at least one capsule comprises a separator configured to subdivide the at least one capsule into compartments.

5. The device of any one of the preceding claims, the device comprising a nosepiece,
wherein the device comprises a compressed gas chamber wall (4), a base (5) and an air chamber gate (8) inside the base for control of passage of pressurized gas locked between the compressed gas chamber wall (4) and the air chamber gate (8);
wherein the air chamber gate comprises a stopper (10) at its proximal end, with the stopper resting against a shoulder in the base, the shoulder preventing proximal movement of the air chamber gate;
wherein the air chamber gate comprises a gate O-ring (9) proximal to the air chamber gate to ensure an air-tight seal between the air chamber gate and the base;
wherein a septum (7) surrounds a distal end of the air chamber gate for providing an air-tight seal between the base and the compressed gas chamber wall; and
wherein upon reconfiguration of the at least one valve from said inactive configuration to said active configuration, the stopper is pressed inward allowing the air chamber gate to slide proximally to generate a gap between the septum and the capsule allowing passage of the predetermined volume V_{gas} of pressurized gas through said at least one substance to generate aerosol.

6. The device of claim 1,
wherein said at least one capsule comprises at least one barrier (101, 102), said barrier configured, when said at least one valve is in said inactive configuration, to provide a gas-tight seal for at least a portion of said at least one capsule and, when said at least one valve is in said active configuration, to allow passage through said at least a portion of said at least one capsule of flowable substance, at least a portion of said barrier being movable relative to said at least one capsule in a manner selected from a group consisting of slideably, rotatably, or any combination thereof; and
wherein said device is configured for a plurality of deliveries of said predetermined amount V_{sub}, said predetermined amount V_{sub} being controllably alterable.

7. The device of claim 1, wherein one of the following is true:
said body orifice is a nasal cavity, the mouth, the throat, an ear, the vagina, the rectum, the urethra, or any combination thereof;
viscosity η of said at least one substance is in a range of 1×10⁻³ poise to 1 poise;
DV50 diameter of particles of said at least one substance, after exit from said device, is less than 100µm;
DV90 diameter of said particles is less than 1000µm;
a full width of a plume of aerosol comprising said at least one substance and said gas subtends an angle Θ of less than 25°;
particles in said plume have velocities in a range of 5 m/s to 50 m/s;
said pressurized gas comprises air, nitrogen, oxygen, carbon dioxide, helium, neon, xenon, or any combination thereof;
during dispensing of said at least one substance, a mixture of said predetermined amount V_{gas} of said pressurized gas with said predetermined amount V_{sub} of said at least one substance entrained within it forms a plume of aerosol; said aerosol having a predetermined distribution, said distribution being either homogeneous or heterogeneous, said heterogeneous distribution is selected from a group consisting of: an arbitrary distribution, a distribution in which the density of said at least one substance within said mixture follows a predetermined pattern, or any combination thereof; characteristics of said aerosol selected from a group consisting of: particle size, particle shape, particle distribution, or any combination thereof, are determinable from characteristics of said device selected from a group consisting of: said predetermined volume of said pressurized gas, said predetermined amount V_{sub} of said at least one substance, said predetermined pressure of said pressurized gas, said predetermined orifice size, or any combination thereof;
said at least one substance is selected from a group consisting of a gas, a liquid, a powder, an aerosol, a slurry, a gel, a suspension, or any combination thereof;
said at least one substance is stored under either an inert atmosphere or under vacuum to prevent reactions during storage;
a dose-response curve is substantially linear for brain concentration of said at least one substance when administered nasally via said device; and
a dose-response curve for brain concentration having a fit selected from a group consisting of logarithmic, parabolic, exponential, sigmoid, power-low, or any combination thereof; of said at least one substance when administered nasally via said device.

8. The device of claim 3, wherein said at least one capsule has a main longitudinal axis, said at least one capsule comprises a number n of compartments, said amount V_{sub} of said at least one substance containable in at least one of said n compartments (130, 140, 150, 155); one of the following being true:
the number n of said compartments is an integer greater than or equal to 1; said at least one compartment has cross-section with shape selected from a group consisting of: wedge shaped, circular, oval, elliptical, polygonal, annular, or any combination thereof;
for said number n of compartments being an integer greater than 1, at least two said compartments have different volumes;
for said number n of compartments being an integer greater than 1, at least two said compartments have the same volume;
for said number n of compartments being an integer greater than 1, at least two said compartments have different cross-sectional areas;
for said number n of compartments being an integer greater than 1, at least two said compartments have the same cross-sectional area;
for said number n of compartments being an integer greater than 1, at least two said compartments contain different substances;
for said number n of compartments being an integer greater than 1, at least two said compartments contain the same substance;
for said number n of compartments being an integer greater than 1, at least two said compartments are disposed coaxially around said main longitudinal axis of said at least one capsule;
for said number n of compartments being an integer greater than 1, at least two said compartments are disposed sequentially along said main longitudinal axis of said at least one capsule;
for said number n of compartments greater than 1, a plurality of said at least one substance mix during said dispensing; and
for said number n of compartments greater than 1, said plurality of said at least one substance react during said dispensing.

9. The device of claim 3, wherein said at least one capsule comprises an injection port (2100) fluidly connectable to an exterior of said device, said injection port configured such that said at least one substance is insertable into a dispensing chamber of said at least one capsule via said injection port.

10. The device of claim 1, wherein said device comprises an injection port cover (2300) configured to provide an air-tight closure for said injection port, said injection port cover slideable along said device, rotatable around said device, rotatable around a hinge on an exterior of said device, or any combination thereof.

11. The device of claim 1, wherein said pressure rate, dP/dT→∞; or
wherein said pressure velocity dP_{gas}/dT is greater than 0.001 barg /ms.

12. The device of claim 1, wherein:
the volume rate dV_{sub}/dT is in at least one range selected from a group consisting of: greater than 0.0001 ml /ms and greater than 0.0001 mg/ms; and/or
said volume rate dV_{gas}/dT is greater than 0.001 ml /ms;
wherein dT is in a range of 0 to 500 millisecond.

## Patentansprüche

1. Vorrichtung zur Verabreichung einer vorbestimmten Menge V_{sub} mindestens einer Substanz in mindestens einen Körperhöhle eines Subjekts, wobei die Vorrichtung aufweist:
mindestens eine Kapsel (1, 6, 21, 24, 10, 100), die bemessen und geformt ist, die vorbestimmte Menge V_{sub} [ml] der mindestens einen Substanz zu enthalten;
ein Abgabeende (9, 4A, 28, 42, 1100) zur Platzierung in der Nähe der mindestens einen Körperhöhle, wobei das Abgabeende in Fluidverbindung mit der mindestens einen Kapsel steht; wobei das Abgabeende mindestens eine Öffnung mit einem Durchmesser D [mm] aufweist;
mindestens ein Ventil, das mechanisch mit der mindestens einen Kapsel verbindbar ist, wobei das Ventil mindestens zwei Konfigurationen aufweist: (i) eine aktive Konfiguration, in der das mindestens eine Ventil die Abgabe einer vorbestimmten Menge V_{sub} der mindestens einen Substanz aus der mindestens einen Kapsel an die mindestens eine Körperhöhle über das Abgabeende ermöglicht; und (ii) eine inaktive Konfiguration, in der das mindestens eine Ventil die Abgabe der vorbestimmten Menge V_{sub} der mindestens einen Substanz aus der mindestens einen Kapsel an die mindestens eine Körperhöhle verhindert;
das mindestens eine Ventil innerhalb einer vorbestimmten Zeitspanne dT als Reaktion auf die Aktivierung des mindestens einen Ventils von der inaktiven Konfiguration in die aktive Konfiguration und umgekehrt umkonfigurierbar ist; und
eine fluiddichte Kammer (2, 4), die konfiguriert ist, ein vorbestimmtes Volumen V_{gas} [ml] eines Druckgases bei einem vorbestimmten Druck P_{gas} [barg] zu enthalten;
wobei das Druckgas, sobald das mindestens eine Ventil von der inaktiven Konfiguration in die aktive Konfiguration umkonfiguriert ist, die mindestens eine Substanz mitreißt und die mindestens eine Substanz über die mindestens eine Öffnung im Abgabeende innerhalb der mindestens einen Körperhöhle abgibt;
wobei die Vorrichtung konfiguriert ist, die vorbestimmte Menge V_{sub} der mindestens einen Substanz und das vorbestimmte Volumen V_{gas} des Druckgases durch die mindestens eine Öffnung mit einem Durchmesser D [mm] mit (a) einer Druckrate von dP_{gas}/dT; (b) einer Volumenrate von dV_{gas}/dT; und (c) einer Volumenrate von dV_{sub}/dT zu verabreichen; und
wobei ferner
(a) der vorbestimmte Druck P_{gas} in einem Bereich von 1 - 10 barg liegt;
(b) das vorbestimmte Volumen V_{gas} im Bereich von 1 - 21 ml liegt;
(c) die vorbestimmte Menge V_{sub} in mindestens einem Bereich liegt, der aus einer Gruppe ausgewählt ist, die besteht aus: 0,01-7 ml und 1-1000 mg; und
(d) der Durchmesser D in einem Bereich von 0,2 - 6 mm liegt,
**dadurch gekennzeichnet, dass** die mindestens eine Kapsel mindestens eine Mischkugel (102) aufweist, die konfiguriert ist, während der Aktivierung eine Mischfunktion der mindestens einen Substanz bereitzustellen.

2. Vorrichtung nach Anspruch 1, wobei die mindestens eine Mischkugel konfiguriert ist, vor der Aktivierung eine Trennfunktion bereitzustellen.

3. Vorrichtung nach Anspruch 1 oder 2, wobei die vorbestimmte Menge V_{sub} der mindestens einen Substanz in der mindestens einen Kapsel enthalten ist.

4. Vorrichtung nach Anspruch 3, wobei die mindestens eine Kapsel einen Separator aufweist, der konfiguriert ist, die mindestens eine Kapsel in Kompartimente zu unterteilen.

5. Vorrichtung nach einem der vorangehenden Ansprüche, wobei die Vorrichtung ein Nasenstück aufweist,
wobei die Vorrichtung eine Druckgaskammerwand (4), eine Basis (5) und ein Luftkammertor (8) im Inneren der Basis aufweist, um den Durchgang von Druckgas zu steuern, das zwischen der Druckgaskammerwand (4) und dem Luftkammertor (8) eingeschlossen ist;
wobei das Luftkammertor an seinem proximalen Ende einen Stopfen (10) aufweist, wobei der Stopfen auf einer Schulter in der Basis ruht, wobei die Schulter eine proximale Bewegung des Luftkammertors verhindert;
wobei das Luftkammertor einen Tor-O-Ring (9) aufweist, der sich in der Nähe des Luftkammertors befindet, um eine luftdichte Abdichtung zwischen dem Luftkammertor und der Basis sicherzustellen;
wobei ein Septum (7) ein distales Ende des Luftkammertors umgibt, um eine luftdichte Abdichtung zwischen der Basis und der Druckgaskammerwand zu gewährleisten; und
wobei bei der Umkonfiguration des mindestens einen Ventils von der inaktiven Konfiguration in die aktive Konfiguration der Stopfen nach innen gedrückt wird, was es dem Luftkammertor ermöglicht, in proximaler Richtung zu gleiten, um einen Spalt zwischen dem Septum und der Kapsel zu erzeugen, der den Durchgang des vorbestimmten Volumens V_{gas} von Druckgas durch die mindestens eine Substanz ermöglicht, um Aerosol zu erzeugen.

6. Vorrichtung nach Anspruch 1,
wobei die mindestens eine Kapsel mindestens eine Barriere (101, 102) aufweist, wobei die Barriere konfiguriert ist, wenn sich das mindestens eine Ventil in der inaktiven Konfiguration befindet, eine gasdichte Abdichtung für mindestens einen Abschnitt der mindestens einen Kapsel bereitzustellen und, wenn sich das mindestens eine Ventil in der aktiven Konfiguration befindet, den Durchgang von fließfähiger Substanz durch den mindestens einen Abschnitt der mindestens einen Kapsel zu ermöglichen, wobei mindestens ein Abschnitt der Barriere relativ zu der mindestens einen Kapsel auf eine Art und Weise beweglich ist, die aus einer Gruppe ausgewählt ist, die aus verschiebbar, drehbar oder einer Kombination davon besteht; und
wobei die Vorrichtung für mehrere Abgaben der vorbestimmten Menge V_{sub} konfiguriert ist, wobei die vorbestimmte Menge V_{sub} steuerbar veränderbar ist.

7. Vorrichtung nach Anspruch 1, wobei eine der folgenden Bedingungen erfüllt ist:
die Körperöffnung ist eine Nasenhöhle, der Mund, der Rachen, ein Ohr, die Vagina, das Rektum, die Harnröhre oder eine Kombination davon;
die Viskosität η der mindestens einen Substanz in einem Bereich von 1×10⁻³ Poise bis 1 Poise liegt;
der DV50-Durchmesser der Partikel der mindestens einen Substanz nach dem Austritt aus der Vorrichtung weniger als 100 µm beträgt;
der DV90-Durchmesser der Partikel weniger als 1000 µm beträgt;
eine volle Breite einer Aerosolwolke, die die mindestens eine Substanz und das Gas aufweist, einen Winkel θ von weniger als 25° einschließt;
die Partikel in der Wolke Geschwindigkeiten in einem Bereich von 5 m/s bis 50 m/s aufweisen;
das Druckgas Luft, Stickstoff, Sauerstoff, Kohlendioxid, Helium, Neon, Xenon oder eine Kombination davon aufweist;
während der Abgabe der mindestens einen Substanz ein Gemisch der vorbestimmten Menge V_{gas} des Druckgases mit der vorbestimmten Menge V_{sub} der mindestens einen darin mitgerissenen Substanz eine Aerosolwolke bildet; wobei das Aerosol eine vorbestimmte Verteilung aufweist, wobei die Verteilung entweder homogen oder heterogen ist, wobei die heterogene Verteilung ausgewählt ist aus einer Gruppe bestehend aus: einer beliebigen Verteilung, einer Verteilung, in der die Dichte der mindestens einen Substanz innerhalb der Mischung einem vorbestimmten Muster folgt, oder einer beliebigen Kombination davon; wobei die Eigenschaften des Aerosols, die aus einer Gruppe ausgewählt sind, die aus der Partikelgröße, der Partikelform, der Partikelverteilung oder einer beliebigen Kombination davon besteht, aus den Eigenschaften der Vorrichtung bestimmbar sind, die aus einer Gruppe ausgewählt sind, die aus dem vorbestimmten Volumen des Druckgases, der vorbestimmten Menge V_{sub} der mindestens einen Substanz, dem vorbestimmten Druck des Druckgases, der vorbestimmten Öffnungsgröße oder einer beliebigen Kombination davon besteht;
die mindestens eine Substanz aus einer Gruppe ausgewählt ist, die aus einem Gas, einer Flüssigkeit, einem Pulver, einem Aerosol, einem Brei, einem Gel, einer Suspension oder einer beliebigen Kombination davon besteht;
die mindestens eine Substanz entweder unter einer inerten Atmosphäre oder unter Vakuum gelagert wird, um Reaktionen während der Lagerung zu verhindern;
eine Dosis-Wirkungs-Kurve für die Gehirnkonzentration der mindestens einen Substanz im Wesentlichen linear ist, wenn sie nasal über die Vorrichtung verabreicht wird; und
eine Dosis-Wirkungs-Kurve für die Gehirnkonzentration der mindestens einen Substanz, wenn sie nasal über die Vorrichtung verabreicht wird, einen Fit aufweist, der aus einer Gruppe ausgewählt ist, die aus logarithmisch, parabolisch, exponentiell, sigmoidal, Power-Low oder einer Kombination davon besteht.

8. Vorrichtung nach Anspruch 3, wobei die mindestens eine Kapsel eine Hauptlängsachse aufweist, die mindestens eine Kapsel eine Anzahl n von Kompartimenten aufweist, die Menge V_{sub} der mindestens einen Substanz in mindestens einem der n Kompartimente (130, 140, 150, 155) enthalten sein kann; wobei eine der folgenden Bedingungen erfüllt ist:
die Anzahl n der Kompartimente ist eine ganze Zahl größer oder gleich 1; das mindestens eine Kompartiment hat einen Querschnitt mit einer Form, die ausgewählt ist aus der Gruppe bestehend aus: keilförmig, kreisförmig, oval, elliptisch, polygonal, ringförmig oder einer beliebigen Kombination davon;
wenn die Anzahl n der Kompartimente eine ganze Zahl größer als 1 ist, haben mindestens zwei der Kompartimente unterschiedliche Volumina;
für die Anzahl n der Kompartimente, die eine ganze Zahl größer als 1 ist, haben mindestens zwei der Kompartimente das gleiche Volumen;
für die Anzahl n der Kompartimente, die eine ganze Zahl größer als 1 ist, haben mindestens zwei der Kompartimente unterschiedliche Querschnittsflächen;
für die Anzahl n der Kompartimente, die eine ganze Zahl größer als 1 ist, haben mindestens zwei der Kompartimente die gleiche Querschnittsfläche;
für die Anzahl n der Kompartimente, die eine ganze Zahl größer als 1 ist, enthalten mindestens zwei der Kompartimente unterschiedliche Substanzen;
für die Anzahl n der Kompartimente, die eine ganze Zahl größer als 1 ist, enthalten mindestens zwei der Kompartimente die gleiche Substanz;
für die Anzahl n der Kompartimente, die eine ganze Zahl größer als 1 ist, sind mindestens zwei der Kompartimente koaxial um die Hauptlängsachse der mindestens einen Kapsel angeordnet;
für die Anzahl n der Kompartimente, die eine ganze Zahl größer als 1 ist, sind mindestens zwei der Kompartimente aufeinanderfolgend entlang der Hauptlängsachse der mindestens einen Kapsel angeordnet;
für die Anzahl n der Kompartimente, die größer als 1 ist, eine Mehrzahl der mindestens einen Substanz während der Abgabe gemischt wird; und
für die Anzahl n der Kompartimente, die größer als 1 ist, reagiert die Mehrzahl der mindestens einen Substanz während der Abgabe.

9. Vorrichtung nach Anspruch 3, wobei die mindestens eine Kapsel eine Injektionsöffnung (2100) aufweist, die mit der Außenseite der Vorrichtung in Fluidverbindung gebracht werden kann, wobei die Injektionsöffnung so konfiguriert ist, dass die mindestens eine Substanz über die Injektionsöffnung in eine Abgabekammer der mindestens einen Kapsel einführbar ist.

10. Vorrichtung nach Anspruch 1, wobei die Vorrichtung eine Injektionsöffnungsabdeckung (2300) aufweist, die konfiguriert ist, einen luftdichten Verschluss für die Injektionsöffnung bereitzustellen, wobei die Injektionsöffnungsabdeckung entlang der Vorrichtung verschiebbar, um die Vorrichtung drehbar, um ein Scharnier an einer Außenseite der Vorrichtung drehbar oder eine beliebige Kombination davon ist.

11. Vorrichtung nach Anspruch 1, wobei die Druckrate dP/dT→∞; oder
wobei die Druckrate dP_{gas}/dT größer als 0,001 barg/ms ist.

12. Vorrichtung nach Anspruch 1, wobei:
die Volumenrate dV_{sub}/dT in mindestens einem Bereich liegt, der aus einer Gruppe ausgewählt ist, die besteht aus: größer als 0,0001 ml/ms und größer als 0,0001 mg/ms; und/oder
die Volumenrate dV_{gas}/dT größer als 0,001 ml/ms ist;
wobei dT in einem Bereich von 0 bis 500 Millisekunden liegt.

## Revendications

1. Dispositif pour administrer une quantité V_{sub} prédéterminée d'au moins une substance, à l'intérieur d'au moins une cavité corporelle d'un sujet, ledit dispositif comprenant :
au moins une capsule (1, 6, 21, 24, 10, 100) dimensionnée et formée de manière à contenir ladite quantité V_{sub} [ml] prédéterminée de ladite au moins une substance ;
une extrémité de distribution (9, 4A, 28, 42, 1100) destinée à être placée à proximité de ladite au moins une cavité corporelle, ladite extrémité de distribution étant en communication fluidique avec ladite au moins une capsule ; ladite extrémité de distribution comprend au moins un orifice de diamètre D [mm] ;
au moins une valve reliée mécaniquement à ladite au moins une capsule, la valve comprenant au moins deux configurations : (i) une configuration active dans laquelle ladite au moins une valve permet l'administration d'une quantité V_{sub} prédéterminée de ladite au moins une substance depuis ladite au moins une capsule vers ladite au moins une cavité corporelle via ladite extrémité de distribution ; et, (ii) une configuration inactive, dans laquelle ladite au moins une valve empêche l'administration de ladite quantité V_{sub} prédéterminée de ladite au moins une substance depuis ladite au moins une capsule vers ladite au moins une cavité corporelle ;
ladite au moins une valve est reconfigurable de ladite configuration inactive à ladite configuration active, et vice versa, au cours d'une période de temps prédéterminée, dT, en réponse à l'activation de ladite au moins une valve ; et
une chambre étanche (2, 4) configurée pour contenir un volume prédéterminé V_{gas} [ml] de gaz sous pression à une pression P_{gas} [barg] prédéterminée ;
ledit gaz sous pression, une fois que ladite au moins une valve est reconfigurée de ladite configuration inactive à ladite configuration active, entraîne ladite au moins une substance et administre ladite au moins une substance via ledit au moins un orifice dans ladite extrémité de distribution à l'intérieur de ladite au moins une cavité corporelle ;
dans lequel ledit dispositif est configuré pour administrer ladite quantité V_{sub} prédéterminée de ladite au moins une substance et ledit volume prédéterminé V_{gas} dudit gaz sous pression à travers ledit au moins un orifice de diamètre D [mm] en (a) taux de pression de dP_{gas}/dT ; (b) taux de volume de dV_{gas}/dT ; et (c) taux de volume de dV_{sub}/dT ; et
dans lequel, en outre
(a) la pression P_{gas} prédéterminée est comprise entre 1 et 10 barg ;
(b) le volume V_{gas} prédéterminé est compris entre 1 et 21 ml ;
(c) la quantité V_{sub} prédéterminée se situe dans au moins un intervalle choisi dans le groupe suivant : 0,01 à 7 ml et 1 à 1000 mg ; et
(d) le diamètre D est compris entre 0,2 et 6 mm,
**caractérisé en ce que** ladite au moins une capsule comprend au moins une bille de mélange (102) configurée pour assurer une fonction de mélange de ladite au moins une substance pendant l'activation.

2. Dispositif de la revendication 1, dans lequel ladite au moins une bille de mélange est configurée pour fournir une fonction de séparation avant l'activation.

3. Dispositif de la revendication 1 ou 2, dans lequel ladite quantité V_{sub} prédéterminée de ladite au moins une substance est contenue dans la au moins une capsule.

4. Dispositif de la revendication 3, dans lequel l'au moins une capsule comprend un séparateur configuré pour subdiviser l'au moins une capsule en compartiments.

5. Dispositif de l'une quelconque des revendications précédentes, le dispositif comprenant un embout nasal,
le dispositif comprenant une paroi de chambre à gaz comprimé (4), une base (5) et un obturateur de chambre à air (8) à l'intérieur de la base pour contrôler le passage du gaz sous pression enfermé entre la paroi de chambre à gaz comprimé (4) et l'obturateur de chambre à air (8) ;
dans lequel l'obturateur de chambre à air comprend un bouchon (10) à son extrémité proximale, le bouchon reposant contre un épaulement dans la base, l'épaulement empêchant le mouvement proximal de l'obturateur de chambre à air ;
dans lequel l'obturateur de chambre à air comprend un joint torique (9) à proximité de l'obturateur de chambre à air afin d'assurer l'étanchéité entre l'obturateur de chambre à air et la base ;
dans lequel un septum (7) entoure l'extrémité distale de l'obturateur de chambre à air pour assurer l'étanchéité entre la base et la paroi de chambre à gaz comprimé ; et
dans lequel, lors de la reconfiguration de l'au moins une valve de ladite configuration inactive à ladite configuration active, le bouchon est pressé vers l'intérieur, ce qui permet à l'obturateur de chambre à air de glisser de manière proximale pour créer un espace entre le septum et la capsule, permettant le passage du volume V_{gas} prédéterminé de gaz sous pression à travers ladite au moins une substance afin de générer un aérosol.

6. Dispositif de la revendication 1, dans lequel ladite au moins une capsule comprend au moins une barrière (101, 102), ladite barrière étant configurée, lorsque ladite au moins une valve est dans ladite configuration inactive, pour fournir un joint étanche au gaz pour au moins une partie de ladite au moins une capsule et, lorsque ladite au moins une valve est dans ladite configuration active, pour permettre le passage à travers ladite au moins une partie de ladite au moins une capsule de substance fluide, au moins une partie de ladite barrière étant mobile par rapport à ladite au moins une capsule d'une manière choisie dans un groupe consistant en un glissement, une rotation, ou toute combinaison de ces mouvements ; et
ledit dispositif étant configuré pour une pluralité d'administrations de ladite quantité V_{sub} prédéterminée, ladite quantité V_{sub} prédéterminée étant modifiable de manière contrôlée.

7. Dispositif de la revendication 1, dans lequel l'un des points suivants est vrai :
ledit orifice corporel est une cavité nasale, la bouche, la gorge, une oreille, le vagin, le rectum, l'urètre, ou toute combinaison de ceux-ci ;
la viscosité η de ladite au moins une substance est comprise entre 1×10⁻³ poise et 1 poise
le diamètre DV50 des particules de ladite au moins une substance, après leur sortie dudit dispositif, est inférieur à 100 µm ;
le diamètre DV90 desdites particules est inférieur à 1000 µm ;
la largeur totale d'un panache d'aérosol comprenant ladite au moins une substance et ledit gaz sous-tend un angle θ inférieur à 25° ;
les particules dudit panache ont des vitesses comprises entre 5 m/s et 50 m/s ;
ledit gaz sous pression comprend de l'air, de l'azote, de l'oxygène, du dioxyde de carbone, de l'hélium, du néon, du xénon ou toute combinaison de ceux-ci ;
pendant l'administration de ladite au moins une substance, un mélange de ladite quantité V_{gas} prédéterminée dudit gaz pressurisé avec ladite quantité V_{sub} prédéterminée de ladite au moins une substance entraînée forme un panache d'aérosol ; ledit aérosol a une administration prédéterminée, ladite administration étant soit homogène, soit hétérogène, ladite administration hétérogène étant choisie dans le groupe suivant : une administration arbitraire, une administration dans laquelle la densité de ladite au moins une substance dans ledit mélange suit un modèle prédéterminé, ou toute combinaison de ces administrations ; les caractéristiques dudit aérosol étant choisies dans un groupe consistant en : la taille des particules, la forme des particules, l'administration des particules, ou toute combinaison de ces éléments, sont déterminables à partir des caractéristiques dudit dispositif choisies dans un groupe consistant en : ledit volume prédéterminé dudit gaz pressurisé, ladite quantité V_{sub} prédéterminée de ladite au moins une substance, ladite pression prédéterminée dudit gaz pressurisé, ladite taille prédéterminée de l'orifice, ou toute combinaison de ces éléments ;
ladite au moins une substance est choisie dans un groupe constitué d'un gaz, d'un liquide, d'une poudre, d'un aérosol, d'une boue, d'un gel, d'une suspension ou de toute combinaison de ces éléments ;
ladite au moins une substance est stockée sous atmosphère inerte ou sous vide afin d'éviter les réactions pendant le stockage ;
une courbe dose-réponse est substantiellement linéaire pour la concentration cérébrale de ladite au moins une substance lorsqu'elle est administrée par voie nasale via ledit dispositif ; et
une courbe dose-réponse pour la concentration cérébrale ayant un ajustement choisi dans le groupe suivant : logarithmique, parabolique, exponentiel, sigmoïde, loi de puissance, ou toute combinaison de ces éléments ; de ladite au moins une substance lorsqu'elle est administrée par voie nasale via ledit dispositif.

8. Dispositif de la revendication 3, dans lequel ladite au moins une capsule présente un axe longitudinal principal, ladite au moins une capsule comprend un nombre n de compartiments, ladite quantité V_{sub} de ladite au moins une substance pouvant être contenue dans au moins un de ces n compartiments (130, 140, 150, 155) ; l'une des conditions suivantes étant vraie :
le nombre n desdits compartiments est un nombre entier supérieur ou égal à 1 ; ledit au moins un compartiment a une section transversale dont la forme est choisie dans le groupe suivant : en forme de coin, circulaire, ovale, elliptique, polygonale, annulaire, ou toute combinaison de ces formes ;
pour ledit nombre n de compartiments étant un nombre entier supérieur à 1, au moins deux desdits compartiments ont des volumes différents ;
pour ledit nombre n de compartiments étant un nombre entier supérieur à 1, au moins deux desdits compartiments ont le même volume ;
pour ledit nombre n de compartiments étant un nombre entier supérieur à 1, au moins deux desdits compartiments ont des surfaces de section transversale différentes ;
pour ledit nombre n de compartiments étant un nombre entier supérieur à 1, au moins deux desdits compartiments ont la même surface de section transversale ;
pour ledit nombre n de compartiments étant un nombre entier supérieur à 1, au moins deux desdits compartiments contiennent des substances différentes ;
pour ledit nombre n de compartiments étant un nombre entier supérieur à 1, au moins deux desdits compartiments contiennent la même substance ;
pour ledit nombre n de compartiments étant un nombre entier supérieur à 1, au moins deux desdits compartiments sont disposés coaxialement autour dudit axe longitudinal principal de ladite au moins une capsule ;
pour ledit nombre n de compartiments étant un nombre entier supérieur à 1, au moins deux desdits compartiments sont disposés séquentiellement le long dudit axe longitudinal principal de ladite au moins une capsule ;
pour ledit nombre n de compartiments supérieur à 1, une pluralité de ladite au moins une substance se mélange pendant ladite administration ; et
pour ledit nombre n de compartiments supérieur à 1, ladite pluralité de ladite au moins une substance réagit pendant ladite administration.

9. Dispositif de la revendications 3, dans lequel ladite au moins une capsule comprend un port d'injection (2100) pouvant être relié de manière fluide à un extérieur dudit dispositif, ledit port d'injection étant configuré de manière à ce que ladite au moins une substance puisse être insérée dans une chambre de distribution de ladite au moins une capsule par l'intermédiaire dudit port d'injection.

10. Dispositif de la revendication 1, dans lequel ledit dispositif comprend un couvercle de port d'injection (2300) configuré pour fournir une fermeture étanche à l'air pour ledit port d'injection, ledit couvercle de port d'injection pouvant être glissé le long dudit dispositif, pouvant tourner autour dudit dispositif, pouvant tourner autour d'une charnière sur l'extérieur dudit dispositif, ou toute combinaison de ces mouvements.

11. Dispositif de la revendication 1, dans lequel ladite vitesse de pression, dP/dT→∞ ; ou dans lequel ladite vitesse de pression dP_{gas}/dT est supérieure à 0,001 barg/ms.

12. Dispositif de la revendication 1, dans lequel :
le taux de volume dV_{sub}/dT se situe dans au moins un intervalle choisi dans le groupe suivant : plus de 0,0001 ml/ms et plus de 0,0001 mg/ms ; et/ou
ledit taux de volume dV_{gas}/dT est supérieur à 0,001 ml/ms ;
où dT est compris entre 0 et 500 millisecondes.
